# EUROPEAN PATENT APPLICATION

(11) **EP 4 737 469 A1**
(43) Date of publication of application: **06.05.2026**
(21) Application number: 24830843.9
(22) Date of filing: 27.06.2024
(51) Int. Cl.: C07H 15/04, C07H 21/02, C07H 1/00, A61K 31/713, A61K 47/54, A61P 1/16

(54) **NUCLEIC ACID CONJUGATES AS WELL AS PREPARATION METHOD THEREFOR AND USE THEREOF**

(30) Priority: 30.06.2023 CN 202310797263
(71) Applicant: Suzhou Siran Biotechnology Co., Ltd., Suzhou, Jiangsu 215000 (CN)
(72) Inventor: SHAO, Hongxia, Suzhou, Jiangsu 215000 (CN); YANG, Zhiwei, Suzhou, Jiangsu 215000 (CN); LIU, Nan, Suzhou, Jiangsu 215000 (CN)
(74) Representative: Bryers Intellectual Property Ltd
(86) International application number: PCT/CN2024/101798
(87) International publication number: WO 2025/002208

(57) **Abstract**

The present invention provides a compound for forming a conjugate with an oligonucleotide, the compound having a structure as shown in Formula (I). The present disclosure also provides a corresponding conjugate, and the use of said conjugate in the preparation of a medicament for preventing and/or treating related diseases. The conjugate of the present disclosure has the advantages of inexpensive raw materials, simple synthesis, and easy process development, while also having good oligonucleotide synthesis efficiency and in vivo biological activity in small animals.

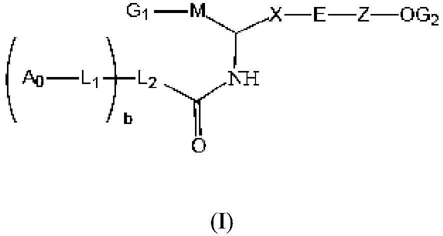

## Description

### Technical Field

The present disclosure belongs to the field of nucleic acid delivery, and specifically relates to a nucleic acid conjugate, a preparation method and a use thereof.

### Background Art

SiRNA is a negatively charged macromolecule composed of two oligonucleotide chains, which cannot effectively target tissues in the body on its own, nor can it enter cells autonomously. SiRNA requires special delivery vehicles to achieve enrichment in target tissues and enable it to enter cells to exert its therapeutic effect.

Lipid Nanoparticles (LNPs) can encapsulate siRNA and effectively achieve liver-targeted delivery. The drug Onpattro, which uses LNP technology, has been marketed. However, the clinical application of LNP technology still has many shortcomings, for example: LNP formulations have a short validity period; immune responses are generated during application, often requiring the synergistic use of immunosuppressants such as dexamethasone, which complicates the clinical application of LNPs.

Targeted delivery of drugs into cells through endocytosis mediated by cell surface receptors is an effective strategy. The asialoglycoprotein receptor (ASGPR) is a receptor that is highly specifically expressed on the surface of hepatocytes, characterized by high abundance and high receptor recycling efficiency. By covalently conjugating ligands that can specifically recognize ASGPR, such as monosaccharides and polysaccharides like galactose, galactosamine, and N-acetylgalactosamine (GalNAc), with siRNA, the siRNA can be targeted for delivery into hepatocytes, allowing the siRNA to exert its gene-silencing effect on the target gene. Therefore, the research and development of targeting ligands suitable for siRNA delivery is of great significance for the clinical application of siRNA.

Currently, some progress has been made globally in research on achieving effective liver-targeted delivery of siRNA by targeting ASGPR. A representative delivery molecule is L96 from Alnylam Pharmaceuticals (its structural formula is shown below), which has been used in four marketed siRNA drugs and has shown good delivery activity and selectivity in both in vitro and in vivo experiments. However, there are still problems such as the in vivo biological activity and efficacy of siRNA can be further improved, and the CMC (Chemistry, Manufacturing, and Controls) process and technology for delivery molecules are complex and production costs are high.

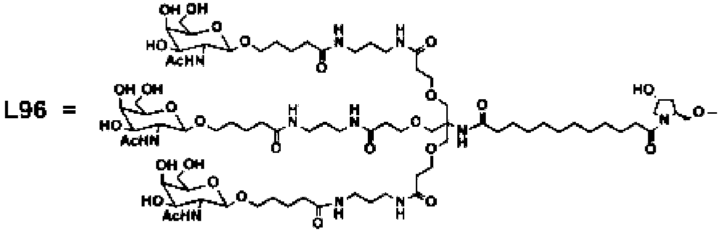

### Summary of the Invention

The delivery compounds and corresponding nucleic acid conjugates of the present disclosure use natural amino acids such as serine, threonine, etc., as the core linking backbone, resulting in a new class of GalNAc conjugates with completely new chemical structures. Compared with existing delivery technologies, they have the advantages of simple molecular structure, inexpensive and readily available raw materials, and easy-to-develop CMC processes, as well as good oligonucleotide synthesis efficiency and in vivo biological activity.

In a first aspect, the present disclosure provides a compound having the structure shown in Formula (I): 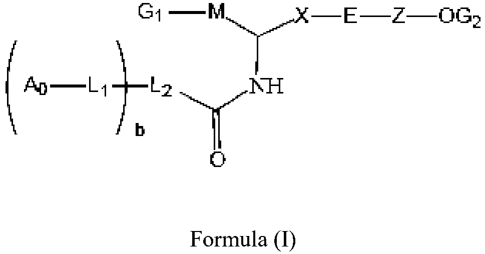 wherein,
*A*₀ represents a ligand having affinity for the asialoglycoprotein receptor on the surface of mammalian liver cells, or represents a group formed by replacing all or part of the hydroxyl groups in said ligand with a KCOO- group, wherein each K is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl;
wherein said ligand is preferably independently selected from any one of the following: D-mannopyranose, L-mannopyranose, D-arabinose, D-xylofuranose, L-xylofuranose, D-glucose, L-glucose, D-galactose, L-galactose, *α*-D-mannofuranose, *β*-D-mannofuranose, *α*-D-mannopyranose, *β-*D-mannopyranose, *α*-D-glucopyranose, *β*-D-glucopyranose, *α*-D-glucofuranose, *β*-D-glucofuranose, *α*-D-fructofuranose, *α*-D-fructopyranose, *α*-D-galactopyranose, *β*-D-galactopyranose, *α*-D-galactofuranose, *β*-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-*β*-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-*α*-neuraminic acid, 5-thio-*β*-D-glucopyranose, methyl 2,3,4-tri-O-acetyl-1-thio-6-O-trityl-*α*-D-glucopyranoside, 4-thio-*β*-D-galactopyranose, ethyl 3,4,6,7-tetra-O-acetyl-2-deoxy-1,5-dithio-*α*-D-glucoheptopyranoside, 2,5-anhydro-D-allononitrile, ribose, D-ribose, D-4-thioribose, L-ribose, L-4-thioribose;
b is an integer ranging from 1 to 4, preferably 1, 2, or 3, more preferably 1 or 3;
*L*₁ and *L*₂ independently represent a linking combination of one or more groups selected from Formula A1-A11: 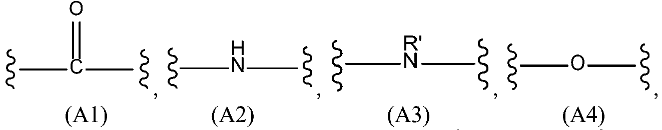 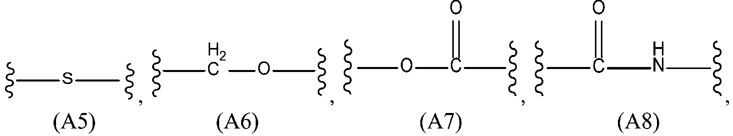 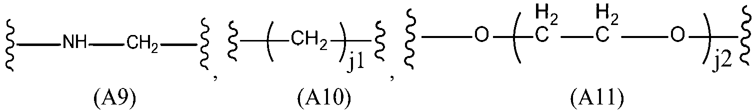
wherein, j1 is an integer from 1-20; j2 is an integer from 1-20; R' is a *C*₁ - *C*₁₀ alkyl; M represents a structural formula as shown in Formula (A12): 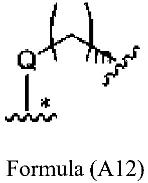
wherein, m represents an integer from 0-6, preferably 0, 1, or 2; * represents the bond connected to *G*₁, Q represents a structural formula as shown in Formula (A13): 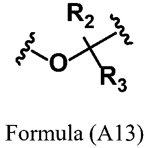
wherein, 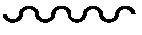 connected to O represents the bond connected to *G*₁; *R*₂, *R*₃ are the same or different, and are independently selected from H, *C*₁ - *C*₂₀ alkyl, *C*₁ - *C*₂₀ alkoxy, *C*₂ - *C*₂₀ alkenyl, *C*₂ - *C*₂₀ or alkynyl, preferably H, *C*₁ - *C*₆ alkyl, *C*₁ - *C*₆ alkoxy, *C*₂ - *C*₆ alkenyl, or *C*₂ - *C*₆ alkynyl;
Z is absent or represents a *C*₁ - *C*₁₀ alkylene, preferably a *C*₁ - *C*₃ alkylene;
X represents a structural formula as shown in Formula (A14-1) or (A14-2): 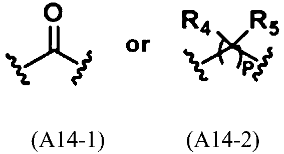
wherein *R*₄, *R*₅ are the same or different, and are independently selected from H, fluoro, hydroxyl, *C*₁ - *C*₂₀ alkyl, *C*₁ - *C*₂₀ alkoxy, *C*₂ - *C*₂₀ alkenyl, or *C*₂ - *C*₂₀ alkynyl, preferably H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is an integer from 1-6, preferably 1, 2, or 3; optionally, *R*₄ and *R*₅ are directly connected to form a three- to six-membered saturated carbocyclic ring;
E represents a structural formula as shown in Formula (A15): 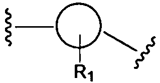 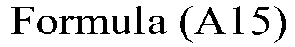
wherein 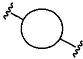 represents a *C*₃ - *C*₁₈ cycloalkyl or a *C*₃ - *C*₁₈ heterocyclyl, preferably represents a *C*₄ - *C*₈ cycloalkyl or a *C*₃ - *C*₈ heterocyclyl, more preferably represents a *C*₃ - *C*₆ heterocyclyl, and even more preferably a four- to six-membered nitrogen-containing saturated cycloalkyl, and *R*₁ is selected from H, fluoro, hydroxyl, cyano, *C*₁ - *C*₂₀ alkyl, *C*₁ - *C*₂₀ alkoxy, *C*₂ - *C*₂₀ alkenyl, or *C*₂ - *C*₂₀ alkynyl, preferably selected from H, fluoro, hydroxyl, cyano, *C*₁ - *C*₆ alkyl, *C*₁ - *C*₆ alkoxy, *C*₂ - *C*₆ alkenyl, or *C*₂ - *C*₆ alkynyl;
*G*₁ and *G*₂ each represent a phosphoramidite functional group having the structure shown in Formula (G-1) or a hydroxyl protecting group, provided that at least one of *G*₁ and *G*₂ has the structure shown in Formula (G-1), said hydroxyl protecting group is selected from any one of trityl, 4-methoxytrityl, 4,4'-dimethoxytrityl (DMTr), and 4,4',4''-trimethoxytrityl, preferably 4,4'-dimethoxytrityl; 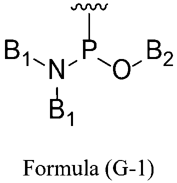
wherein, *B*₁ is selected from substituted or unsubstituted *C*₁ - *C*₅ hydrocarbyl, optionally, *B*₁ is methyl, ethyl, or isopropyl; *B*₂ is selected from one of *C*₁ - *C*₅ alkyl, cyanoethyl, cyanopropyl, or cyanobutyl, optionally, *B*₂ is cyanoethyl;

Herein, 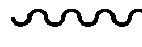 represents the site of covalent bond attachment of the group.

In a second aspect, the present invention provides a nucleic acid conjugate, wherein the conjugate contains one or more structures as shown in Formula (II) connected to any position on an oligonucleotide sequence, preferably two, three, or four consecutively connected structures as shown in Formula (II): 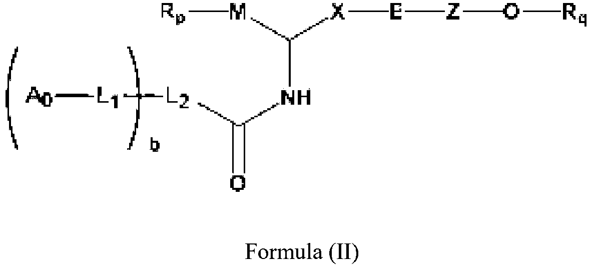
wherein, *Rₚ* and *R_{q}* are each H or have the structure shown in Formula A16, provided that at least one of *Rₚ* and *R_{q}* has the structure shown in Formula A16; 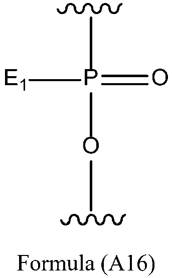
wherein, *E*₁ is OH, SH, or *BH*₂,
*A*₀, b, *L*₁, *L*₂, M, Z, X, E are the same as in Formula (I), and are not repeated here.

In a third aspect, the present invention provides a preparation method for the nucleic acid conjugate of the second aspect.

In a fourth aspect, the present invention provides the use of the nucleic acid conjugate of the present invention in the preparation of a medicament for treating and/or preventing liver-derived diseases.

In a fifth aspect, the present invention provides a method for treating a pathological condition or disease caused by the expression of a gene in hepatocytes, said method comprising administering the nucleic acid conjugate of the second aspect to a patient suffering from the disease.

In a sixth aspect, the present invention provides a kit, wherein the kit comprises the nucleic acid conjugate of the present invention.

### Brief Description of the Drawings

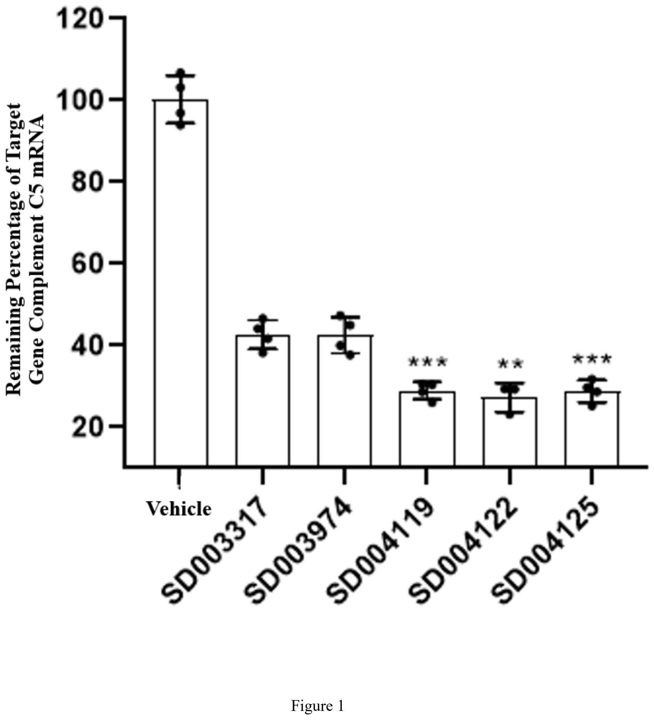

Figure 1 shows the experimental results of the activity of siRNA conjugates tested in vivo in mice in Example 10; wherein ** in Figure 1 represents P less than 0.01, and *** represents P less than 0.001.

### Detailed Description of Embodiments

The specific embodiments of the present disclosure are described in detail below. It should be understood that the specific embodiments described here are only for illustrating and explaining the present disclosure, and are not intended to limit the present disclosure.

### Definitions

It should be noted that, unless otherwise defined, the technical or scientific terms used in this application shall have the ordinary meaning as understood by a person skilled in the art.

In the present disclosure, the compound represented by general formula (I) includes its tautomers, racemates, enantiomers, diastereomers, mixtures thereof, and the like.

As used herein, a hyphen ("-") that is not between two letters or two symbols is used to indicate the point of attachment of a substituent.

As used herein, "optional" or "optionally" means that the event or circumstance described thereafter may or may not occur, and that the description includes instances where the event or circumstance occurs and instances where it does not. For example, "optionally substituted alkyl" includes "alkyl" and "substituted alkyl" as defined below. Those skilled in the art will understand that, for any group containing one or more substituents, these groups are not intended to introduce any substitution or substitution pattern that is sterically impractical, synthetically infeasible, and/or inherently unstable. As used herein, "alkyl" refers to straight and branched chains having the specified number of carbon atoms. When an alkyl residue having a specific number of carbons is mentioned, it is intended to cover all branched and straight-chain forms having that number of carbons; thus, for example, "butyl" is meant to include n-butyl, sec-butyl, isobutyl, and tert-butyl. Alkylene is a subset of alkyl, referring to the same residue as alkyl but having two points of attachment.

As used herein, "alkenyl" refers to an unsaturated branched or straight-chain alkyl having at least one carbon-carbon double bond, which is obtained by the loss of one hydrogen atom from each of adjacent carbon atoms of the parent alkyl. The group may be in the cis or trans configuration of the double bond. Typical alkenyl groups include, but are not limited to: ethenyl; propenyls, such as prop-1-en-1-yl, prop-1-en-2-yl, prop-2-en-1-yl (allyl), prop-2-en-2-yl; butenyls, such as but-1-en-1-yl, but-1-en-2-yl, 2-methylprop-1-en-1-yl, but-2-en-1-yl, but-2-en-2-yl, but-1,3-dien-1-yl, but-1,3-dien-2-yl, and the like. Alkenylene is a subset of alkenyl, referring to the same residue as alkenyl but having two points of attachment.

As used herein, "alkynyl" refers to an unsaturated branched or straight-chain alkyl having at least one carbon-carbon triple bond, which is obtained by the loss of two hydrogen atoms from each of adjacent carbon atoms of the parent alkyl. Typical alkynyl groups include, but are not limited to: ethynyl; propynyls, such as prop-1-yn-1-yl, prop-2-yn-1-yl; butynyls, such as but-1-yn-1-yl, but-1-yn-3-yl, but-3-yn-1-yl, and the like.

As used herein, "alkoxy" refers to an alkyl group of the specified number of carbon atoms attached through an oxygen bridge, for example, methoxy, ethoxy, propoxy, isopropoxy, n-butoxy, sec-butoxy, tert-butoxy, pentoxy, 2-pentoxy, isopentoxy, neopentoxy, hexoxy, 2-hexoxy, 3-hexoxy, 3-methylpentoxy, and the like. Alkoxy generally has 1 to 10, 1 to 8, 1 to 6, or 1 to 4 carbon atoms attached through an oxygen bridge.

As used herein, "cycloalkyl" refers to a non-aromatic carbocycle, typically having 3 to 7 ring carbon atoms. The ring can be saturated or have one or more carbon-carbon double bonds. Examples of cycloalkyl include cyclopropyl, cyclobutyl, cyclopentyl, cyclopentenyl, cyclohexyl, and cyclohexenyl, as well as bridged and caged ring groups such as norbornane.

As used herein, "halogen substituent" or "halo" refers to fluoro, chloro, bromo, and iodo, and the term "halogen" includes fluorine, chlorine, bromine, and iodine.

As used herein, "haloalkyl" refers to an alkyl group of the specified number of carbon atoms substituted with one or more, up to the maximum allowable number, of halogen atoms. Examples of haloalkyl include but are not limited to trifluoromethyl, difluoromethyl, 2-fluoroethyl, and pentafluoroethyl. "Heterocyclyl" refers to a stable 3- to 18-membered non-aromatic ring group containing 2-12 carbon atoms and 1-6 heteroatoms selected from nitrogen, oxygen, and sulfur. Unless otherwise specified in the specification, the heterocyclyl is a monocyclic, bicyclic, tricyclic, or tetracyclic system, which may include fused or bridged ring systems. The heteroatoms in the heterocyclyl may optionally be oxidized. One or more nitrogen atoms (if present) are optionally quaternized. The heterocyclyl is partially saturated or fully saturated. The heterocyclyl may be attached to the remainder of the molecule through any ring atom. Examples of such heterocyclyl groups include, but are not limited to: dioxanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuranyl, trithianyl, tetrahydropyranyl, thiomorpholinyl, thiamorpholinyl, 1-oxo-thiomorpholinyl, and 1,1-dioxo-thiomorpholinyl.

Various hydroxyl protecting groups may be used in the present disclosure. In general, protecting groups render a chemical functional group non-reactive to specific reaction conditions and can be attached to and removed from that functional group in a molecule without substantially damaging the rest of the molecule. Representative hydroxyl protecting groups are disclosed in Beaucage et al., Tetrahedron 1992, 48, 2223-2311, and Greene and Wuts, Protective Groups in Organic Synthesis, Chapter 2, 2d ed, John Wiley & Sons, New York, 1991, which are incorporated herein by reference in their entirety. In some embodiments, the protecting group is stable under basic conditions but can be removed under acidic conditions. In some embodiments, non-exclusive examples of hydroxyl protecting groups that can be used herein include dimethoxytrityl (DMT), monomethoxytrityl, 9-phenylxanthen-9-yl (Pixyl), and 9-(p-methoxyphenyl)xanthen-9-yl (Mox). In some embodiments, non-exclusive examples of hydroxyl protecting groups that can be used herein include Tr (trityl), MMTr (4-methoxytrityl), DMTr (4,4'-dimethoxytrityl), and TMTr (4,4',4"-trimethoxytrityl) and tert-butyldimethylsilyl (TBS or TBDMS). Non-exclusive examples of hydroxyl protecting groups that can be used herein include hydrocarbyl acyl.

The term "subject," as used herein, refers to any animal, such as a mammal or a marsupial. Subjects of the present disclosure include, but are not limited to, humans, non-human primates (e.g., rhesus monkeys or other types of macaques), mice, pigs, horses, donkeys, cattle, sheep, rats, and poultry of any species.

As used herein, "treating," "alleviating," or "ameliorating" may be used interchangeably herein. These terms refer to methods of obtaining a beneficial or desired result, including but not limited to therapeutic benefit. "Therapeutic benefit" means the eradication or amelioration of the underlying disorder being treated. Furthermore, a therapeutic benefit is obtained by eradicating or ameliorating one or more physiological symptoms associated with the underlying disorder, such that an improvement is observed in the subject, although the subject may still be afflicted with the underlying disorder.

As used herein, "preventing" and "prevention" may be used interchangeably. These terms refer to methods of obtaining a beneficial or desired result, including but not limited to prophylactic benefit. For "prophylactic benefit," a conjugate or composition may be administered to a subject at risk of developing a particular disease, or to a subject reporting one or more pathological symptoms of the disease, even if a diagnosis of the disease may not have been made.

According to the first aspect of the present invention, a compound is provided having the structure shown in Formula (I): 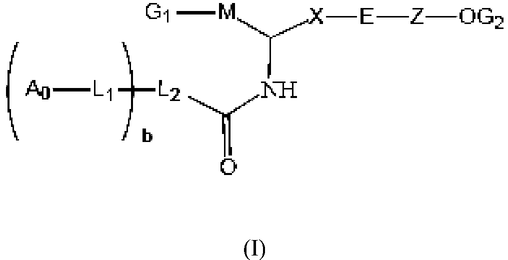 wherein,
*A*₀ represents a ligand having affinity for the asialoglycoprotein receptor on the surface of mammalian liver cells, or represents a group formed by replacing all or part of the hydroxyl groups in said ligand with a KCOO- group, wherein each K is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl;
wherein said ligand is preferably independently selected from any one of the following: D-mannopyranose, L-mannopyranose, D-arabinose, D-xylofuranose, L-xylofuranose, D-glucose, L-glucose, D-galactose, L-galactose, *α*-D-mannofuranose, *β*-D-mannofuranose, *α*-D-mannopyranose, *β-*D-mannopyranose, *α*-D-glucopyranose, *β*-D-glucopyranose, *α*-D-glucofuranose, *β*-D-glucofuranose, *α*-D-fructofuranose, *α*-D-fructopyranose, *α*-D-galactopyranose, *β*-D-galactopyranose, *α*-D-galactofuranose, *β*-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-*β*-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-*α*-neuraminic acid, 5-thio-*β*-D-glucopyranose, methyl 2,3,4-tri-O-acetyl-1-thio-6-O-trityl-*α*-D-glucopyranoside, 4-thio-*β*-D-galactopyranose, ethyl 3,4,6,7-tetra-O-acetyl-2-deoxy-1,5-dithio-*α*-D-glucoheptopyranoside, 2,5-anhydro-D-allononitrile, ribose, D-ribose, D-4-thioribose, L-ribose, L-4-thioribose;
b is an integer ranging from 1 to 4, preferably 1, 2, or 3, more preferably 1 or 3;
*L*₁ and *L*₂ independently represent a linking combination of one or more groups selected from Formula A1-A11: 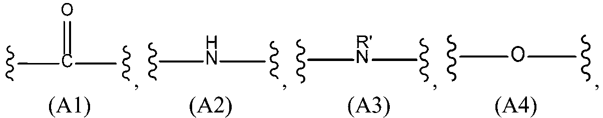 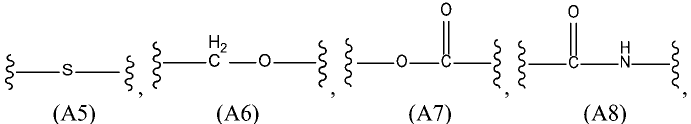 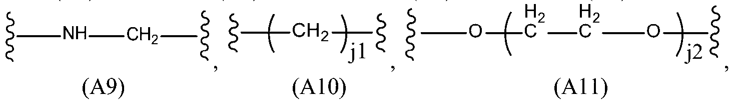
wherein, j1 is an integer from 1-20; j2 is an integer from 1-20; R' is a *C*₁ - *C*₁₀ alkyl; M represents a structural formula as shown in Formula (A12): 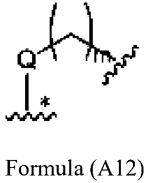
wherein, m represents an integer from 0-6, preferably 0, 1, or 2; * represents the bond connected to *G*₁, Q represents a structural formula as shown in Formula (A13): 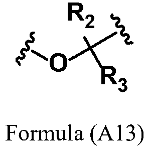
wherein, 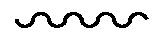 connected to O represents the bond connected to *G*₁; *R*₂, *R*₃ are the same or different, and are independently selected from H, *C*₁ - *C*₂₀ alkyl, *C*₁ - *C*₂₀ alkoxy, *C*₂ - *C*₂₀ alkenyl, *C*₂ - *C*₂₀ or alkynyl, preferably H, *C*₁ - *C*₆ alkyl, *C*₁ - *C*₆ alkoxy, *C*₂ - *C*₆ alkenyl, or *C*₂ - *C*₆ alkynyl;
Z is absent or represents a *C*₁ - *C*₁₀ alkylene, preferably a *C*₁ - *C*₃ alkylene;
X represents a structural formula as shown in Formula (A14-1) or (A14-2): 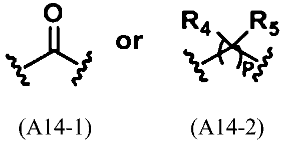
wherein *R*₄, *R*₅ are the same or different, and are independently selected from H, fluoro, hydroxyl, *C*₁ - *C*₂₀ alkyl, *C*₁ - *C*₂₀ alkoxy, *C*₂ - *C*₂₀ alkenyl, or *C*₂ - *C*₂₀ alkynyl, preferably H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is an integer from 1-6, preferably 1, 2, or 3; optionally, *R*₄ and *R*₅ are directly connected to form a three- to six-membered saturated carbocyclic ring;
E represents a structural formula as shown in Formula (A15): 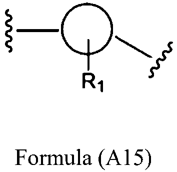
wherein, 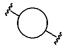 represents a *C*₃ - *C*₁₈ cycloalkyl or a *C*₃ - *C*₁₈ heterocyclyl, preferably represents a *C*₄ - *C*₈ cycloalkyl or a *C*₃ - *C*₈ heterocyclyl, more preferably represents a *C*₃ - *C*₆ heterocyclyl, and even more preferably a four- to six-membered nitrogen-containing saturated cycloalkyl, and *R*₁ is selected from H, fluoro, hydroxyl, cyano, *C*₁ - *C*₂₀ alkyl, *C*₁ - *C*₂₀ alkoxy, *C*₂ - *C*₂₀ alkenyl, or *C*₂ - *C*₂₀ alkynyl, preferably selected from H, fluoro, hydroxyl, cyano, *C*₁ - *C*₆ alkyl, *C*₁ - *C*₆ alkoxy, *C*₂ - *C*₆ alkenyl, or *C*₂ - *C*₆ alkynyl;
*G*₁ and *G*₂ each represent a phosphoramidite functional group having the structure shown in Formula (G-1) or a hydroxyl protecting group, provided that at least one of *G*₁ and *G*₂ has the structure shown in Formula (G-1), said hydroxyl protecting group is selected from any one of trityl, 4-methoxytrityl, 4,4'-dimethoxytrityl (DMTr), and 4,4',4''-trimethoxytrityl, preferably 4,4'-dimethoxytrityl; 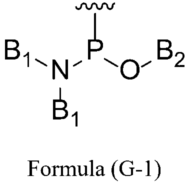
wherein, 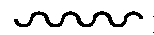 represents the site of covalent bond attachment of the group;
*B*₁ is selected from substituted or unsubstituted *C*₁ - *C*₅ hydrocarbyl, optionally, *B*₁ is methyl, ethyl, or isopropyl; *B*₂ is selected from one of *C*₁ - *C*₅ alkyl, cyanoethyl, cyanopropyl, or cyanobutyl, optionally, *B*₂ is cyanoethyl.

In some embodiments, *L*₁ and *L*₂ are independently selected from a linking combination of one or more of the following: A1, A2, A4, A6, A7, A8, A9, and A10. In some embodiments, *L*₁ and *L*₂ are independently selected from a linking combination of at least 2 of A1, A2, A4, A8, A9, and A10. In some embodiments, wherein *L*₁ and *L*₂ are independently selected from a linking combination of at least 2 of A1, A4, A8, and A10. In some embodiments, the length of *L*₁ or *L*₂ is independently 1-25 atoms, wherein the length of *L*₁ or *L*₂ refers to the number of chain-forming atoms in the longest straight chain. In some embodiments, the length of *L*₁ or *L*₂ is independently 1-20 atoms.

In some embodiments, each j1 is independently an integer from 1-10, and each j2 is independently an integer from 1-10.

In some embodiments, each j1 is independently an integer from 1-8, and each j2 is independently an integer from 1-8.

In some embodiments, *A*₀ is an N-acetylgalactosamine (GalNAc) group as shown below or a group formed by replacing all or part of its hydroxyl groups with a KCOO- group, wherein each K is independently selected from methyl, trifluoromethyl, difluoromethyl, monofluoromethyl, trichloromethyl, dichloromethyl, monochloromethyl, ethyl, n-propyl, isopropyl, phenyl, halophenyl, or alkylphenyl:

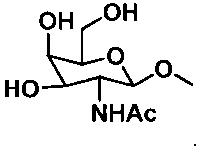

In some embodiments, said compound has the structure (I-1) shown below:

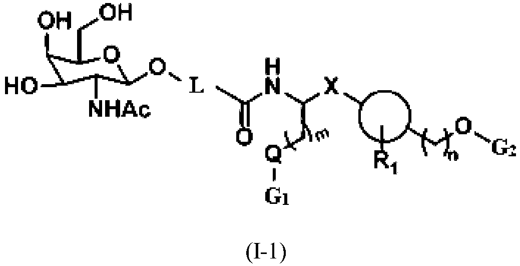

In some embodiments, L is A10, wherein j1 is an integer from 2-8, or is A11, wherein j2 is an integer from 1-7.

In some embodiments, m is 0, 1, or 2.

In some embodiments, n is 0, 1, 2, or 3.

In some embodiments, 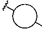 represents a four- to eight-membered all-carbon or nitrogen-containing saturated ring. In some embodiments, 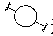 is a four-, five-, or six-membered saturated cycloalkyl containing one or two nitrogen atoms.

In a specific embodiment, the compound of the present disclosure has any one of the structures shown below:

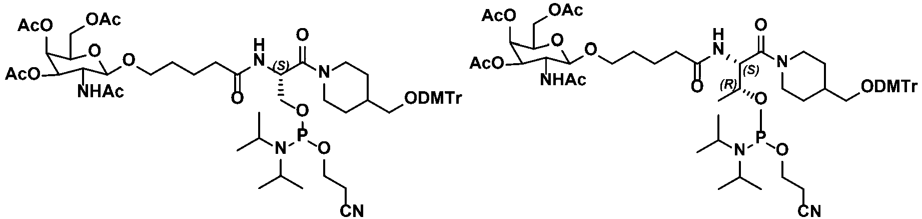

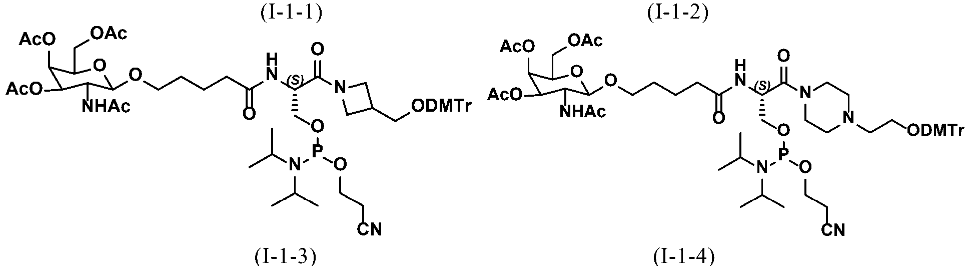

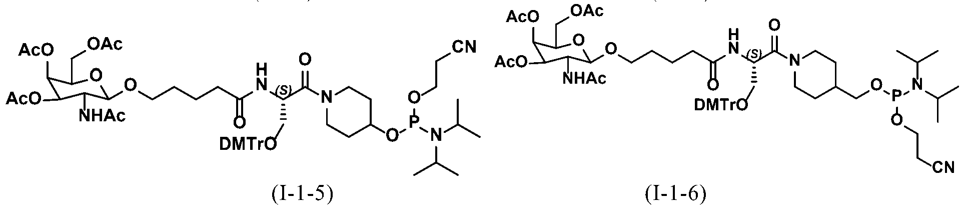

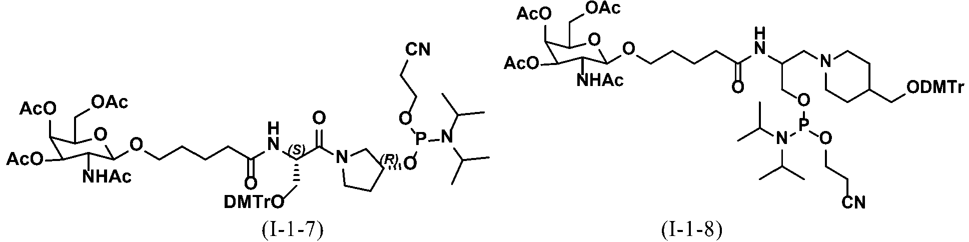

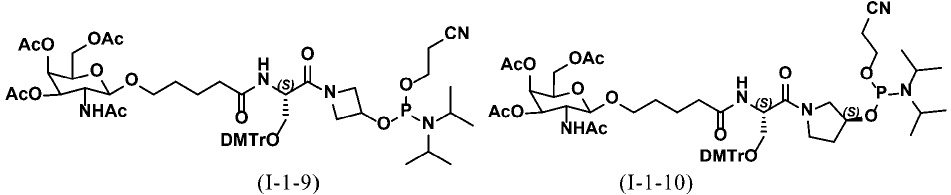

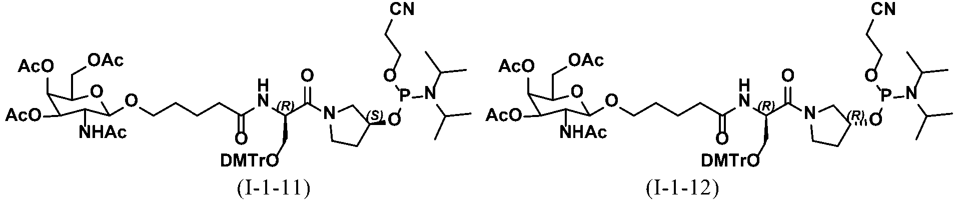

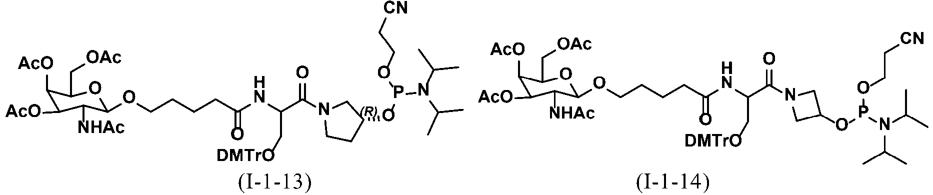

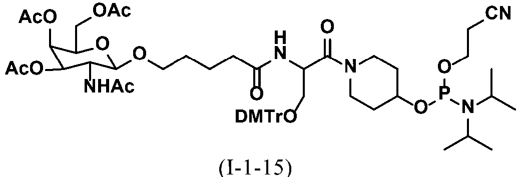

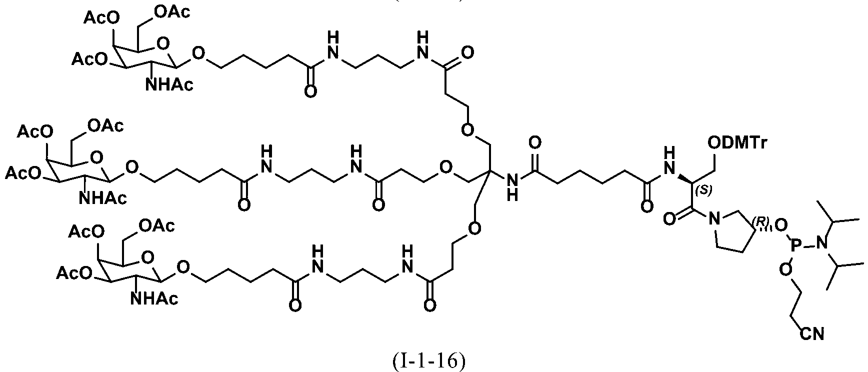

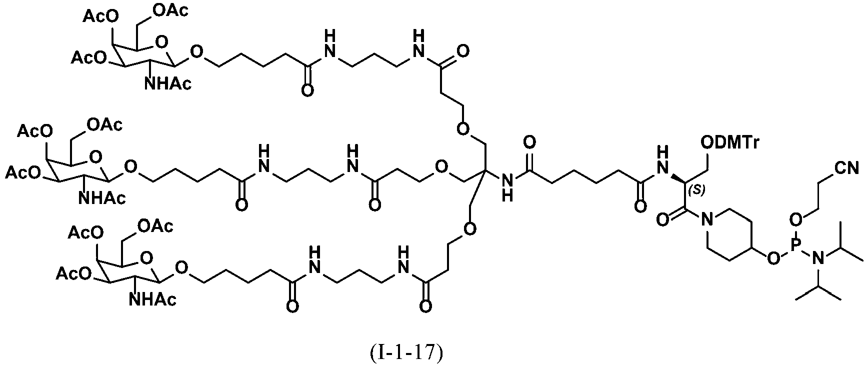

According to the second aspect of the present invention, a nucleic acid conjugate is provided, wherein the conjugate contains one or more structures as shown in Formula (II) connected to any position on an oligonucleotide sequence, preferably two, three, or four consecutively connected structures as shown in Formula (II): 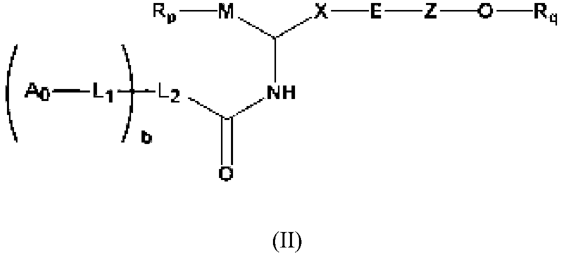
wherein, *Rₚ* and *R_{q}* are each H or have the structure shown in Formula A16, provided that at least one of *Rₚ* and *R_{q}* has the structure shown in Formula A16; 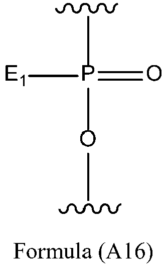 wherein, *E*₁ is OH, SH, or *BH*₂,
*A*₀ represents a ligand having affinity for the asialoglycoprotein receptor on the surface of mammalian liver cells, or represents a group formed by replacing all or part of the hydroxyl groups in said ligand with a KCOO- group, wherein each K is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl;
wherein said ligand is preferably independently selected from any one of the following: D-mannopyranose, L-mannopyranose, D-arabinose, D-xylofuranose, L-xylofuranose, D-glucose, L-glucose, D-galactose, L-galactose, *α*-D-mannofuranose, *β*-D-mannofuranose, *α*-D-mannopyranose, *β-*D-mannopyranose, *α*-D-glucopyranose, *β*-D-glucopyranose, *α*-D-glucofuranose, *β*-D-glucofuranose, *α*-D-fructofuranose, *α*-D-fructopyranose, *α*-D-galactopyranose, *β*-D-galactopyranose, *α*-D-galactofuranose, *β*-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-*β*-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-*α*-neuraminic acid, 5-thio-*β*-D-glucopyranose, methyl 2,3,4-tri-O-acetyl-1-thio-6-O-trityl-*α*-D-glucopyranoside, 4-thio-*β*-D-galactopyranose, ethyl 3,4,6,7-tetra-O-acetyl-2-deoxy-1,5-dithio-*α*-D-glucoheptopyranoside, 2,5-anhydro-D-allononitrile, ribose, D-ribose, D-4-thioribose, L-ribose, L-4-thioribose;
b is an integer ranging from 1 to 4, preferably 1, 2, or 3, more preferably 1 or 3;
*L*₁ and *L*₂ independently represent a linking combination of one or more groups selected from Formula A1-A11: 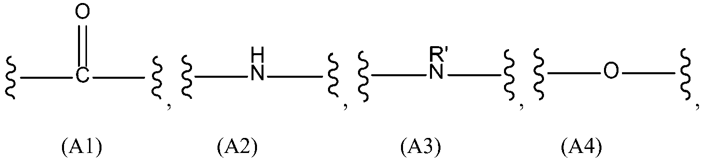 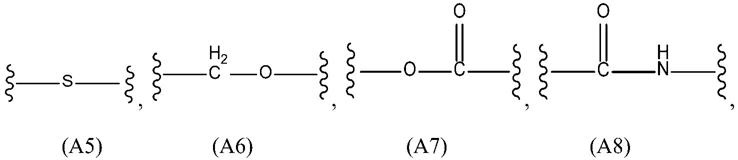 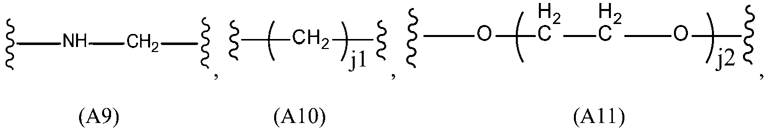
wherein, j1 is an integer from 1-20;
j2 is an integer from 1-20;
R' is a *C*₁ - *C*₁₀ alkyl;
M represents a structural formula as shown in Formula (A12): 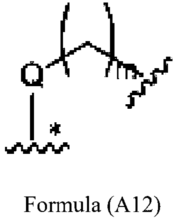
wherein, m represents an integer from 0-6, preferably 0, 1, or 2; * represents the bond connected to *Rₚ*, Q represents a structural formula as shown in Formula (A13): 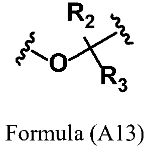
wherein, 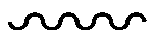 connected to O represents the bond connected to *Rₚ*; *R*₂, *R*₃ are the same or different, and are independently selected from H, *C*₁ - *C*₂₀ alkyl, *C*₁ - *C*₂₀ alkoxy, *C*₂ - *C*₂₀ alkenyl, or *C*₂ - *C*₂₀ alkynyl, preferably H, *C*₁ - *C*₆ alkyl, *C*₁ - *C*₆ alkoxy, *C*₂ - *C*₆ alkenyl, or *C*₂ - *C*₆ alkynyl;
Z is absent or represents a *C*₁ - *C*₁₀ alkylene, preferably a *C*₁ - *C*₃ alkylene;
X represents a structural formula as shown in Formula (A14-1) or (A14-2): 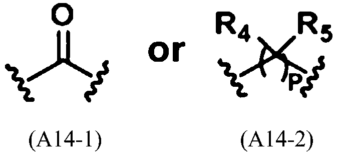
wherein *R*₄, *R*₅ are the same or different, and are independently selected from H, fluoro, hydroxyl, *C*₁ - *C*₂₀ alkyl, *C*₁ - *C*₂₀ alkoxy, *C*₂ - *C*₂₀ alkenyl, or *C*₂ - *C*₂₀ alkynyl, preferably H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is an integer from 1-6, preferably 1, 2, or 3; optionally, *R*₄ and *R*₅ are directly connected to form a three- to six-membered saturated carbocyclic ring;
E represents a structural formula as shown in Formula (A15): 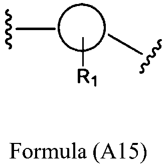
wherein, 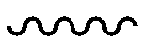 represents the site of covalent bond attachment of the group; 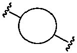 represents a *C*₃ - *C*₁₈ cycloalkyl or a *C*₃ - *C*₁₈ heterocyclyl, preferably represents a *C*₄ - *C*₈ cycloalkyl or a *C*₃ - *C*₈ heterocyclyl, more preferably represents a *C*₃ - *C*₆ heterocyclyl, and even more preferably a four- to six-membered nitrogen-containing saturated cycloalkyl, and *R*₁ is selected from H, fluoro, hydroxyl, cyano, *C*₁ - *C*₂₀ alkyl, *C*₁ - *C*₂₀ alkoxy, *C*₂ - *C*₂₀ alkenyl, or *C*₂ - *C*₂₀ alkynyl, preferably selected from H, fluoro, hydroxyl, cyano, *C*₁ - *C*₆ alkyl, *C*₁ - *C*₆ alkoxy, *C*₂ - *C*₆ alkenyl, or *C*₂ - *C*₆ alkynyl.

In a third aspect, the present invention provides a nucleic acid conjugate, wherein said conjugate contains one or more structures as shown in Formula (II-A) connected to any position on an oligonucleotide sequence: 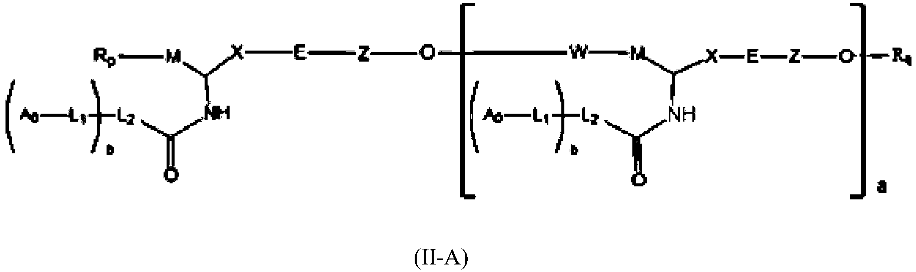 wherein,
a represents an integer from 0-7, preferably an integer from 0-3, more preferably an integer of 0, 1, 2, or 3;
*Rₚ* and *R_{q}* are each H or have the structure shown in Formula A16, and wherein at least one has the structure shown in Formula A16;

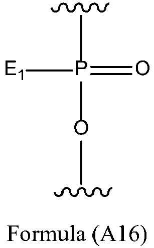

The structure of W is as shown in the following formula: 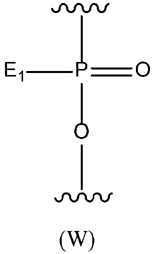
wherein, *E*₁ is OH, SH, or *BH*₂,
*A*₀, b, *L*₁, *L*₂, M, Z, X, E are the same as in Formula (II), and are not repeated here.

In some embodiments, *L*₁ and *L*₂ are independently selected from a linking combination of one or more of the following: A1, A2, A4, A6, A7, A8, A9, and A10. In some embodiments, *L*₁ and *L*₂ are independently selected from a linking combination of at least 2 of A1, A2, A4, A8, A9, and A10. In some embodiments, wherein *L*₁ and *L*₂ are independently selected from a linking combination of at least 2 of A1, A4, A8, and A10. In some embodiments, the length of *L*₁ or *L*₂ is independently 1-25 atoms, wherein the length of *L*₁ or *L*₂ refers to the number of chain-forming atoms in the longest straight chain. In some embodiments, the length of *L*₁ or *L*₂ is independently 1-20 atoms.

In some embodiments, each j1 is independently an integer from 1-10, and each j2 is independently an integer from 1-10.

In some embodiments, each j1 is independently an integer from 1-8, and each j2 is independently an integer from 1-8.

In some embodiments, *A*₀ is an N-acetylgalactosamine (GalNAc) group as shown below or a group formed by replacing all or part of its hydroxyl groups with a KCOO- group, wherein each K is independently selected from methyl, trifluoromethyl, difluoromethyl, monofluoromethyl, trichloromethyl, dichloromethyl, monochloromethyl, ethyl, n-propyl, isopropyl, phenyl, halophenyl, or alkylphenyl:

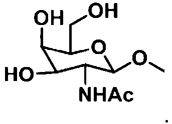

In the context of the present disclosure, unless otherwise specified, "conjugation" refers to the connection of two or more chemical moieties, each having a specific function, to each other in a covalent manner; accordingly, a "conjugate" refers to a compound formed by the covalent connection between these individual chemical moieties. Furthermore, a "nucleic acid conjugate" represents a compound formed by covalently connecting one or more chemical moieties with specific functions to an oligonucleotide. Hereinafter, the nucleic acid conjugate of the present disclosure is also sometimes referred to simply as a "conjugate."

In a specific embodiment of the present invention, the nucleic acid conjugate of the present invention has any one of the structures shown below: 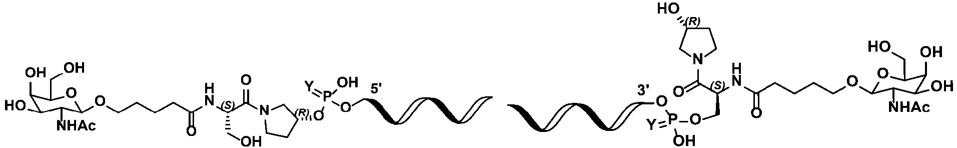 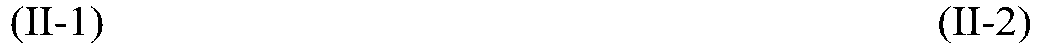 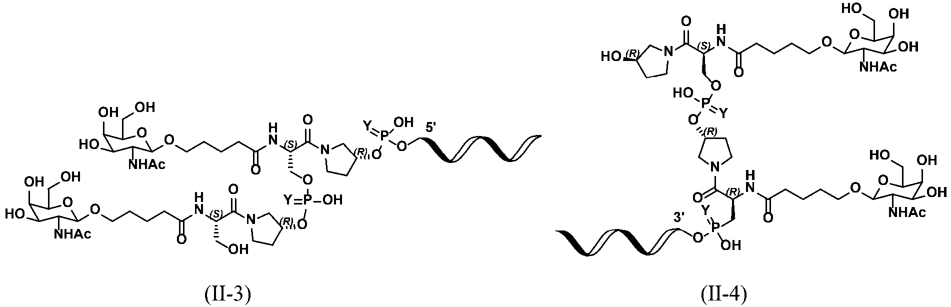 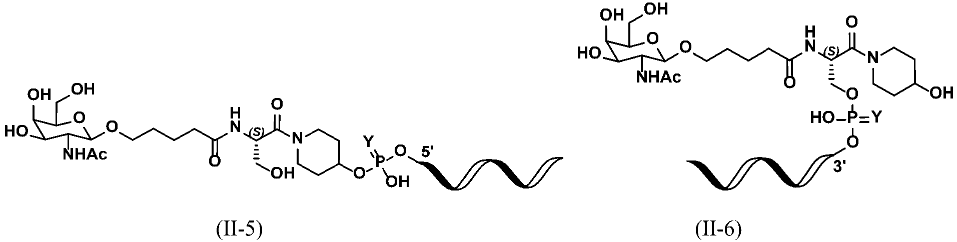 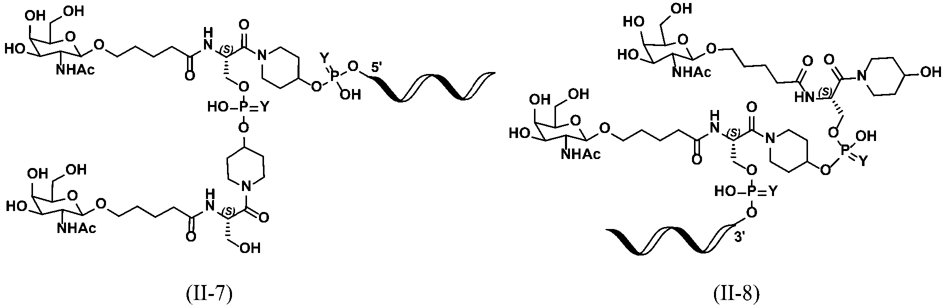 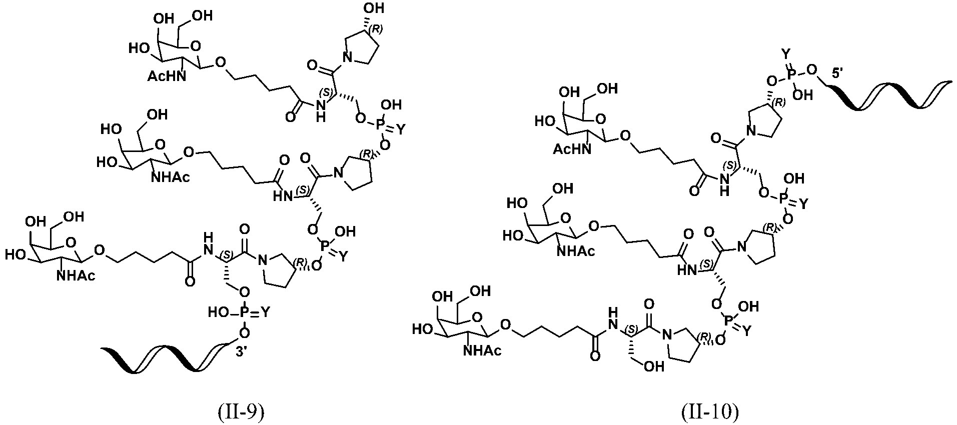 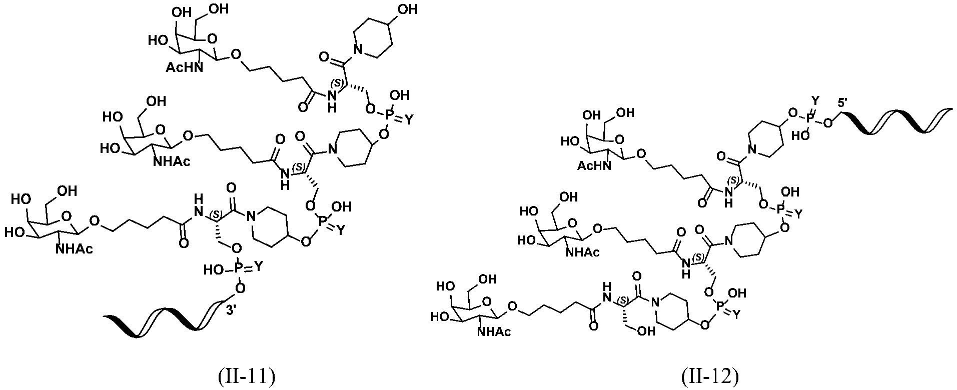 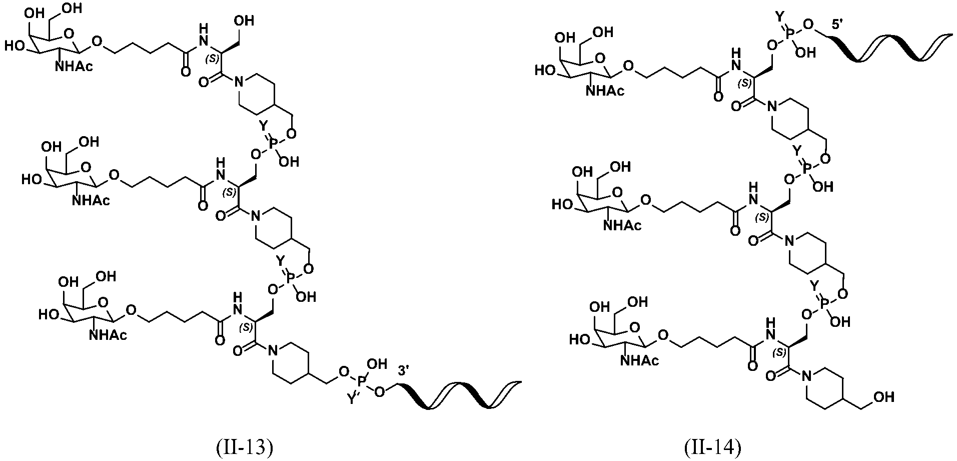 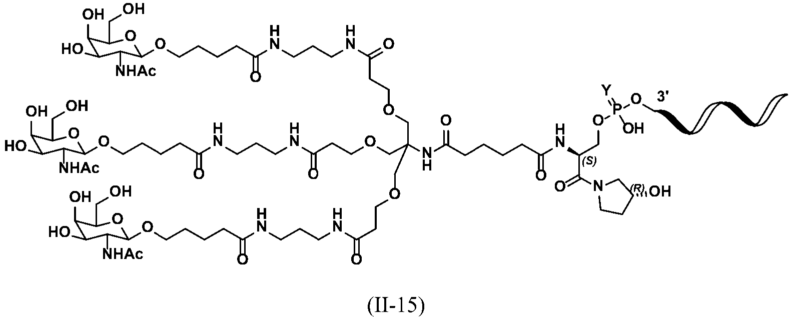 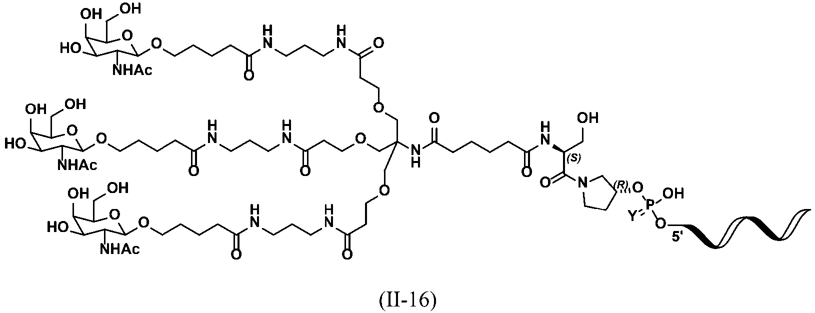 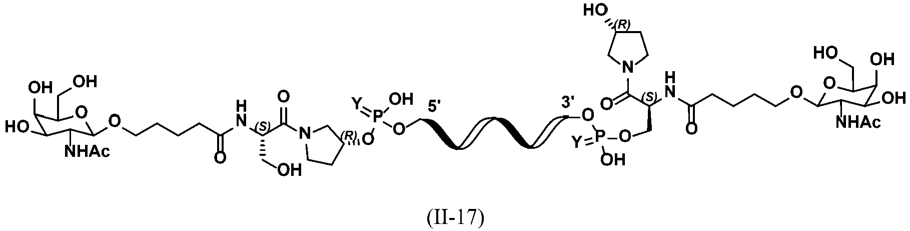 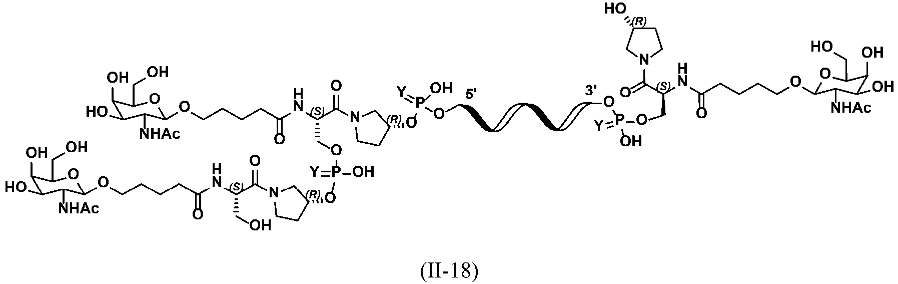 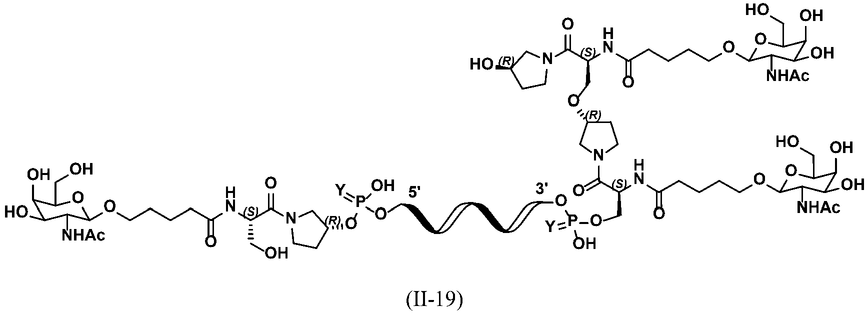 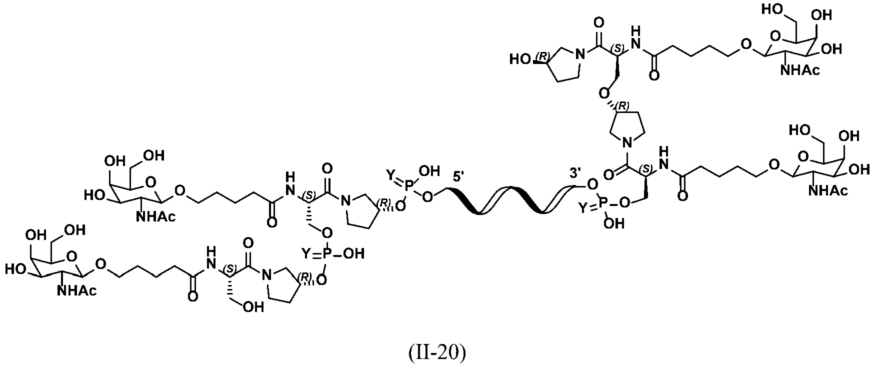 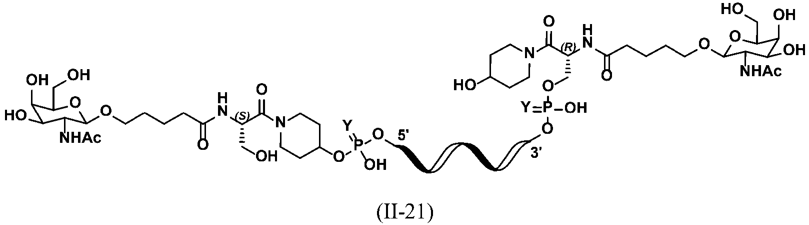 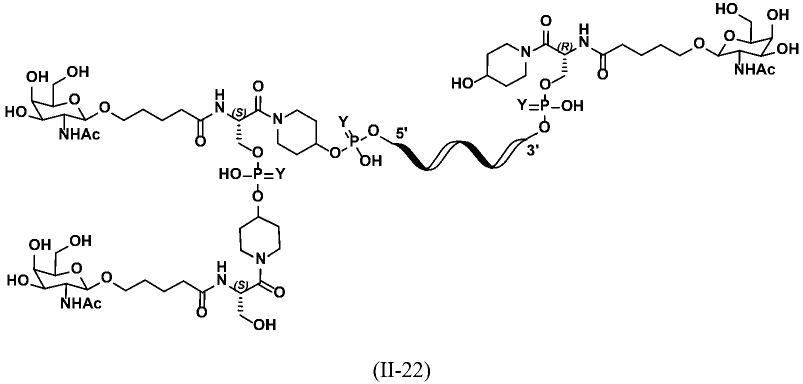 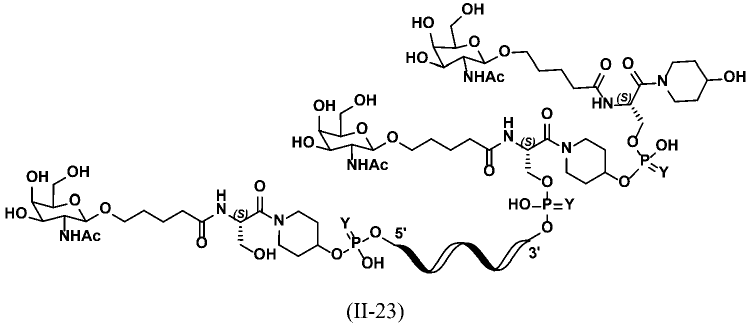 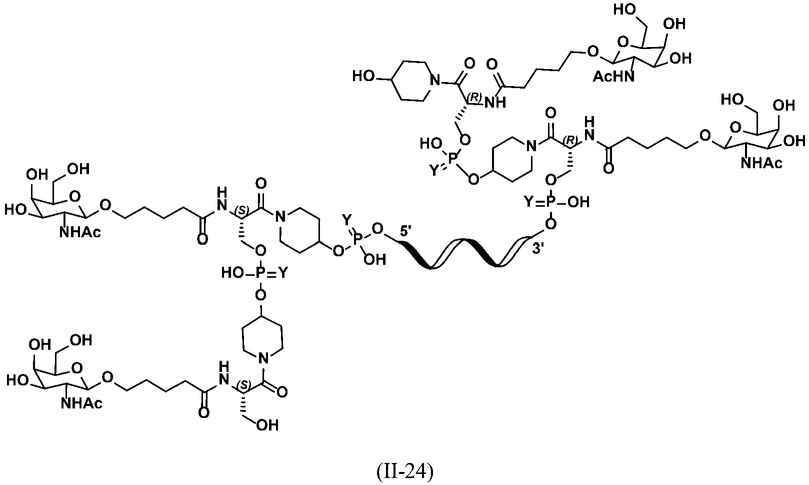 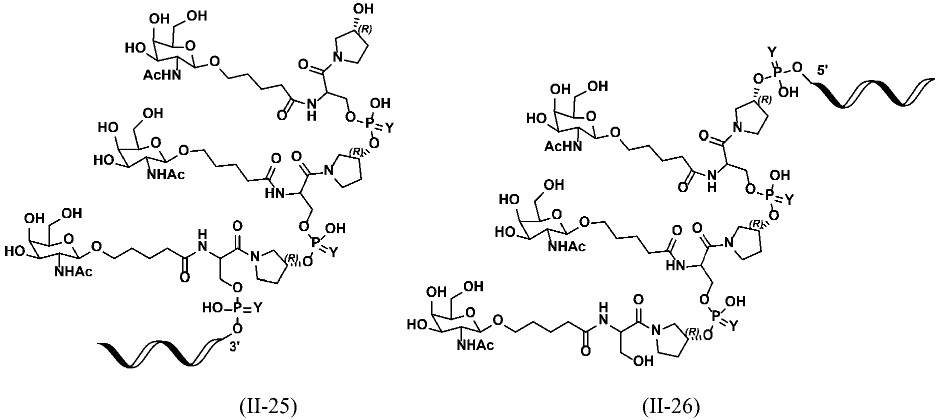 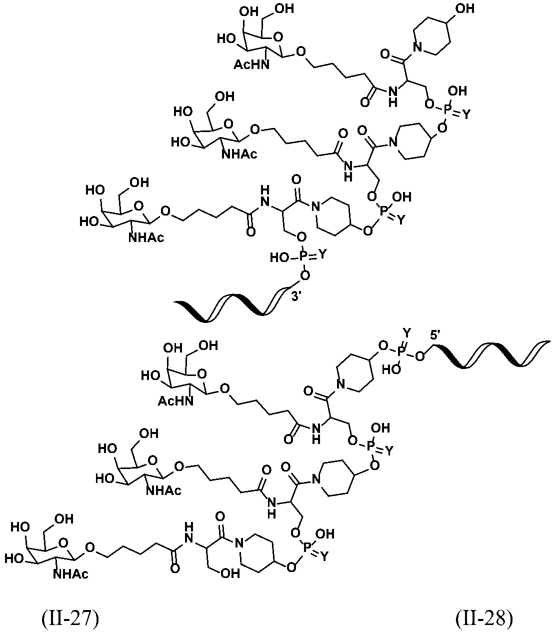 wherein Y is O or S, 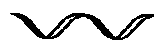 is an oligonucleotide.

In some embodiments of the present disclosure, said oligonucleotide is selected from one of the following: small interfering RNA, microRNA, anti-microRNA, microRNA antagonist, microRNA mimetic, decoy oligonucleotide, immunostimulant, G-quadruplex, splice variant, single-stranded RNA, antisense nucleic acid, nucleic acid aptamer, stem-loop RNA, mRNA fragment, and activating RNA; optionally, said oligonucleotide is a single-stranded oligonucleotide or a double-stranded oligonucleotide; optionally, said oligonucleotide is a single-stranded oligonucleotide, the P atom in Formula (A16) is connected to an end of said single-stranded oligonucleotide, said end of the single-stranded oligonucleotide refers to the first 4 nucleotides from one end of said single-stranded oligonucleotide; optionally, the P atom in Formula (A16) is connected to a terminus of said single-stranded oligonucleotide; optionally, the P atom in Formula (A16) is connected to the 3' terminus of said single-stranded oligonucleotide;
optionally, said oligonucleotide is a double-stranded oligonucleotide, said double-stranded oligonucleotide comprises a sense strand and an antisense strand, the P atom in said Formula (A16) is connected to an end of said double-stranded oligonucleotide, said end of the double-stranded oligonucleotide refers to the first 4 nucleotides from one end of said sense strand or said antisense strand; optionally, the P atom in Formula (A16) is connected to a terminus of said sense strand or said antisense strand; optionally, the P atom in Formula (A16) is connected to the 5' terminus of said antisense strand; optionally, the P atom in Formula (A16) is connected to the 2', 3', or 5' position of a nucleotide in said nucleic acid conjugate by forming a phosphodiester bond.

In some embodiments, the "target sequence" is a target mRNA. In the context of the present disclosure, "target mRNA" refers to an mRNA corresponding to a gene that is abnormally expressed in hepatocytes, which can be either an mRNA corresponding to an overexpressed gene or an mRNA corresponding to an underexpressed gene. Since most diseases originate from the overexpression of mRNA, in the present disclosure, target mRNA especially refers to mRNA corresponding to an overexpressed gene. In some embodiments of the present disclosure, corresponding to the above-mentioned abnormally expressed genes, said target mRNA may be mRNA corresponding to genes such as ApoB, ApoC, ANGPTL3, PCSK9, SCD1, TIMP-1, Col1A1, FVII, STAT3, p53, HBV, HCV, etc. In some embodiments, said target mRNA may be an mRNA transcribed from the corresponding HBV gene, or the mRNA corresponding to the ANGPTL3 gene, or the mRNA corresponding to the APOC3 gene.

It is well known to those skilled in the art that siRNA contains nucleotide groups as basic structural units, said nucleotide groups containing a phosphate group, a ribose group, and a base. Generally, an active, i.e., functional, siRNA has a length of about 12-40 nucleotides, and in some embodiments about 15-30 nucleotides, wherein each nucleotide in said siRNA can independently be a modified or unmodified nucleotide, and to increase stability, at least one nucleotide in said siRNA is a modified nucleotide.

The inventors of the present disclosure have found that the siRNAs described in the following embodiments have high activity and/or stability, and thus can serve as the inventive objective of the siRNA in the present disclosure.

In some embodiments, each nucleotide in the siRNA of the siRNA conjugate of the present disclosure (hereinafter, also referred to as the siRNA of the present disclosure) is independently a modified or unmodified nucleotide, said siRNA contains a sense strand and an antisense strand, wherein said sense strand comprises nucleotide sequence 1, said antisense strand comprises nucleotide sequence 2, the lengths of said nucleotide sequence 1 and said nucleotide sequence 2 are both 10, 11, 12, 13, 14, 15, 16, 17, 18, 19, 20, 21, 22, 23, 24, or 25 nucleotides, and are at least partially reverse complementary to form a complementary duplex region, at least a portion of said nucleotide sequence 2 is complementary to a first nucleotide sequence segment, said first nucleotide sequence segment being a nucleotide sequence segment in a target mRNA.

In some embodiments, said nucleotide sequence 1 is equal in length to said first nucleotide sequence segment, with no more than 3 nucleotide differences; said nucleotide sequence 2 is equal in length to nucleotide sequence B, with no more than 3 nucleotide differences; said nucleotide sequence B is a nucleotide sequence that is completely reverse complementary to said first nucleotide sequence segment. Without wishing to be bound, these specific nucleotide differences do not significantly reduce the target gene inhibition ability of the siRNA conjugate, and these siRNA conjugates containing specific nucleotide differences are also within the protection scope of the present disclosure.

In some embodiments, said nucleotide sequence 1 and said nucleotide sequence 2 are substantially reverse complementary, substantially fully reverse complementary, or fully reverse complementary.

In some embodiments, said nucleotide sequence 1 has no more than 1 nucleotide difference from said first nucleotide sequence segment, and/or said nucleotide sequence 2 has no more than 1 nucleotide difference from said nucleotide sequence B. In some embodiments, the nucleotide difference between said nucleotide sequence 2 and said nucleotide sequence B includes a difference at the position of the first nucleotide Z' on said nucleotide sequence 2, in the 5' to 3' direction. In some embodiments, in the 5' to 3' direction, the last nucleotide Z on said nucleotide sequence 1 is a nucleotide complementary to Z'. In some embodiments, said sense strand further contains nucleotide sequence 3, said antisense strand further contains nucleotide sequence 4, said nucleotide sequence 3 and said nucleotide sequence 4 have equal lengths of 1-4 nucleotides, said nucleotide sequence 3 is connected to the 5' end of said nucleotide sequence 1, and said nucleotide sequence 4 is connected to the 3' end of said nucleotide sequence 2, said nucleotide sequence 4 is complementary to a second nucleotide sequence segment, which refers to the nucleotide sequence in the target mRNA that is adjacent to said first nucleotide sequence segment and has the same length as said nucleotide sequence 4. In some embodiments, said nucleotide sequence 3 and said nucleotide sequence 4 are substantially fully reverse complementary or fully reverse complementary. Therefore, the length of the sense strand and the antisense strand can be 19-23 nucleotides.

In some embodiments, the siRNA of the present disclosure further contains nucleotide sequence 5, the length of said nucleotide sequence 5 is 1 to 3 nucleotides, connected to the 3' end of said antisense strand, thereby forming a 3' overhang of said antisense strand; in some embodiments, the length of said nucleotide sequence 5 is 1 or 2 nucleotides. Thus, in some embodiments, the ratio of the lengths of the sense strand to the antisense strand of the siRNA of the present disclosure can be 19/20, 19/21, 20/21, 20/22, 21/22, 21/23, 22/23, 22/24, 23/24, or 23/25.

In one embodiment, the length of said nucleotide sequence 5 is 2 nucleotides, and in the 5' to 3' direction, said nucleotide sequence 5 is two consecutive deoxythymidine nucleotides, two consecutive uridine nucleotides, or is complementary to a third nucleotide sequence segment, said third sequence refers to the nucleotide sequence in the target mRNA that is adjacent to said first nucleotide sequence segment, or adjacent to said second nucleotide sequence segment, and has a length equal to said nucleotide sequence 5. In one embodiment, the ratio of the lengths of the sense strand to the antisense strand of the siRNA of the present disclosure is 19/21 or 21/23, at which point the siRNA of the present disclosure exhibits better hepatocyte mRNA silencing activity.

In some embodiments, the nucleotides in the siRNA of the present disclosure are each independently a modified or unmodified nucleotide. In some embodiments, the siRNA of the present disclosure does not contain modified nucleotide groups; in some embodiments, the siRNA of the present disclosure contains modified nucleotide groups.

Currently, there are various ways available in the art to modify siRNAs, including backbone modifications (also known as internucleotide linkage modifications, such as phosphate group modifications), ribose group modifications, and base modifications (for example, see Watts, J.K., G.F. Deleavey and M.J. Damha, Chemically modified siRNA: tools and applications. Drug Discov Today, 2008. 13(19-20): p. 842-55, the entire content of which is incorporated herein by reference).

In the context of the present disclosure, the term "modified nucleotide" as used refers to a nucleotide in which the ribose group of the nucleotide is modified, such as a nucleotide or nucleotide analog formed by replacing the 2' hydroxyl group with another group, or a nucleotide in which the base on the nucleotide is a modified base.

In some embodiments of the present disclosure, at least one nucleotide in said sense strand or said antisense strand is a modified nucleotide, and/or at least one phosphate group is a phosphate group with a modifying group, in other words, at least a part of the phosphate groups and/or ribose groups in the phosphate-sugar backbone of at least one single strand of said sense strand and said antisense strand are phosphate groups with modifying groups and/or ribose groups with modifying groups (or modified phosphate groups and/or modified ribose groups). In some embodiments of the present disclosure, all nucleotides in said sense strand and/or said antisense strand are modified nucleotides.

In some embodiments, each nucleotide in the sense and antisense strands is independently a fluoro-modified nucleotide or a non-fluoro-modified nucleotide.

A fluoro-modified nucleotide refers to a nucleotide formed by replacing the 2'-position hydroxyl group of the ribose of a nucleotide with fluorine, having the structure shown in Formula (17) below.

A non-fluoro-modified nucleotide refers to a nucleotide or nucleotide analog formed by replacing the 2'-position hydroxyl group of the ribose of a nucleotide with a non-fluoro group. In some embodiments, each non-fluoro-modified nucleotide is independently selected from one of the nucleotides or nucleotide analogs formed by replacing the 2'-position hydroxyl group of the ribose of a nucleotide with a non-fluoro group.

Nucleotides formed by replacing the 2'-position hydroxyl group of the ribose with a non-fluoro group are well known to those skilled in the art. These nucleotides may be selected from one of 2'-alkoxy-modified nucleotides, 2'-substituted alkoxy-modified nucleotides, 2'-alkyl-modified nucleotides, 2'-substituted alkyl-modified nucleotides, 2'-amino-modified nucleotides, 2'-substituted amino-modified nucleotides, and 2'-deoxyribonucleotides.

In some embodiments, the 2'-alkoxy-modified nucleotide is a methoxy-modified nucleotide (2'-OMe), as shown in Formula (18). A 2'-substituted alkoxy-modified nucleotide, for example, can be a 2'-O-methoxyethyl-modified nucleotide (2'-MOE), as shown in Formula (19). In some embodiments, a 2'-amino-modified nucleotide (2' - *NH*₂) is as shown in Formula (20). In some embodiments, a 2'-deoxyribonucleotide (DNA) is as shown in Formula (21).

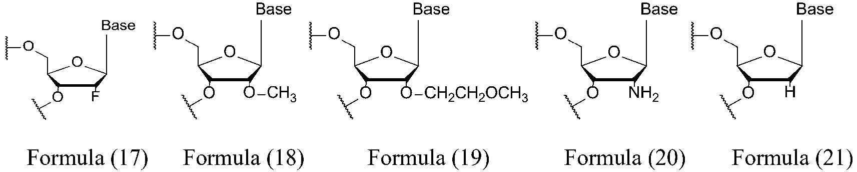

A nucleotide analog refers to a group that can replace a nucleotide in a nucleic acid but has a structure different from adenosine ribonucleotide, guanosine ribonucleotide, cytidine ribonucleotide, uridine ribonucleotide, or thymidine. In some embodiments, said nucleotide analog may be, for example, an isonucleotide, a bridged nucleic acid (BNA) nucleotide, or an acyclic nucleotide. BNA nucleotides are constrained or inaccessible nucleotides. BNA can contain a five-, six-, or seven-membered ring with a "locked" C3'-endo sugar pucker bridge structure. This bridge is typically incorporated at the 2'- and 4'-positions of the ribose ring to provide a 2', 4'-BNA nucleotide, such as LNA, ENA, cET BNA, etc., wherein LNA is as shown in Formula (22), ENA is as shown in Formula (23), and cET BNA is as shown in Formula (24).

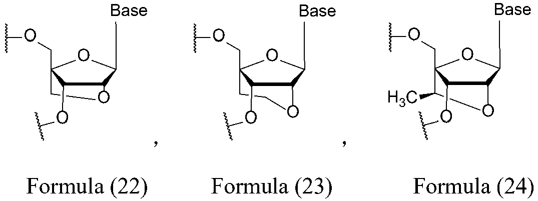

Acyclic nucleotides are a class of nucleotides formed by opening the sugar ring of a nucleotide, such as unlocked nucleic acid (UNA) nucleotides or glycerol nucleic acid (GNA) nucleotides, wherein UNA is as shown in Formula (25) and GNA is as shown in Formula (26). 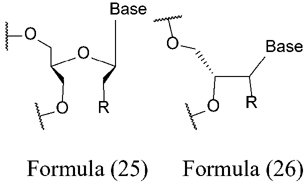 wherein, R is selected from H, OH, or alkoxy (O-alkyl).

Isonucleotides refer to compounds formed by changing the position of the base on the ribose ring in a nucleotide, for example, compounds formed by moving the base from the 1'-position of the ribose ring to the 2'- or 3'-position, as shown in Formula (27) or (28). 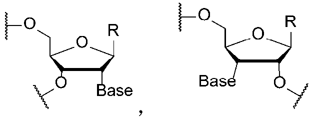 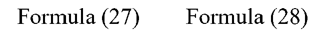 wherein, Base represents a base, for example, A, U, G, C, or T; R is selected from H, OH, F, or a non-fluoro group as described above.

In some embodiments, the nucleotide analog is selected from one of isonucleotide, LNA, ENA, cET, UNA, and GNA. In some embodiments, each non-fluoro-modified nucleotide is a methoxy-modified nucleotide, said methoxy-modified nucleotide referring to a nucleotide formed by replacing the 2'-hydroxyl group of the ribose with a methoxy group.

In the preceding and following text, "fluoro-modified nucleotide," "2'-fluoro-modified nucleotide," "nucleotide with the 2'-hydroxyl of the ribose group substituted by fluorine," and "2'-fluoro ribosyl" have the same meaning, all referring to a compound with the structure as shown in Formula (17) formed by replacing the 2'-hydroxyl group of a nucleotide with fluorine; "methoxy-modified nucleotide," "2'-methoxy-modified nucleotide," "nucleotide with the 2'-hydroxyl of the ribose group replaced by a methoxy group," and "2'-methoxy ribosyl" have the same meaning, all referring to a structure as shown in Formula (18) formed by replacing the 2'-hydroxyl group of the nucleotide ribose group with a methoxy group.

In some embodiments, the siRNA of the present disclosure is an siRNA with the following modifications: both the sense and antisense strands contain fluoro-modified nucleotides and non-fluoro-modified nucleotides, said fluoro-modified nucleotides are located in the aforementioned nucleotide sequence 1 and nucleotide sequence 2, the number of fluoro-modified nucleotides in said nucleotide sequence 1 is no more than 5, and, in the 5' to 3' direction, the nucleotides at positions 7, 8, and 9 of said nucleotide sequence 1 are fluoro-modified nucleotides; the number of fluoro-modified nucleotides in said nucleotide sequence 2 is no more than 7, and, in the 5' to 3' direction, the nucleotides at positions 2, 6, 14, and 16 of said nucleotide sequence 2 are fluoro-modified nucleotides. In some embodiments, the siRNA of the present disclosure is an siRNA with the following modifications: in the 5' to 3' direction, the nucleotides at positions 7, 8, and 9 of said nucleotide sequence 1 in the sense strand of said siRNA are fluoro-modified nucleotides, and the nucleotides at the remaining positions in said sense strand are methoxy-modified nucleotides; in said antisense strand, the nucleotides at positions 2, 6, 14, and 16 of said nucleotide sequence 2 are fluoro-modified nucleotides, and the nucleotides at the remaining positions in said antisense strand are methoxy-modified nucleotides; in some embodiments, the siRNA of the present disclosure is an siRNA with the following modifications: in the 5' to 3' direction, the nucleotides at positions 5, 7, 8, and 9 of said nucleotide sequence 1 in the sense strand of said siRNA are fluoro-modified nucleotides, and the nucleotides at the remaining positions in said sense strand are methoxy-modified nucleotides; in said antisense strand, the nucleotides at positions 2, 6, 8, 9, 14, and 16 of said nucleotide sequence 2 are fluoro-modified nucleotides, and the nucleotides at the remaining positions in said antisense strand are methoxy-modified nucleotides; in some embodiments, the siRNA of the present disclosure is an siRNA with the following modifications: in the 5' to 3' direction, the nucleotides at positions 7, 8, and 9 of said nucleotide sequence 1 in the sense strand of said siRNA are fluoro-modified nucleotides, and the nucleotides at the remaining positions in said sense strand are methoxy-modified nucleotides, and, in the 5' to 3' direction, the nucleotides at positions 2, 6, 14, and 16 of said nucleotide sequence 2 in the antisense strand of said siRNA are fluoro-modified nucleotides, and the nucleotides at the remaining positions in said antisense strand are methoxy-modified nucleotides.

In some specific embodiments of the siRNA of the present disclosure, said nucleotide contains a phosphate group modification. In the context of the present disclosure, the phosphate group modification in one embodiment is a phosphorothioate modification as shown in Formula (11) below, that is, replacing a non-bridging oxygen atom in a phosphodiester bond with a sulfur atom, thereby replacing the phosphodiester bond with a phosphorothioate diester bond. In some embodiments, this modification can stabilize the structure of siRNA and maintain high specificity and high affinity of base pairing.

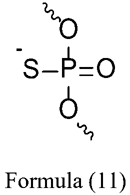

According to some embodiments of the present disclosure, in said siRNA, a phosphorothioate linkage is present at least at one of the locations in the group consisting of: between the first and second nucleotides at either end of the sense or antisense strand; between the second and third nucleotides at either end of the sense or antisense strand; or any combination of the above. In some embodiments, phosphorothioate linkages are present at all of the above locations except for the 5' end of the sense strand. In some embodiments, phosphorothioate linkages are present at all of the above locations except for the 3' end of the sense strand. In some embodiments, a phosphorothioate linkage is present at least at one of the following positions:
the linkage between the 1st and 2nd nucleotides from the 5' end of said sense strand;
the linkage between the 2nd and 3rd nucleotides from the 5' end of said sense strand;
the linkage between the 1st and 2nd nucleotides from the 3' end of said sense strand;
the linkage between the 2nd and 3rd nucleotides from the 3' end of said sense strand;
the linkage between the 1st and 2nd nucleotides from the 5' end of said antisense strand;
the linkage between the 2nd and 3rd nucleotides from the 5' end of said antisense strand;
the linkage between the 1st and 2nd nucleotides from the 3' end of said antisense strand; and
the linkage between the 2nd and 3rd nucleotides from the 3' end of said antisense strand.

According to some embodiments of the present disclosure, the 5'-terminal nucleotide of the antisense strand sequence of said siRNA molecule is a 5'-phosphate nucleotide or a 5'-phosphate analog-modified nucleotide.

In some embodiments, a 5'-phosphate nucleotide can have the structure shown in Formula (12):

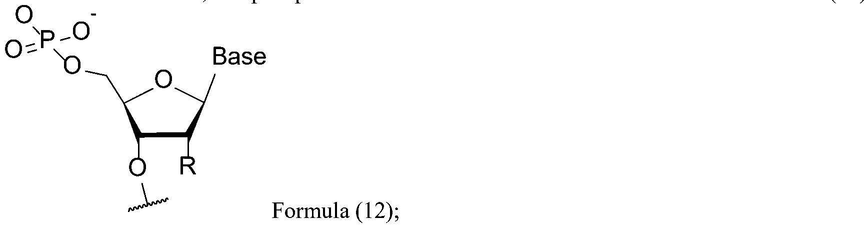

Meanwhile, the types of commonly used 5'-phosphate analog-modified nucleotides are well known to those skilled in the art, for example, the four types of nucleotides shown in Formulas (13)-(16) below, as disclosed in Anastasia Khvorova and Jonathan K. Watts, The chemical evolution of oligonucleotide therapies of clinical utility. Nature Biotechnology, 2017, 35(3): 238-48: 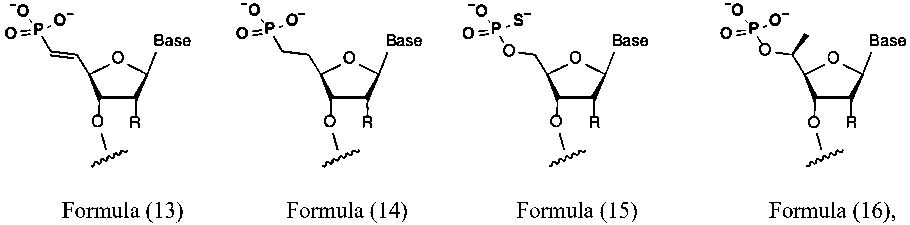 wherein, R represents a group selected from the group consisting of H, OH, F, and methoxy;

Base represents a base selected from A, U, C, G, or T.

In some embodiments, the 5'-phosphate nucleotide or 5'-phosphate analog-modified nucleotide is a nucleotide containing E-vinylphosphonate (E-VP) as shown in Formula (13), a nucleotide containing a 5'-phosphate modification as shown in Formula (12), or a nucleotide containing a 5'-thiophosphate modification as shown in Formula (15).

In some embodiments of the present disclosure, said double-stranded oligonucleotide is an siRNA. According to a third aspect of the present invention, a method for preparing the nucleic acid conjugate disclosed in the present invention is provided, comprising oxidizing the phosphoramidite functional group in the compound of Formula (I) of the present invention to the structure shown in Formula (W), then coupling with an oligonucleotide, followed by cleavage and removal of protecting groups to obtain said conjugate. 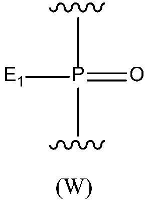
wherein, 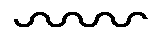 represents the site of covalent bond attachment of the group;
*E*₁ is OH, SH, or *BH*₂.

Any reasonable synthetic route can be employed to prepare the nucleic acid conjugates of the present disclosure.

For example, the preparation method of the nucleic acid conjugate of the present disclosure may include: under the conditions of phosphoramidite solid-phase synthesis, sequentially linking nucleoside monomers in the 3' to 5' direction according to the type and sequence of nucleotides of said functional oligonucleotide, wherein the linking of each nucleoside monomer includes four reaction steps: deprotection, coupling, capping, and oxidation or sulfurization.

In some embodiments of the present disclosure, said coupling reaction is carried out in the presence of an activator, said activator is, for example, selected from one or more of 1H-tetrazole, 5-ethylthio-1H-tetrazole, 5-benzylthio-1H-tetrazole, and in some embodiments is 5-ethylthio-1H-tetrazole.

In some embodiments of the present disclosure, said oxidation reaction conditions include a temperature of 0-50°C, in some embodiments 15-35°C, a reaction time of 1-100 seconds, in some embodiments 5-50 seconds, and the oxidizing agent in some embodiments is iodine (in further embodiments, provided in the form of iodine water).

According to a fourth aspect of the present invention, the use of the nucleic acid conjugate disclosed in the present invention in the preparation of a medicament for treating and/or preventing liver-derived diseases is provided.

According to a fifth aspect of the present invention, a method for treating a pathological condition or disease caused by the expression of a gene in hepatocytes is provided, said method comprising administering the nucleic acid conjugate disclosed in the present invention to a patient suffering from the disease.

According to a sixth aspect of the present invention, a kit is provided, wherein the kit comprises the nucleic acid conjugate disclosed in the present invention.

The following examples are intended to illustrate the invention in an exemplary manner only and not as a limitation. Unless otherwise specified, the raw materials, reagents, etc., used in the following examples are commercially available products.

### Example 1 Preparation of Compound I-1-1

### 1.1 Preparation of Intermediate 1-1

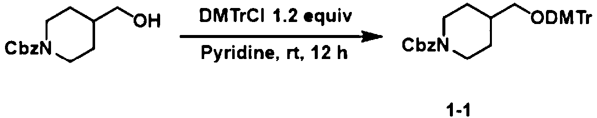

Compound 1-Cbz-4-hydroxymethylpiperidine (commercially available, purchased from Shanghai Titan Scientific Co., Ltd.) (40.1 mmol, 10.0 g) was placed in a clean, dry reaction flask, 100 mL of pyridine was added, and 4,4'-dimethoxytrityl chloride (1.2 equiv, 48.1 mmol, 16.3 g) was added at room temperature, followed by continued stirring at room temperature for 1 hour. After the reaction, 150 mL of ethyl acetate was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (petroleum ether/ethyl acetate = 20/1 - 4/1) to obtain compound 1-1 as a pale yellow oil (21.7 g, 39.3 mmol, 98% yield). Molecular formula of compound 1-1: *C*₃₅*H*₃₇*O*₅*N*, Molecular weight: 551.3, LC-MS found: 552.4 (M+H). ¹H NMR (400 MHz, DMSO-d₆): δ 7.31 - 7.22 (m, 9H), 7.17 (d, J = 8.6 Hz, 5H), 6.82 (d, J = 8.7 Hz, 4H), 4.99 (s, 2H), 3.93 (d, J = 12.6 Hz, 2H), 3.66 (s, 6H), 2.78 - 2.61 (m, 4H), 2.44 (s, 1H), 1.62 (d, J = 12.6 Hz, 2H), 0.97 (qd, J = 12.7, 3.6 Hz, 2H).

### 1.2 Preparation of Intermediate 1-2

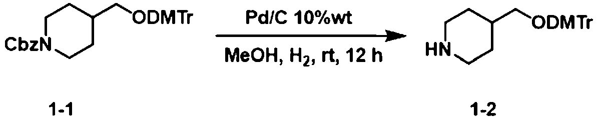

Compound 1-1 (39.3 mmol, 21.7 g) was placed in a clean, dry reaction flask, 150 mL of methanol was added, and palladium on carbon (wet basis, 10% Pd/C) (10%wt, 2.2 g) was added under hydrogen at room temperature, followed by continued stirring at room temperature for 12 hours. After the reaction, the palladium on carbon was removed by filtration, and the filtrate was concentrated to obtain crude product compound 1-2 as a white solid (16.1 g, 38.5 mmol, 98% yield), which was used directly in the next step without purification. Molecular formula of compound 1-2: *C*₂₇*H*₃₁*O*₃*N*, Molecular weight: 417.2, LC-MS found: 418.4 (M+H).

### 1.3 Preparation of Intermediate 1-3

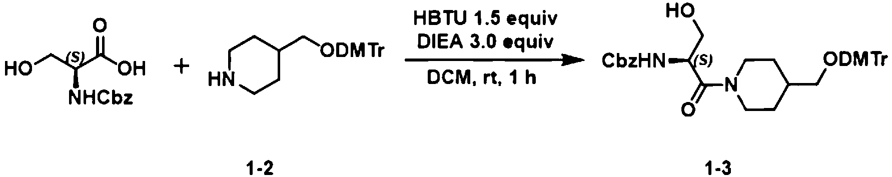

Compound N-benzyloxycarbonyl-L-serine (commercially available, purchased from Shanghai Titan Scientific Co., Ltd.) (35.0 mmol, 8.4 g) was placed in a clean, dry reaction flask, 100 mL of dichloromethane was added, and benzotriazole-N,N,N',N'-tetramethyluronium hexafluorophosphate (HBTU) (1.5 equiv, 52.5 mmol, 19.9 g), compound 1-2 (1.1 equiv, 38.5 mmol, 16.1 g), and N,N-diisopropylethylamine (3.0 equiv, 105.0 mmol, 13.5 g) were added at room temperature, followed by stirring at room temperature for 1 hour. After the reaction, 150 mL of dichloromethane was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (petroleum ether/ethyl acetate = 10/1 - 2/3) to obtain compound 1-3 as a white solid (14.9 g, 23.5 mmol, 67% yield). Molecular formula of compound 1-3: *C*₃₈*H*₄₂*O*₇*N*₂, Molecular weight: 638.3, LC-MS found: 639.4 (M+H). ¹H NMR (400 MHz, DMSO-d₆): δ 7.42 - 7.26 (m, 9H), 7.23 (d, J = 8.7 Hz, 5H), 6.89 (d, J = 8.8 Hz, 4H), 5.03 - 4.96 (m, 2H), 4.79 (dt, J = 25.7, 5.4 Hz, 1H), 4.55 - 4.48 (m, 1H), 4.36 (d, J = 10.7 Hz, 1H), 4.01 (dt, J = 24.8, 8.8 Hz, 1H), 3.73 (s, 6H), 3.56 (ddd, J = 18.7, 12.7, 5.8 Hz, 1H), 3.43 - 3.38 (m, 1H), 3.00 (t, J = 12.3 Hz, 1H), 2.85 - 2.79 (m, 2H), 2.69 (s, 1H), 2.56 (d, J = 10.2 Hz, 1H), 1.88 (d, J = 21.0 Hz, 1H), 1.69 (dd, J = 36.5, 11.3 Hz, 2H), 1.19 - 0.98 (m, 2H).

### 1.4 Preparation of Intermediate 1-4

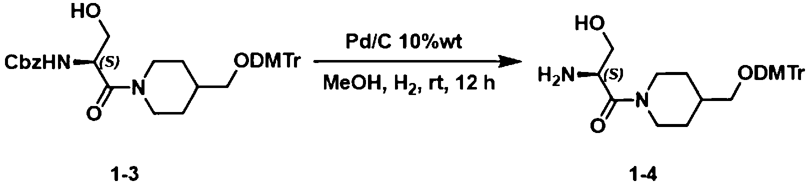

Compound 1-3 (23.5 mmol, 14.9 g) was placed in a clean, dry reaction flask, 100 mL of methanol was added, and palladium on carbon (wet basis, 10% Pd/C) (10%wt, 1.5 g) was added under hydrogen at room temperature, followed by continued stirring at room temperature for 12 hours. After the reaction, the palladium on carbon was removed by filtration, and the filtrate was concentrated to obtain crude product compound 1-4 as a white solid (11.6 g, 23.0 mmol, 98% yield), which was used directly in the next step without purification. Molecular formula of compound 1-4: *C*₃₀*H*₃₆*O*₅*N*₂, Molecular weight: 504.2, LC-MS found: 527.6 (M+Na). ¹H NMR (400 MHz, DMSO-d₆): δ 7.36 (d, J = 7.5 Hz, 2H), 7.31 (t, J = 7.6 Hz, 2H), 7.23 (d, J = 8.8 Hz, 5H), 6.89 (d, J = 8.8 Hz, 4H), 4.39 (t, J = 12.2 Hz, 1H), 3.97 (d, J = 13.0 Hz, 1H), 3.73 (s, 6H), 3.69 (t, J = 6.3 Hz, 1H), 3.43 - 3.36 (m, 5H), 3.01 - 2.92 (m, 2H), 2.83 (d, J = 6.1 Hz, 2H), 1.84 (dd, J = 11.6, 8.1 Hz, 1H), 1.72 (d, J = 12.0 Hz, 2H), 1.17 - 0.91 (m, 2H).

### 1.5 Preparation of Intermediate 1-5

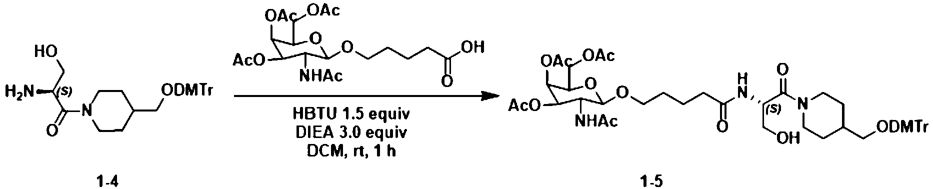

Compound 5-(((2R,3R,4R,5R,6R)-3-acetamido-4,5-diacetoxy-6-(acetoxymethyl)tetrahydro-2H-pyran-2-yl)oxy)pentanoic acid (commercially available, purchased from Norgalins Pharmaceutical Tech (Suzhou) Co., Ltd.) (9.3 g, 20.9 mmol) was placed in a clean, dry reaction flask, 100 mL of dichloromethane was added, and benzotriazole-N,N,N',N'-tetramethyluronium hexafluorophosphate (HBTU) (1.5 equiv, 31.3 mmol, 11.9 g) was added at room temperature. After stirring for ten minutes, compound 1-4 (1.1 equiv, 23.0 mmol, 11.6 g) and N,N-diisopropylethylamine (3.0 equiv, 62.7 mmol, 8.1 g) were added to the reaction system, followed by continued stirring at room temperature for 1 hour. After the reaction, 150 mL of dichloromethane was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (dichloromethane/methanol = 50/1 - 20/1) to obtain compound 1-5 as a white solid (15.4 g, 16.5 mmol, 79% yield). Molecular formula of compound 1-5: *C*₄₉*H*₆₃*O*₁₃*N*₅, Molecular weight: 933.4, LC-MS found: 932.5 (M-H). ¹H NMR (400 MHz, DMSO-d₆): δ 7.93 (dd, J = 24.8, 8.3 Hz, 1H), 7.80 (dd, J = 8.8, 5.3 Hz, 1H), 7.36 (d, J = 7.8 Hz, 2H), 7.31 (t, J = 7.6 Hz, 2H), 7.23 (d, J = 8.7 Hz, 5H), 6.89 (d, J = 8.8 Hz, 4H), 5.21 (d, J = 3.3 Hz, 1H), 4.97 (dd, J = 11.2, 3.4 Hz, 1H), 4.79 (d, J = 4.7 Hz, 1H), 4.47 (dd, J = 8.0, 3.6 Hz, 1H), 4.37 (d, J = 12.7 Hz, 1H), 4.05 - 4.00 (m, 5H), 3.87 (dd, J = 20.2, 10.3 Hz, 1H), 3.73 (s, 6H), 3.57 - 3.50 (m, 1H), 3.14 (qd, J = 7.3, 4.3 Hz, 1H), 2.99 (t, J = 14.2 Hz, 1H), 2.82 (d, J = 3.8 Hz, 2H), 2.10 (s, 5H), 1.99 - 1.98 (m, 6H), 1.89 (s, 3H), 1.76 (d, J = 9.2 Hz, 4H), 1.25 (dd, J = 12.6, 6.5 Hz, 5H), 1.17 (t, J = 7.1 Hz, 3H).

### 1.6 Preparation of Compound I-1-1

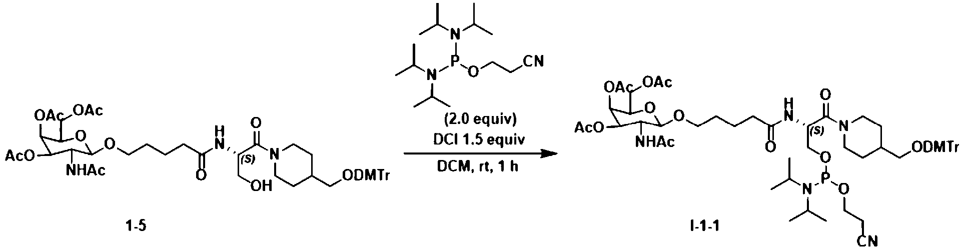

Compound 1-5 (16.5 mmol, 15.4 g) was placed in a clean, dry reaction flask, and 50 mL of anhydrous dichloromethane was added. Under argon protection at room temperature, compound 2-cyanoethyl N,N,N',N'-tetraisopropylphosphorodiamidite (2.0 equiv, 33.0 mmol, 9.9 g) and 4,5-dicyanoimidazole (1.5 equiv, 24.7 mmol, 2.9 g) were added, and stirring was continued at room temperature for one hour. After the reaction, 50 mL of dichloromethane was added to the reaction mixture, and it was washed with 100 mL of saturated sodium bicarbonate solution. The organic phase was dried, filtered, and concentrated. The resulting crude product was purified by preparative C18 reverse-phase column (specification: 30µm; 100 Å, commercially available, purchased from Shanghai Boinn Technology Co., Ltd.) (MeCN:*H*₂*O* = 75%:25%) to obtain I-1-1 as a white solid (11.6 g, 10.2 mmol, 62% yield). Molecular formula of compound I-1-1: *C*₅₈*H*₈₀*O*₁₅*N*₆*P*, Molecular weight: 1133.5, LC-MS found: 1132.4 (M-H). ¹H NMR (400 MHz, DMSO-d₆): δ 8.22 - 8.04 (m, 1H), 7.79 (t, J = 8.2 Hz, 1H), 7.35 (d, J = 2.3 Hz, 2H), 7.30 (t, J = 7.6 Hz, 2H), 7.22 (d, J = 8.5 Hz, 5H), 6.88 (d, J = 8.7 Hz, 4H), 5.21 (d, J = 3.3 Hz, 1H), 4.93 (ddd, J = 18.3, 11.7, 4.3 Hz, 2H), 4.49 - 4.45 (m, 1H), 4.40 - 4.30 (m, 1H), 3.99 (p, J = 8.9 Hz, 4H), 3.85 (dt, J = 13.0, 6.6 Hz, 2H), 3.73 (s, 6H), 3.71 (s, 2H), 3.68 - 3.62 (m, 2H), 3.49 (ddd, J = 15.7, 13.6, 6.6 Hz, 2H), 3.42 - 3.36 (m, 1H), 3.00 (t, J = 14.4 Hz, 1H), 2.82 - 2.79 (m, 2H), 2.77 - 2.72 (m, 1H), 2.69 - 2.64 (m, 1H), 2.60 - 2.54 (m, 1H), 2.10 (s, 5H), 1.98 (dd, J = 8.7, 3.5 Hz, 3H), 1.89 (s, 4H), 1.77 - 1.63 (m, 5H), 1.50 - 1.37 (m, 4H), 1.19 - 1.10 (m, 12H). ³¹P NMR (162 MHz, DMSO-d₆): δ 147.90, 147.30.

### Example 2 Preparation of Compound I-1-7

### 2.1 Preparation of Intermediate 2-1

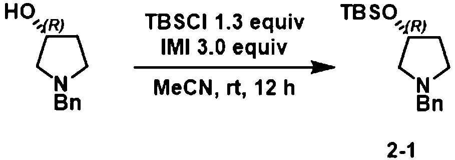

Compound (R)-(+)-N-benzyl-3-hydroxypyrrolidine (commercially available, purchased from Shanghai Titan Scientific Co., Ltd.) (16.9 mmol, 3.0 g) and imidazole (3.0 equiv, 50.7 mmol, 3.45 g) were placed in a clean, dry reaction flask, 50 mL of acetonitrile was added, and tert-butyldimethylsilyl chloride (1.3 equiv, 21.9 mmol, 3.31 g) was added slowly at room temperature, followed by continued stirring at room temperature for 12 hours. After the reaction, 100 mL of ethyl acetate was added to the reaction mixture, and it was washed with 100 mL of saturated sodium bicarbonate solution and 100 mL of saturated brine. The organic phase was dried, filtered, and concentrated. The resulting crude product was purified by silica gel column chromatography (petroleum ether/ethyl acetate = 20/1 - 5/1) to obtain compound 2-1 as a colorless oil (4.9 g, 16.8 mmol, 99% yield). Molecular formula of compound 2-1: *C*₁₇*H*₂₉*ONSi*, Molecular weight: 291.2, LC-MS found: 292.4 (M+H).

### 2.2 Preparation of Intermediate 2-2

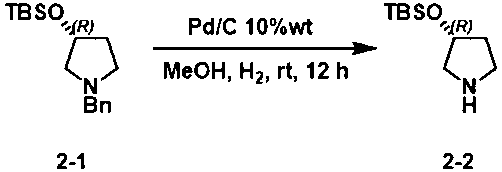

Compound 2-1 (16.8 mmol, 4.9 g) was placed in a clean, dry reaction flask, 100 mL of methanol was added, and palladium on carbon (wet basis, 10% Pd/C) (10%wt, 490.0 mg) was added under hydrogen at room temperature, followed by continued stirring at room temperature for 12 hours. After the reaction, the palladium on carbon was removed by filtration, and the filtrate was concentrated to obtain crude product compound 2-2 as a white solid (3.31 g, 16.5 mmol, 98% yield), which was used directly in the next step without purification. Molecular formula of compound 2-2: *C*₁₀*H*₂₃*ONSi*, Molecular weight: 201.1, LC-MS found: 202.3 (M+H).

### 2.3 Preparation of Intermediate 2-3

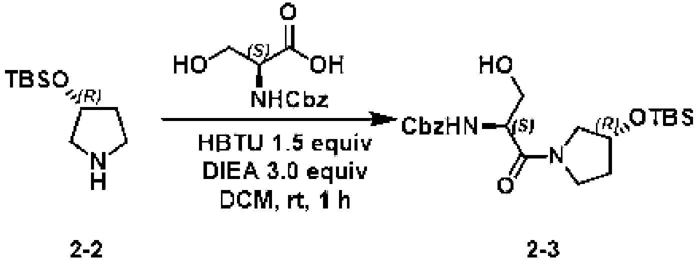

Compound N-benzyloxycarbonyl-L-serine (commercially available, purchased from Shanghai Titan Scientific Co., Ltd.) (15.0 mmol, 3.58 g) was placed in a clean, dry reaction flask, 100 mL of dichloromethane was added, and benzotriazole-N,N,N',N'-tetramethyluronium hexafluorophosphate (1.5 equiv, 22.5 mmol, 8.53 g), compound 2-2 (1.1 equiv, 16.5 mmol, 3.31 g), and N,N-diisopropylethylamine (3.0 equiv, 45.0 mmol, 5.78 g) were added at room temperature, followed by stirring at room temperature for 1 hour. After the reaction, 150 mL of dichloromethane was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (petroleum ether/ethyl acetate = 10/1 - 1/3) to obtain compound 2-3 as a white solid (4.5 g, 10.65 mmol, 63% two-step yield). Molecular formula of compound 2-3: *C*₂₁*H*₃₄*O*₅*N*₂*Si*, Molecular weight: 422.2, LC-MS found: 423.3 (M+H). ¹H NMR (400 MHz, CDCl₃): δ 7.35 - 7.29 (m, 5H), 5.96 (dd, J = 14.3, 8.3 Hz, 1H), 5.10 (s, 2H), 4.61 - 4.40 (m, 2H), 3.85 - 3.68 (m, 2H), 3.65 - 3.49 (m, 2H), 3.41 (d, J = 12.7 Hz, 1H), 3.31 (s, 1H), 1.95 (qdd, J = 15.0, 11.8, 5.3 Hz, 2H), 1.77 (s, 1H), 0.86 (s, 9H), 0.06 (d, J = 3.1 Hz, 6H).

### 2.4 Preparation of Intermediate 2-4

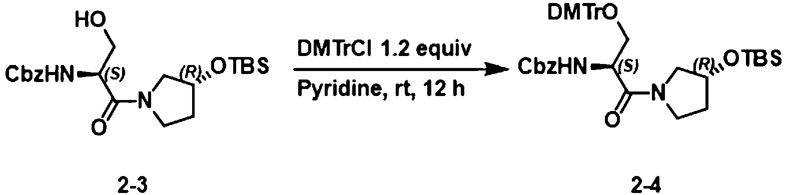

Compound 2-3 (10.65 mmol, 4.5 g) was placed in a clean, dry reaction flask, 100 mL of pyridine was added, and 4,4'-dimethoxytrityl chloride (1.2 equiv, 12.78 mmol, 4.32 g) was added at room temperature, followed by continued stirring at room temperature for 12 hours. After the reaction, 150 mL of ethyl acetate was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (petroleum ether/ethyl acetate = 20/1 - 1/1) to obtain compound 2-4 as a pale yellow oil (7.56 g, 10.43 mmol, 98% yield). Molecular formula of compound 2-4: *C*₄₂*H*₅₂*O*₇*N*₂*Si*, Molecular weight: 724.3, LC-MS found: 747.4 (M+Na). ¹H NMR (400 MHz, CDCl₃): δ 7.35 - 7.31 (m, 1H), 7.28 (d, J = 4.7 Hz, 4H), 7.27 - 7.23 (m, 2H), 7.23 - 7.14 (m, 5H), 7.13 - 7.11 (m, 2H), 6.80 - 6.78 (m, 1H), 6.76 (dd, J = 7.7, 5.4 Hz, 4H), 5.72 (dd, J = 22.7, 8.3 Hz, 1H), 5.08 - 4.99 (m, 2H), 4.69 - 4.59 (m, 1H), 4.35 - 4.30 (m, 1H), 3.73 (dd, J = 4.5, 3.7 Hz, 6H), 3.65 - 3.44 (m, 2H), 3.36 - 3.20 (m, 3H), 1.86 - 1.81 (m, 1H), 1.70 (s, 1H), 0.80 (d, J = 13.1 Hz, 9H), -0.02 (dd, J = 14.9, 4.2 Hz, 6H).

### 2.5 Preparation of Intermediate 2-5

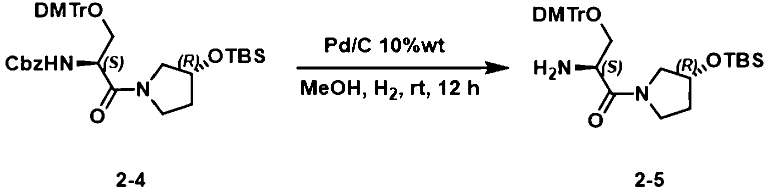

Compound 2-4 (10.43 mmol, 7.56 g) was placed in a clean, dry reaction flask, 100 mL of methanol was added, and palladium on carbon (wet basis, 10% Pd/C) (10%wt, 750.0 mg) was added under hydrogen at room temperature, followed by continued stirring at room temperature for 12 hours. After the reaction, the palladium on carbon was removed by filtration, and the filtrate was concentrated to obtain crude product compound 2-5 as a white solid (6.0 g, 10.22 mmol, 98% yield), which was used directly in the next step without purification. Molecular formula of compound 2-5: *C*₃₄*H*₄₆*O*₅*N*₂*Si*, Molecular weight: 590.3, LC-MS found: 591.6 (M+H).

### 2.6 Preparation of Intermediate 2-6

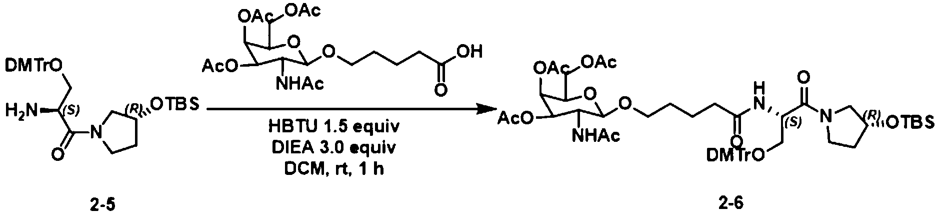

Compound 5-(((2R,3R,4R,5R,6R)-3-acetamido-4,5-diacetoxy-6-(acetoxymethyl)tetrahydro-2H-pyran-2-yl)oxy)pentanoic acid (commercially available, purchased from Norgalins Pharmaceutical Tech (Suzhou) Co., Ltd.) (2.06 g, 4.62 mmol) was placed in a clean, dry reaction flask, 100 mL of dichloromethane was added, and benzotriazole-N,N,N',N'-tetramethyluronium hexafluorophosphate (1.5 equiv, 6.93 mmol, 2.63 g) was added at room temperature. After stirring for ten minutes, compound 2-5 (1.1 equiv, 5.08 mmol, 3.0 g) and N,N-diisopropylethylamine (3.0 equiv, 13.86 mmol, 17.91 g) were added to the reaction system, followed by continued stirring at room temperature for 1 hour. After the reaction, 150 mL of dichloromethane was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (dichloromethane/methanol = 50/1 - 10/1) to obtain compound 2-6 as a white solid (3.58 g, 3.51 mmol, 76% yield). Molecular formula of compound 2-6: *C*₅₃*H*₇₃*O*₁₅*N*₃*Si*, Molecular weight: 1019.3, LC-MS found: 1018.3 (M-H).

### 2.7 Preparation of Intermediate 2-7

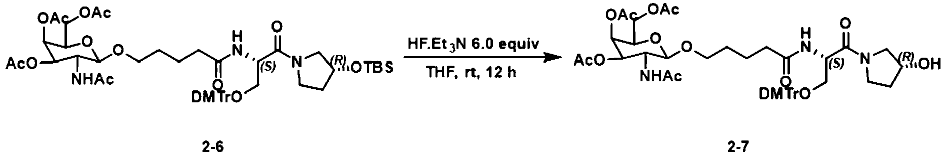

Compound 2-6 (3.51 mmol, 3.58 g) was placed in a clean, dry reaction flask, 50 mL of pyridine was added, and triethylamine hydrofluoride (5.0 equiv, 17.55 mmol, 2.97 g) was added at room temperature, followed by continued stirring at room temperature for 12 hours. After the reaction, 100 mL of ethyl acetate was added to the reaction mixture, and it was washed with 100 mL of saturated sodium bicarbonate solution and 100 mL of saturated brine. The organic phase was dried, filtered, and concentrated. The resulting crude product was purified by silica gel column chromatography (dichloromethane/methanol = 50/1 - 10/1) to obtain compound 2-7 as a pale yellow oil (2.4 g, 2.65 mmol, 76% yield). Molecular formula of compound 2-7: *C*₄₇*H*₅₉*O*₁₅*N*₃, Molecular weight: 905.3, LC-MS found: 904.4 (M-H). ¹H NMR (400 MHz, CDCl₃): δ 7.30 (dd, J = 4.2, 1.9 Hz, 1H), 7.29 - 7.25 (m, 5H), 7.19 - 7.15 (m, 4H), 6.85 - 6.81 (m, 4H), 5.40 - 5.34 (m, 1H), 5.30 (s, 1H), 5.26 - 5.19 (m, 1H), 5.13 (ddd, J = 14.5, 10.9, 3.3 Hz, 1H), 4.85 - 4.78 (m, 1H), 4.75 - 4.71 (m, 1H), 4.68 - 4.61 (m, 1H), 4.50 (d, J = 11.5 Hz, 1H), 4.18 - 4.11 (m, 2H), 4.06 - 3.97 (m, 1H), 3.90 (dt, J = 10.6, 6.8 Hz, 2H), 3.80 (s, 6H), 3.67 - 3.46 (m, 4H), 3.25 - 3.19 (m, 2H), 2.34 - 2.23 (m, 1H), 2.17 - 2.15 (m, 3H), 2.10 - 2.03 (m, 5H), 1.98 (dt, J = 12.9, 2.4 Hz, 4H), 1.76 - 1.61 (m, 4H), 1.37 (t, J = 7.3 Hz, 3H).

### 2.8 Preparation of Compound I-1-7

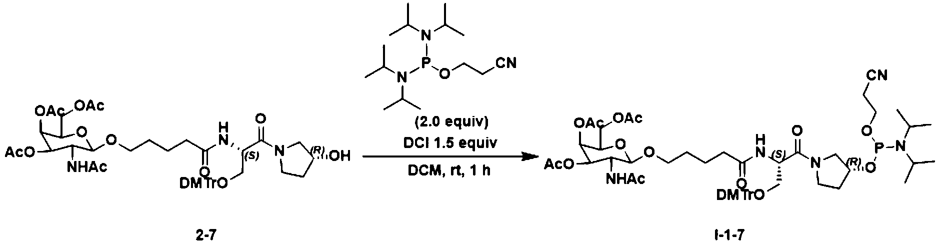

Compound 2-7 (2.65 mmol, 2.4 g) was placed in a clean, dry reaction flask, and 50 mL of anhydrous dichloromethane (commercially available, purchased from Shanghai Titan Scientific Co., Ltd.) was added. Under argon protection at room temperature, compound 2-cyanoethyl N,N,N',N'-tetraisopropylphosphorodiamidite (2.0 equiv, 5.3 mmol, 1.6 g) and 4,5-dicyanoimidazole (1.5 equiv, 3.97 mmol, 470.0 mg) were added, and stirring was continued at room temperature for one hour. After the reaction, 50 mL of dichloromethane was added to the reaction mixture, and it was washed with 100 mL of saturated sodium bicarbonate solution. The organic phase was dried, filtered, and concentrated. The resulting crude product was purified by preparative C18 reverse-phase column (specification: 30µm; 100 Å, commercially available, purchased from Shanghai Boinn Technology Co., Ltd.) (MeCN:*H*₂*O* = 75%:25%) to obtain I-1-7 as a white solid (2.03 g, 1.84 mmol, 70% yield). Molecular formula of compound I-1-7: *C*₅₆*H*₇₆*O*₁₅*N*₆*P*, Molecular weight: 1105.5, LC-MS found: 1128.4 (M+Na). ¹H NMR (400 MHz, DMSO-d₆): δ 8.12 - 8.06 (m, 1H), 7.78 (dd, J = 9.1, 2.0 Hz, 1H), 7.35 - 7.28 (m, 4H), 7.23 - 7.17 (m, 5H), 6.88 (d, J = 7.5 Hz, 4H), 5.21 (d, J = 3.3 Hz, 1H), 4.97 (dd, J = 11.2, 3.0 Hz, 1H), 4.86 - 4.75 (m, 1H), 4.52 (s, 1H), 4.47 (d, J = 8.5 Hz, 1H), 4.04 - 3.98 (m, 3H), 3.91 - 3.83 (m, 1H), 3.74 (s, 6H), 3.70 - 3.60 (m, 3H), 3.58 - 3.47 (m, 3H), 3.39 (t, J = 11.7 Hz, 2H), 3.31 (d, J = 4.1 Hz, 1H), 3.19 (qd, J = 8.7, 3.8 Hz, 1H), 3.02 (dt, J = 19.9, 6.8 Hz, 1H), 2.78 - 2.70 (m, 1H), 2.60 (td, J = 5.8, 1.8 Hz, 1H), 2.09 (s, 5H), 1.98 (s, 4H), 1.89 (s, 3H), 1.73 (s, 3H), 1.44 (s, 4H), 1.20 - 0.88 (m, 14H). ³¹P NMR (162 MHz, DMSO-d₆): δ 148.22 (s), 146.82 (t, J = 35.8 Hz).

### Example 3 Preparation of Compound I-1-5

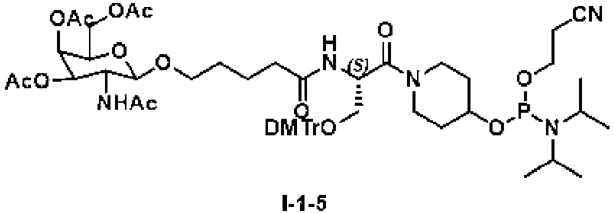

According to the synthesis method of Example 2, starting from raw material N-benzyl-4-hydroxypiperidine (commercially available, purchased from Shanghai Titan Scientific Co., Ltd.), I-1-5 was prepared as a white solid (3.0 g, 2.68 mmol, 72% yield). Molecular formula of compound I-1-5: *C*₅₇*H*₇₈*O*₁₅*N*₆*P*, Molecular weight: 1119.5, LC-MS found: 1142.4 (M+Na). ¹H NMR (400 MHz, DMSO-d₆): δ 8.14 - 8.08 (m, 1H), 7.80 (d, J = 9.2 Hz, 1H), 7.35 (d, J = 7.6 Hz, 2H), 7.29 (t, J = 7.6 Hz, 2H), 7.22 (d, J = 7.5 Hz, 5H), 6.87 (d, J = 7.8 Hz, 4H), 5.22 (d, J = 3.3 Hz, 1H), 5.02 - 4.96 (m, 2H), 4.49 (d, J = 8.4 Hz, 1H), 4.02 (tt, J = 7.9, 3.8 Hz, 4H), 3.88 (dd, J = 19.8, 8.9 Hz, 1H), 3.74 (s, 6H), 3.72 - 3.66 (m, 3H), 3.63 - 3.54 (m, 3H), 3.38 (dd, J = 11.9, 7.7 Hz, 3H), 3.32 - 3.31 (m, 1H), 3.19 (t, J = 7.2 Hz, 1H), 3.06 (s, 1H), 2.77 - 2.74 (m, 2H), 2.14 - 2.07 (m, 5H), 1.98 (s, 3H), 1.90 (s, 3H), 1.75 (s, 4H), 1.46 (s, 6H), 1.27 - 1.05 (m, 13H). ³¹P NMR (162 MHz, DMSO-d₆): δ 148.24 (s), 145.47 (dd, J = 15.7, 11.5 Hz).

### Example 4 Preparation of Compound I-1-2

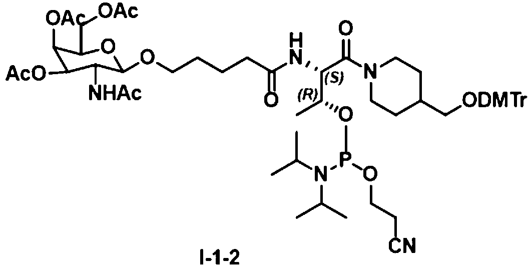

According to the synthesis method of Example 1, starting from raw material L-threonine (commercially available, purchased from Shanghai Titan Scientific Co., Ltd.), I-1-2 was prepared as a white solid (1.2 g, 1.05 mmol, 56% yield). Molecular formula of compound I-1-2: *C*₅₉*H*₈₂*O*₁₅*N*₆*P*, Molecular weight: 1147.5, LC-MS found: 1170.3 (M+Na). ¹H NMR (400 MHz, DMSO-d₆): δ 7.79 (dt, J = 12.8, 4.5 Hz, 1H), 7.37 - 7.29 (m, 4H), 7.22 (d, J = 7.6 Hz, 5H), 6.88 (d, J = 7.7 Hz, 4H), 5.21 (d, J = 3.3 Hz, 1H), 4.97 (dd, J = 11.0, 3.3 Hz, 1H), 4.88 (dt, J = 11.6, 4.7 Hz, 1H), 4.47 (dd, J = 10.6, 5.5 Hz, 1H), 4.41 - 4.32 (m, 1H), 4.16 - 4.08 (m, 1H), 4.04 - 3.99 (m, 3H), 3.92 - 3.82 (m, 1H), 3.73 (s, 6H), 3.70 - 3.63 (m, 2H), 3.30 - 3.24 (m, 5H), 2.90 (dd, J = 10.7, 5.2 Hz, 1H), 2.82 (dt, J = 9.3, 5.6 Hz, 2H), 2.76 - 2.66 (m, 1H), 2.34 - 2.32 (m, 1H), 2.20 - 2.14 (m, 2H), 2.10 (s, 3H), 2.01 - 1.96 (m, 4H), 1.89 - 1.81 (m, 4H), 1.77 - 1.65 (m, 5H), 1.53 - 1.38 (m, 5H), 1.23 - 0.94 (m, 17H). ³¹P NMR (162 MHz, DMSO-d₆): δ 148.02.

### Example 5 Preparation of Compound I-1-9

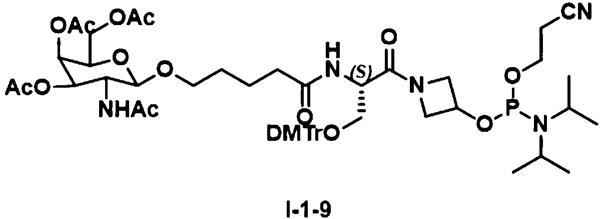

According to the synthesis method of Example 2, starting from raw material 1-benzyloxycarbonyl-3-hydroxyazetidine (commercially available, purchased from Shanghai Titan Scientific Co., Ltd.), I-1-9 was prepared as a white solid (3.02 g, 2.77 mmol, 83% yield). Molecular formula of compound I-1-9: *C*₅₅*H*₇₄*O*₁₅*N*₆*P*, Molecular weight: 1091.4, LC-MS found: 1114.3 (M+Na). ¹H NMR (400 MHz, DMSO-d₆): δ 8.09 - 8.02 (m, 1H), 7.78 (d, J = 9.1 Hz, 1H), 7.37 - 7.29 (m, 4H), 7.24 - 7.19 (m, 5H), 6.89 (d, J = 8.9 Hz, 4H), 5.21 (d, J = 3.4 Hz, 1H), 4.97 (dd, J = 11.2, 3.1 Hz, 1H), 4.77 - 4.61 (m, 1H), 4.57 - 4.50 (m, 1H), 4.47 (dd, J = 8.5, 2.6 Hz, 1H), 4.24 - 4.06 (m, 2H), 4.04 - 3.98 (m, 3H), 3.92 - 3.81 (m, 1H), 3.78 (dd, J = 6.7, 3.8 Hz, 1H), 3.69 (ddd, J = 9.9, 5.5, 2.7 Hz, 2H), 3.61 - 3.51 (m, 2H), 3.40 - 3.35 (m, 1H), 3.16 (q, J = 7.7 Hz, 1H), 3.04 - 2.98 (m, 1H), 2.80 - 2.74 (m, 2H), 2.21 - 2.06 (m, 5H), 1.98 (s, 3H), 1.89 (s, 3H), 1.74 (d, J = 2.5 Hz, 3H), 1.51 - 1.37 (m, 4H), 1.18 - 1.10 (m, 12H), 1.06 (d, J = 6.7 Hz, 2H). ³¹P NMR (162 MHz, DMSO-d₆): δ 146.95 (dd, J = 65.4, 20.5 Hz).

### Example 6 Preparation of Compound I-1-8

### 6.1 Preparation of Intermediate 6-1

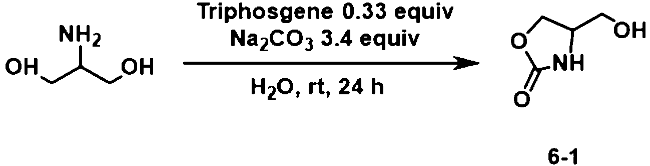

Compound serinol (commercially available, purchased from Adamas, Shanghai Titan Scientific Co., Ltd.) (54.9 mmol, 5.0 g) was placed in a clean reaction flask, dissolved in 90 mL of water, and sodium carbonate (3.4 equiv, 183.8 mmol, 19.5 g) was added at room temperature. Under an ice-water bath, triphosgene (0.33 equiv, 18.3 mmol, 5.4 g) was added slowly in portions, followed by continued stirring at room temperature for 24 hours. The solvent was removed by concentration under reduced pressure, ethanol was added, and the mixture was stirred for 2 h, filtered by suction, and concentrated. The resulting crude product was purified by silica gel column chromatography (dichloromethane/methanol = 100/1 - 9/1) to obtain compound 6-1 as a white solid (4.6 g, 39.3 mmol, 72% yield). Molecular formula of compound 6-1: *C*₄*H*₇*NO*₃, Molecular weight: 117.1, LC-MS found: 118.4 (M+H). ¹H NMR (400 MHz, DMSO-d₆): δ 7.58 (s, 1H), 4.31 (t, J = 8.6 Hz, 1H), 4.05 (dd, J = 8.5, 5.0 Hz, 1H), 3.78 - 3.72 (m, 1H), 3.36 (d, J = 5.0 Hz, 2H).

### 6.2 Preparation of Intermediate 6-2

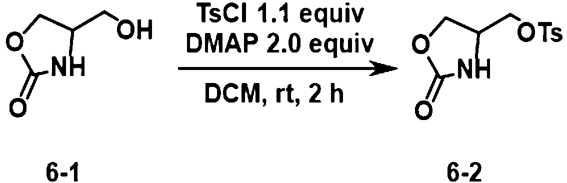

Compound 6-1 (47.0 mmol, 5.5 g) was placed in a clean, dry reaction flask, 300 mL of dichloromethane was added, DMAP (2.0 equiv, 93.9 mmol, 11.5 g) was added, and under an ice-water bath, TsCl (1.1 equiv, 51.7 mmol, 9.8 g) was added slowly in portions, followed by continued stirring at room temperature for 2 hours. After the reaction, the reaction mixture was washed successively with 100 mL of 1M HCl, 100 mL of water, and 100 mL of saturated brine, dried, and evaporated to dryness to obtain 6-2 as a white powder (12.3 g, 45.3 mmol, 97% yield). The crude product was used directly in the next step without purification. Molecular formula of compound 6-2: *C*₁₁*H*₁₃*NO*₅*S*, Molecular weight: 271.1, LC-MS found: 272.4 (M+H).

### 6.3 Preparation of Intermediate 6-3

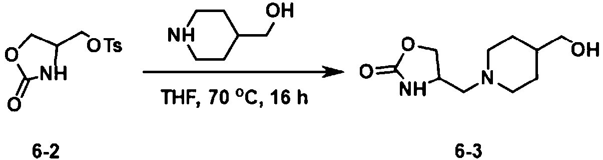

Compound 6-2 (45.5 mmol, 12.3 g) was placed in a clean, dry reaction flask, dissolved in 50 mL of tetrahydrofuran, 4-piperidinemethanol (3.0 equiv, 136.6 mmol, 15.7 g) was added, and the mixture was heated to reflux for 16 h. The reaction mixture was concentrated, and the resulting crude product was purified by silica gel column chromatography (dichloromethane/methanol = 100/1 - 9/1) to obtain compound 6-3 as a colorless syrup (7.9 g, 36.8 mmol, 81% yield). Molecular formula of compound 6-3: *C*₁₀*H*₁₈*N*₂*O*₃, Molecular weight: 214.1, LC-MS found: 215.3 (M+H). ¹H NMR (400 MHz, DMSO-d₆): δ 7.61 (s, 1H), 4.38 (d, J = 5.3 Hz, 1H), 4.33 (t, J = 8.2 Hz, 1H), 3.97 (dd, J = 8.3, 5.5 Hz, 1H), 3.94 - 3.87 (m, 1H), 3.22 (t, J = 5.7 Hz, 2H), 2.82 (t, J = 10.5 Hz, 2H), 2.37 - 2.28 (m, 2H), 1.97 - 1.86 (m, 2H), 1.59 (d, J = 12.4 Hz, 2H), 1.34 - 1.23 (m, 1H), 1.10 (pd, J = 12.3, 3.9 Hz, 2H).

### 6.4 Preparation of Intermediate 6-4

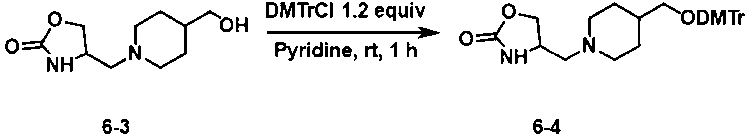

Compound 6-3 (23.3 mmol, 5.0 g) was placed in a clean, dry reaction flask, 50 mL of pyridine was added, and 4,4'-dimethoxytrityl chloride (1.2 equiv, 28.0 mmol, 9.5 g) was added at room temperature, followed by continued stirring at room temperature for 1 hour. After the reaction, 150 mL of ethyl acetate was added to the reaction mixture, and it was washed separately with 150 mL of saturated sodium bicarbonate solution and 150 mL of saturated brine. The organic phase was dried, filtered, and concentrated. The resulting crude product was purified by silica gel column chromatography (dichloromethane/methanol = 100/0 - 100/3) to obtain compound 6-4 as an off-white foam-like solid (8.2 g, 15.9 mmol, 68% yield). Molecular formula of compound 6-4: *C*₃₁*H*₃₆*N*₂*O*₅, Molecular weight: 516.2, LC-MS found: 515.3 (M-H). ¹H NMR (400 MHz, DMSO-d₆): δ 7.61 (s, 1H), 7.38 - 7.35 (m, 2H), 7.30 (t, J = 7.7 Hz, 2H), 7.24 - 7.21 (m, 5H), 6.88 (d, J = 8.9 Hz, 4H), 4.32 (t, J = 8.2 Hz, 1H), 3.96 (dd, J = 8.2, 5.4 Hz, 1H), 3.89 (dt, J = 13.2, 6.8 Hz, 1H), 3.73 (s, 6H), 2.80 (t, J = 9.2 Hz, 4H), 2.37 - 2.27 (m, 2H), 1.94 (dd, J = 26.3, 11.7 Hz, 2H), 1.63 (d, J = 12.3 Hz, 2H), 1.56 - 1.50 (m, 1H), 1.23 - 1.08 (m, 2H).

### 6.5 Preparation of Intermediate 6-5

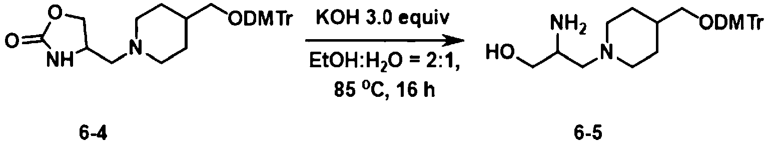

Compound 6-4 (15.9 mmol, 8.2 g) was placed in a clean, dry reaction flask, dissolved in 82 mL of ethanol, and a 41 mL aqueous solution of potassium hydroxide (3.0 equiv, 47.6 mmol, 2.7 g) was added at room temperature. The temperature was raised to 85°C and the reaction was stirred overnight. The solvent was removed by concentration, 100 mL of ethyl acetate was added to the reaction mixture, and it was washed with 60 mL of saturated sodium bicarbonate solution. The aqueous phase was back-extracted with 50 mL of ethyl acetate. The combined organic phases were dried, filtered, and concentrated to obtain 6-5 as a white foam-like solid (7.6 g, 15.5 mmol, 98% yield). The crude product was used directly in the next step without purification. Molecular formula of compound 6-5: *C*₃₀*H*₃₈*N*₂*O*₄, Molecular weight: 490.2, LC-MS found: 491.3 (M+H).

### 6.6 Preparation of Intermediate 6-6

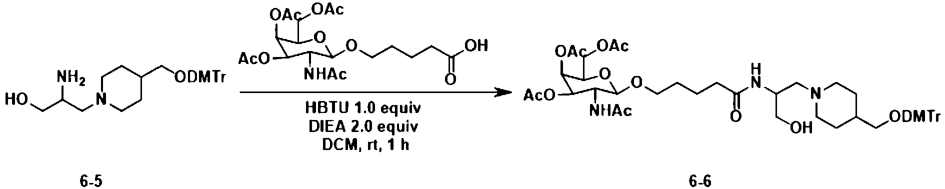

Compound 5-(((2R,3R,4R,5R,6R)-3-acetamido-4,5-diacetoxy-6-(acetoxymethyl)tetrahydro-2H-pyran-2-yl)oxy)pentanoic acid (commercially available, purchased from Norgalins Pharmaceutical Tech (Suzhou) Co., Ltd.) (6.9 g, 15.5 mmol) was placed in a clean, dry reaction flask, 80 mL of dichloromethane was added, and benzotriazole-N,N,N',N'-tetramethyluronium hexafluorophosphate (1.0 equiv, 15.5 mmol, 5.9 g) was added at room temperature. After stirring for ten minutes, compound 6-5 (1.0 equiv, 15.5 mmol, 7.6 g) and N,N-diisopropylethylamine (2.0 equiv, 31.0 mmol, 4.0 g) were added to the reaction system, followed by continued stirring at room temperature for 1 hour. After the reaction, 150 mL of dichloromethane was added to the reaction mixture, and it was washed with 150 mL of saturated sodium bicarbonate solution and 150 mL of saturated brine. The organic phase was dried, filtered, and concentrated. The resulting crude product was purified by silica gel column chromatography (dichloromethane/methanol = 100/1 - 10/1) to obtain compound 6-6 as a white foam-like solid (7.7 g, 8.4 mmol, 54% yield). Molecular formula of compound 6-6: *C*₄₉*H*₆₅*N*₃*O*₁₄, Molecular weight: 919.4, LC-MS found: 918.3 (M-H). ¹H NMR (400 MHz, DMSO-d₆): δ 7.81 (dd, J = 9.2, 3.1 Hz, 1H), 7.45 (d, J = 6.0 Hz, 1H), 7.36 (d, J = 7.3 Hz, 2H), 7.30 (t, J = 7.7 Hz, 2H), 7.24 - 7.21 (m, 5H), 6.88 (d, J = 8.9 Hz, 4H), 5.21 (d, J = 3.4 Hz, 1H), 4.97 (dd, J = 11.2, 3.0 Hz, 1H), 4.48 (dd, J = 8.4, 1.4 Hz, 1H), 4.04 - 3.98 (m, 3H), 3.87 (dd, J = 19.9, 9.3 Hz, 2H), 3.73 (s, 6H), 3.71 - 3.67 (m, 1H), 3.43 - 3.35 (m, 2H), 2.84 (dd, J = 17.8, 8.8 Hz, 4H), 2.10 (s, 3H), 2.05 (t, J = 6.7 Hz, 2H), 1.98 (d, J = 1.4 Hz, 4H), 1.89 (s, 3H), 1.77 (s, 3H), 1.65 (d, J = 9.9 Hz, 2H), 1.53 - 1.41 (m, 5H), 1.26 - 1.23 (m, 1H), 1.14 (dt, J = 22.4, 9.2 Hz, 2H).

### 6.7 Preparation of Compound I-1-8

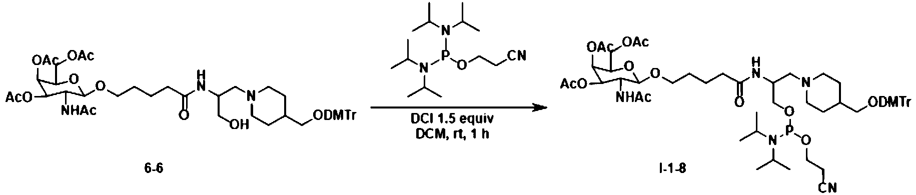

Compound 6-6 (2.2 mmol, 2.0 g) was placed in a clean, dry reaction flask, and 20 mL of anhydrous dichloromethane was added. Under argon protection at room temperature, compound 2-cyanoethyl N,N,N',N'-tetraisopropylphosphorodiamidite (2.0 equiv, 4.4 mmol, 1.3 g) and 4,5-dicyanoimidazole (1.5 equiv, 3.3 mmol, 0.38 g) were added, and stirring was continued at room temperature for one hour. After the reaction, 20 mL of dichloromethane was added to the reaction mixture, and it was washed with 50 mL of saturated sodium bicarbonate solution. The organic phase was dried, filtered, and concentrated. The resulting crude product was purified by preparative C18 reverse-phase column (specification: 30µm; 100 Å, commercially available, purchased from Shanghai Boinn Technology Co., Ltd.) (MeCN:*H*₂*O* = 75%:25%) to obtain I-1-8 as a white solid (1.1 g, 1.0 mmol, 45% yield). Molecular formula of compound I-1-8: *C*₅₈*H*₈₂*O*₁₅*N*₅*P*, Molecular weight: 1119.5, LC-MS found: 1118.4 (M-H). ¹H NMR (400 MHz, DMSO-d₆): δ 7.83 (d, J = 9.2 Hz, 1H), 7.36 (d, J = 7.3 Hz, 2H), 7.31 (dd, J = 9.2, 6.0 Hz, 2H), 7.22 (d, J = 8.6 Hz, 5H), 6.88 (dd, J = 8.8, 1.5 Hz, 4H), 5.21 (d, J = 3.2 Hz, 1H), 4.97 (dd, J = 11.3, 3.3 Hz, 1H), 4.48 (d, J = 8.4 Hz, 1H), 4.06 - 3.96 (m, 4H), 3.87 (dd, J = 20.0, 9.0 Hz, 1H), 3.73 (s, 6H), 3.72 - 3.69 (m, 2H), 3.59 - 3.52 (m, 1H), 2.81 (d, J = 6.0 Hz, 3H), 2.73 (dt, J = 9.7, 6.0 Hz, 2H), 2.09 (s, 3H), 2.07 - 2.03 (m, 2H), 1.99 - 1.98 (m, 4H), 1.88 (d, J = 5.1 Hz, 4H), 1.77 (s, 3H), 1.69 - 1.59 (m, 2H), 1.48 - 1.40 (m, 5H), 1.23 - 1.11 (m, 12H). ³¹P NMR (162 MHz, DMSO-d₆): δ 146.45.

### Example 7 Preparation of Compound I-1-13

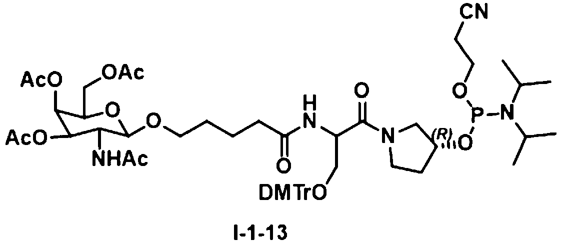

According to the synthesis method of Example 2, starting from raw material N-benzyloxycarbonyl-DL-serine (commercially available, purchased from Shanghai Titan Scientific Co., Ltd.), I-1-13 was prepared as a white solid (2.32 g, 2.1 mmol, 81% yield). Molecular formula of compound I-1-13: *C*₅₆*H*₇₆*O*₁₅*N*₆*P*, Molecular weight: 1105.5, LC-MS found: 1128.6 (M+Na). ¹H NMR (400 MHz, DMSO-d₆): δ 8.09 - 8.03 (m, 1H), 7.75 (dd, J = 9.1, 2.0 Hz, 1H), 7.32 - 7.25 (m, 4H), 7.17 (ddd, J = 13.7, 8.2, 2.3 Hz, 5H), 6.85 (d, J = 7.5 Hz, 4H), 5.18 (d, J = 3.3 Hz, 1H), 4.94 (dd, J = 11.2, 3.0 Hz, 1H), 4.83 - 4.72 (m, 1H), 4.49 - 4.43 (m, 2H), 4.01 - 3.95 (m, 3H), 3.88 - 3.80 (m, 1H), 3.71 (s, 6H), 3.67 - 3.44 (m, 6H), 3.36 (t, J = 11.7 Hz, 2H), 3.28 (d, J = 4.1 Hz, 1H), 3.16 (qd, J = 8.7, 3.8 Hz, 1H), 2.99 (dt, J = 19.9, 6.8 Hz, 1H), 2.75 - 2.67 (m, 1H), 2.57 (td, J = 5.8, 1.8 Hz, 1H), 2.06 (s, 5H), 1.95 (s, 4H), 1.86 (s, 3H), 1.70 (s, 3H), 1.41 (s, 3H), 1.16 - 0.96 (m, 16H). ³¹P NMR (162 MHz, DMSO-d₆): δ 147.08, 146.82, 146.78, 146.64.

### Example 8 Preparation of Compound I-1-15

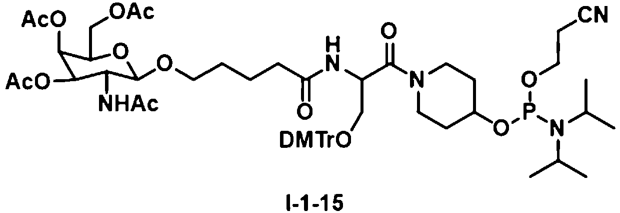

According to the synthesis method of Example 2, starting from raw materials N-benzyl-4-hydroxypiperidine (commercially available, purchased from Shanghai Titan Scientific Co., Ltd.) and N-benzyloxycarbonyl-DL-serine (commercially available, purchased from Shanghai Titan Scientific Co., Ltd.), I-1-15 was prepared as a white solid (2.1 g, 1.88 mmol, 78% yield). Molecular formula of compound I-1-15: *C*₅₇*H*₇₈*O*₁₅*N*₆*P*, Molecular weight: 1119.5, LC-MS found: 1142.6 (M+Na). ¹H NMR (400 MHz, DMSO-d₆): δ 8.10 - 8.05 (m, 1H), 7.76 (d, J = 9.2 Hz, 1H), 7.32 (d, J = 7.6 Hz, 2H), 7.25 (t, J = 7.6 Hz, 2H), 7.18 (d, J = 7.5 Hz, 5H), 6.84 (d, J = 7.8 Hz, 4H), 5.18 (d, J = 3.3 Hz, 1H), 4.98 - 4.92 (m, 2H), 4.45 (d, J = 8.4 Hz, 1H), 4.01 - 3.95 (m, 4H), 3.84 (dd, J = 19.8, 8.9 Hz, 1H), 3.70 (s, 6H), 3.69 - 3.63 (m, 3H), 3.59 - 3.50 (m, 2H), 3.34 (dd, J = 11.9, 7.7 Hz, 2H), 3.28 (d, J = 1.6 Hz, 1H), 3.16 (t, J = 7.2 Hz, 2H), 2.73 - 2.70 (m, 2H), 2.11 - 2.03 (m, 5H), 1.95 (s, 3H), 1.86 (s, 3H), 1.72 (s, 4H), 1.43 (s, 6H), 1.12 - 1.09 (m, 15H). ³¹P NMR (162 MHz, DMSO-d6): δ 145.56, 145.49, 145.46, 145.39.

### Example 9 Preparation of siRNA conjugates

Using the solid-phase phosphoramidite method, the specially modified compounds prepared in the above steps and commercially purchased conventionally modified monomers (phosphoramidite monomers for synthesizing modified nucleotides dT, Am, Cm, Gm, Um, Af, Cf, Gf, Uf, all purchased from Shanghai Zhaowei Technology Development Co., Ltd.) were used to link nucleoside monomers one by one in the 3' to 5' direction according to the nucleotide sequence. The specially modified anti-off-target compound was placed in the seed region of the antisense strand (any position from 4-8 counting from the 5' end), and the delivery monomer compound was freely placed at the 3' or 5' end as a normal monomer. The connection of each nucleoside monomer includes four reaction steps: deprotection, coupling, capping, and oxidation or sulfurization.

Synthesis conditions used for the sense and antisense strands.

Equipment model: MerMade 12 Oligonucleotide synthesizer solid-phase synthesizer, Beijing Haijing 6 mL synthesis column, Cytiva SourceTM 15Q 4.6/100PE purification column.

Reagents used for synthesizing siRNA conjugates were purchased from Suzhou Cole-Parmer Biotechnology Co., Ltd.

A brief description of the synthesis is as follows:
The single-strand synthesis reaction process proceeds in the 3' to 5' direction and is completed on a solid-phase synthesizer. It includes four main reaction steps:
a. De-DMTr reaction: The protecting group DMTr on the nucleotide is removed with dichloroacetic acid to obtain a 5'-hydroxyl end;
b. Coupling reaction: The protected nucleoside phosphoramidite monomer is mixed with the activator ethylthiotetrazole, the phosphoramidite group is activated, and the 5'-hydroxyl, which is still protected by DMTr, undergoes a condensation reaction with the 5'-hydroxyl group linked to the solid support, forming a phosphite triester;
c. Oxidation reaction: Under the action of the oxidizing agent iodine, the phosphite triester obtained from the previous condensation reaction is converted into a more stable phosphate ester (i.e., oxidizing trivalent phosphorus to pentavalent phosphorus);
d. Sulfurization reaction: Under the action of the sulfurizing agent PADS (phenylacetyl disulfide), the phosphite triester obtained from the previous condensation reaction is converted into a phosphorothioate ester (oxidation or sulfurization is chosen according to the sequence design).
e. Capping reaction: A very small number of 5'-hydroxyl groups (less than 2%) may not have participated in the condensation reaction. They are reacted with acetic anhydride and 1-methylimidazole to form an acetate ester cap that cannot participate in subsequent reactions, preventing further reaction. These short fragments can be removed during purification.

The cycle of the above four steps is repeated until the required sequence is synthesized. The main chemical reaction equations are as follows:
After the last nucleoside monomer is connected, the nucleic acid sequence linked to the solid support is subjected to cleavage, deprotection, purification, and desalting in sequence, followed by lyophilization to obtain the sense and antisense strands. Wherein:
Cleavage and deprotection conditions are as follows: First, prepare the ammonolysis solution (a mixture of ammonia water:ethanol=3:1 to a volume of 2 mL). The solid support is added to the above reaction flask and shaken thoroughly to mix well. Ammonolysis is carried out at 50°C in a constant temperature water bath for 16 hours. After 16 hours of ammonolysis, it is cooled to room temperature (25°C±2°C) in a water bath, filtered with a fritted funnel, and the filtrate is collected in a round-bottom flask. The filter residue is rinsed with a 50% aqueous ethanol solution, the filtrate is collected, concentrated on a rotary evaporator, and then transferred to a glass vial. A crude sample is taken and sent to the analysis department for crude LC-MS testing. The detection method is as follows: A Waters Acquity UPLC-LTQ LCMS (column: ACQUITY UPLC BEH C18) is used to detect the purity of the above sense and antisense strands and analyze their molecular weights. The measured values are consistent with the theoretical values, see Table 1.

Purification and desalting conditions are as follows: An ion-exchange chromatography column is used for purification, in conjunction with a Cytiva HiPrepTM 26/10 Desalting gel column for desalting, followed by lyophilization of the single strands. After lyophilization of the single strands, a sample needs to be taken for LC-MS measurement.

Finally, the obtained sense and antisense strands need to be annealed into a double strand.

Annealing operation is as follows: The purified sense and antisense strands are separately dissolved in water for injection to prepare solutions of 0.1mg/mL-40mg/mL. They are mixed in equimolar ratios calibrated with a Thermo Scientific Nanodrop Eight, heated at 90°C for 5 minutes, and then slowly cooled naturally, allowing them to form a double-stranded structure through hydrogen bonds. A sample is taken to test the SEC purity of the product, see Table 2, and the double-stranded sample is lyophilized.

**Table 1: siRNA numbers and sequence information for delivery molecule conjugation**

| Conjugate No. | Single Strand Base Sequence Information | | Single Strand Theoretical MW | Single Strand Measured MW |
|---|---|---|---|---|
| SD003 317 | Sense strand base sequence (5'→3') | AmsAmsGfCmAfAmGfAmUfAfUfUmUfU mUmAfUmAfAmUmAmL96 | 8682.3 | 8681.5 |
| | Antisense strand base sequence (5'→3') | UmsAfsUfUmAfUmAmAfAmAfAmUmA mUfCmUfUmGfCmUmUmsUmsUmdTdT | 8100.2 | 8099.0 |
| SD003 974 | Sense strand base sequence (5'→3') | AmsAmsGfCmAfAmGfAmUfAfUfUmUfU mUmAfUmAfAmUmAm I-1-1 I-1-1 I-1-1 | 8595.9 | 8593.4 |
| | Antisense strand base sequence (5'→3') | UmsAfsUfUmAfUmAmAfAmAfAmUmA mUfCmUfUmGfCmUmUmsUmsUmdTdT | 8100.2 | 8099.0 |
| SD004 119 | Sense strand base sequence (5'→3') | AmsAmsGfCmAfAmGfAmUfAfUfUmUfU mUmAfUmAfAmUmAm I-1-7 I-1-7 I-1-7 | 8511.8 | 8510.9 |
| | Antisense strand base sequence (5'→3') | UmsAfsUfUmAfUmAmAfAmAfAmUmA mUfCmUfUmGfCmUmUmsUmsUmdTdT | 8100.2 | 8099.0 |
| SD004 122 | Sense strand base sequence (5'→3') | AmsAmsGfCmAfAmGfAmUfAfUfUmUfU mUmAfUmAfAmUmAm I-1-5 I-1-5 I-1-5 | 8553.8 | 8551.8 |
| | Antisense strand base sequence (5'→3') | UmsAfsUfUmAfUmAmAfAmAfAmUmA mUfCmUfUmGfCmUmUmsUmsUmdTdT | 8100.2 | 8099.0 |
| SD004 125 | Sense strand base sequence (5'→3') | AmsAmsGfCmAfAmGfAmUfAfUfUmUfU mUmAfUmAfAmUmAm I-1-13 I-1-13 I-1-13 | 8511.8 | 8510.3 |
| | Antisense strand base sequence (5'→3') | UmsAfsUfUmAfUmAmAfAmAfAmUmA mUfCmUfUmGfCmUmUmsUmsUmdTdT | 8100.2 | 8099.0 |

**Table 2: Duplex SEC-HPLC Purity**

| Conjugate No. | Duplex SEC-HPLC Purity |
|---|---|
| SD003317 | 96% |
| SD003974 | 92% |
| SD004119 | 92% |
| SD004122 | 92% |
| SD004125 | 94% |

### Example 10: In vivo activity test of siRNA conjugates in mice

SPF-grade female C57BL/6J mice, 6-8 weeks old, with a body weight of 20±2 g, were selected. Before administration, the mice were weighed and their condition was observed. Animals with uniform body weight and no abnormal conditions were selected for random grouping, with 4 mice per group. The experimental group mice were given the conjugate, and the vehicle group mice were given phosphate-buffered saline (PBS). Subcutaneous administration was performed at a dose of 1 mg/kg of conjugate per mouse. 20 days after administration, the animals were euthanized, and liver tissue was collected. Following conventional methods, the liver was cut into pieces and placed in RNALater (Invitrogen, AM7021M) for subsequent RNA extraction. The liver tissue was placed in lysis buffer (Changchun Zhi'ang Biotechnology Co., Ltd., MNTR/FX96) and ground (Shanghai Jingxin Industrial Development Co., Ltd., JXFSTPRP-48L) to extract total RNA, which was reverse transcribed into cDNA (Takara, 6210B). The expression level of the target gene complement C5 mRNA was detected by fluorescence qPCR (Vazyme, Q711).

Target gene primers:
Forward primer: CCAGCCCAATCAAGTTCCTAGAG;
Reverse primer: CGGCGTGTAAACAGGTTTGTC;
Internal reference gene GAPDH primers:
   Forward primer: TGCACCACCAACTGCTTAG;
   Reverse primer: GATGCAGGGATGATGTTC;

The results are expressed as the remaining expression level of the siRNA administration group compared to the vehicle group (vehicle group is 100%). The siRNA sequences of the conjugates used for injection are shown in Table 1. As shown in Figure 1 and Table 3, compared with the positive conjugate SD003317, conjugate SD003974 showed comparable silencing activity of the target gene complement C5 mRNA; conjugates SD004119, SD004122, and SD004125 showed significantly superior silencing activity of the complement C5 mRNA of the target gene than conjugate SD003317.

**Table 3: Relative remaining expression level of the target gene complement C5 mRNA 20 days after administration of the test conjugates**

| Conjugate No. | Relative Remaining Percentage % |
|---|---|
| Vehicle | 100.25 |
| SD003317 | 42.70 |
| SD003974 | 42.55 |
| SD004119 | 28.95 |
| SD004122 | 27.33 |
| SD004125 | 28.87 |

In the sequences mentioned above and in Table 1, the respective symbols are used to represent the following modified nucleotides:
A = adenosine-3'-phosphate; C = cytidine-3'-phosphate; G = guanosine-3'-phosphate; U = uridine-3'-phosphate; dT = thymidine deoxyribonucleotide; Am = 2'-O-methyladenosine-3'-phosphate; Ams = 2'-O-methyladenosine-3'-phosphorothioate; Cm = 2'-O-methylcytidine-3'-phosphate; Cms = 2'-O-methylcytidine-3'-phosphorothioate; Gm = 2'-O-methylguanosine-3'-phosphate; Gms = 2'-O-methylguanosine-3'-phosphorothioate; Um = 2'-O-methyluridine-3'-phosphate; Ums = 2'-O-methyluridine-3'-phosphorothioate; Af = 2'-fluoroadenosine-3'-phosphate; Afs = 2'-fluoroadenosine-3'-phosphorothioate; Cf = 2'-fluorocytidine-3'-phosphate; Cfs = 2'-fluorocytidine-3'-phosphorothioate; Gf = 2'-fluoroguanosine-3'-phosphate; Gfs = 2'-fluoroguanosine-3'-phosphorothioate; Uf = 2'-fluorouridine-3'-phosphate; Ufs = 2'-fluorouridine-3'-phosphorothioate.

Exemplary embodiments have been disclosed herein, and although specific terms are employed, they are used and are to be interpreted in a generic and descriptive sense only and not for purposes of limitation. In some instances, as would be apparent to one of ordinary skill in the art as of the filing of the present application, features, characteristics, and/or elements described in connection with a particular embodiment may be used singly or in combination with features, characteristics, and/or elements described in connection with other embodiments unless otherwise specifically indicated. It will thus be understood by those skilled in the art that various changes in form and details may be made without departing from the spirit and scope of the invention as set forth in the appended claims.

## Claims

1. A compound, wherein the compound has the structure shown in Formula (I): 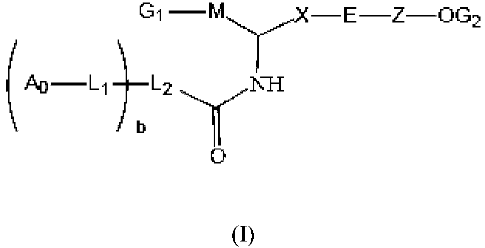 wherein,
*A*₀ represents a ligand having affinity for the asialoglycoprotein receptor on the surface of mammalian liver cells, or represents a group formed by replacing all or part of the hydroxyl groups in the ligand with a KCOO- group, wherein each K is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl;
b is an integer in the range of 1-4;
*L*₁ and *L*₂ independently represent a linking combination of one or more groups selected from Formula A1-A11: 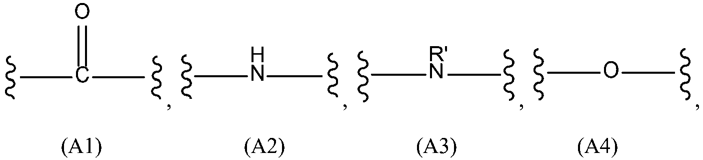 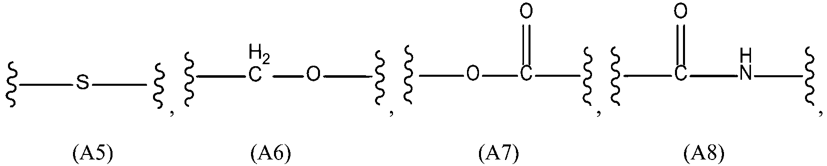 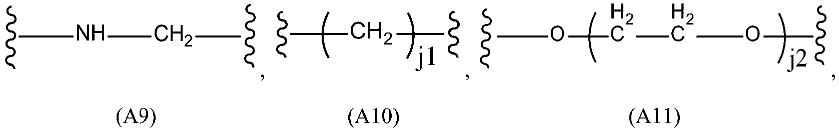
wherein, j1 is an integer from 1-20; j2 is an integer from 1-20; R' is a *C*₁ - *C*₁₀ alkyl;
M represents a structural formula as shown in Formula (A12): 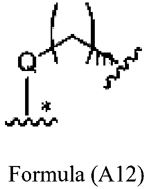
wherein, m represents an integer from 0-6; * represents the bond connected to *G*₁,
Q represents a structural formula as shown in Formula (A13): 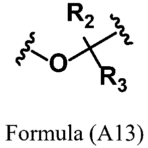
wherein, 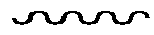 connected to O represents the bond connected to *G*₁; *R*₂ and *R*₃ are the same or different, and are independently selected from H, *C*₁ - *C*₂₀ alkyl, *C*₁ - *C*₂₀ alkoxy, *C*₂ - *C*₂₀ alkenyl, or *C*₂ - *C*₂₀ alkynyl;
Z is absent or represents a *C*₁ - *C*₁₀ alkylene;
X represents a structural formula as shown in Formula (A14-1) or (A14-2): 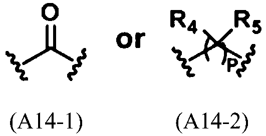
wherein *R*₄ and *R*₅ are the same or different, and are independently selected from H, fluoro, hydroxyl, *C*₁ - *C*₂₀ alkyl, *C*₁ - *C*₂₀ alkoxy, *C*₂ - *C*₂₀ alkenyl, or *C*₂ - *C*₂₀ alkynyl; p is an integer from 1-6; optionally, *R*₄ and *R*₅ are directly connected to form a three- to six-membered saturated carbocyclic ring;
E represents a structural formula as shown in Formula (A15): 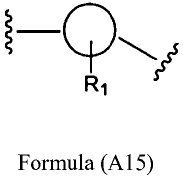
wherein, 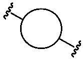 represents a *C*₃ - *C*₁₈ cycloalkyl or a *C*₃ - *C*₁₈ heterocyclyl, and *R*₁ is selected from H, fluoro, hydroxyl, cyano, *C*₁ - *C*₂₀ alkyl, *C*₁ - *C*₂₀ alkoxy, *C*₂ - *C*₂₀ alkenyl, or *C*₂ - *C*₂₀ alkynyl;
*G*₁ and *G*₂ each represent a phosphoramidite functional group having the structure shown in Formula (G-1) or a hydroxyl protecting group, provided that at least one of *G*₁ and *G*₂ has the structure shown in Formula (G-1), the hydroxyl protecting group is selected from any one of trityl, 4-methoxytrityl, 4,4'-dimethoxytrityl (DMTr), and 4,4',4''-trimethoxytrityl; 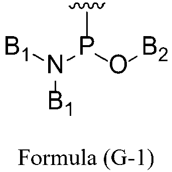
wherein, *B*₁ is selected from substituted or unsubstituted *C*₁ - *C*₅ hydrocarbyl;
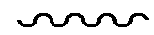 represents the site of covalent bond attachment of the group.

2. The compound according to claim 1, wherein *L*₁ and *L*₂ are independently selected from a linking combination of one or more of the following: A1, A2, A4, A6, A7, A8, A9, A10, and A11;
the ligand is independently selected from any one of the following: D-mannopyranose, L-mannopyranose, D-arabinose, D-xylofuranose, L-xylofuranose, D-glucose, L-glucose, D-galactose, L-galactose, *α*-D-mannofuranose, *β*-D-mannofuranose, *α*-D-mannopyranose, *β*-D-mannopyranose, *α*-D-glucopyranose, *β*-D-glucopyranose, *α*-D-glucofuranose, *β*-D-glucofuranose, *α*-D-fructofuranose, *α*-D-fructopyranose, *α*-D-galactopyranose, *β*-D-galactopyranose, *α*-D-galactofuranose, *β*-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-*β*-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-*α*-neuraminic acid, 5-thio-*β*-D-glucopyranose,methyl 2,3,4-tri-O-acetyl-1-thio-6-O-trityl-*α*-D-glucopyranoside, 4-thio-*β*-D-galactopyranose, ethyl 3,4,6,7-tetra-O-acetyl-2-deoxy-1,5-dithio-*α*-D-glucoheptopyranoside, 2,5-anhydro-D-allononitrile, ribose, D-ribose, D-4-thioribose, L-ribose, and L-4-thioribose;
b is 1, 2, or 3;
m is 0, 1, or 2;
*R*₂ and *R*₃ are independently selected from H, *C*₁ - *C*₆ alkyl, *C*₁ - *C*₆ alkoxy, *C*₂ - *C*₆ alkenyl, or *C*₂ - *C*₆ alkynyl;
Z is absent or represents a *C*₁ - *C*₃ alkylene;
*R*₄ and *R*₅ are independently selected from H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is 1, 2, or 3; 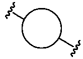
represents a *C*₄ - *C*₈ cycloalkyl or a *C*₃ - *C*₈ heterocyclyl; and *R*₁ is selected from H, fluoro, hydroxyl, cyano, *C*₁ - *C*₆ alkyl, *C*₁ - *C*₆ alkoxy, *C*₂ - *C*₆ alkenyl, or *C*₂ - *C*₆ alkynyl;
*B*₁ is methyl, ethyl, or isopropyl; *B*₂ is selected from one of *C*₁ - *C*₅ alkyl, cyanoethyl, cyanopropyl, or cyanobutyl.

3. The compound according to claim 2, wherein b is 1 or 3; 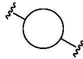
represents a *C*₃ - *C*₆ heterocyclyl;
*A*₀ is an N-acetylgalactosamine (GalNAc) group as shown below or a group formed by replacing all or part of the hydroxyl groups in the N-acetylgalactosamine group with a KCOO-group, wherein each K is independently selected from methyl, trifluoromethyl, difluoromethyl, monofluoromethyl, trichloromethyl, dichloromethyl, monochloromethyl, ethyl, n-propyl, isopropyl, phenyl, halophenyl, or alkylphenyl: 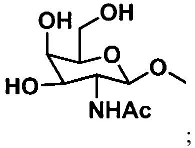
the hydroxyl protecting group is 4,4'-dimethoxytrityl;
the length of *L*₁ or *L*₂ is independently 1-25 atoms, wherein the length of *L*₁ or *L*₂ refers to the number of chain-forming atoms in the longest straight chain;
each j1 is independently an integer from 1-10, and each j2 is independently an integer from 1-10.

4. The compound according to claim 3, wherein 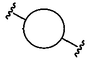 represents a four- to six-membered nitrogen-containing saturated cycloalkyl;
the length of *L*₁ or *L*₂ is independently 1-20 atoms;
each j1 is independently an integer from 1-8, and each j2 is independently an integer from 1-8.

5. The compound according to claim 2, wherein *L*₁ and *L*₂ are independently selected from a linking combination of at least 2 of A1, A2, A4, A8, A9, A10, and A11.

6. The compound according to claim 2, wherein *L*₁ and *L*₂ are independently selected from a linking combination of at least 2 of A1, A4, A8, A10, and A11.

7. The compound according to claim 1, wherein the compound has the structure (I-1) shown below: 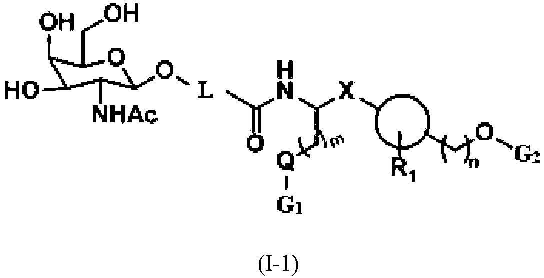 wherein,
L is A10, wherein j1 is an integer from 2-8, or is A11, wherein j2 is an integer from 1-7;
m is 0, 1, or 2;
n is 0, 1, 2, or 3;
Q represents a structural formula as shown in Formula (A13): 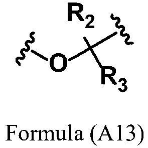
wherein, 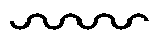 represents the site of covalent bond attachment of the group; *R*₂ and *R*₃ are independently selected from H, *C*₁ - *C*₆ alkyl, *C*₁ - *C*₆ alkoxy, *C*₂ - *C*₆ alkenyl, or *C*₂ - *C*₆ alkynyl; X represents a structural formula as shown in Formula (A14-1) or (A14-2): 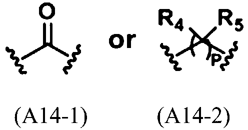
wherein *R*₄ and *R*₅ are independently selected from H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is 1, 2, or 3; optionally, *R*₄ and *R*₅ are directly connected to form a three- to six-membered saturated carbocyclic ring; 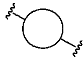
represents a four- to eight-membered all-carbon or nitrogen-containing saturated ring,
*R*₁ is selected from H, fluoro, hydroxyl, cyano, *C*₁ - *C*₆ alkyl, *C*₁ - *C*₆ alkoxy, *C*₂ - *C*₆ alkenyl, or *C*₂ - *C*₆ alkynyl;
wherein, the hydroxyl groups in 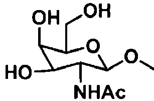 are all or partially replaced by a KCOO- group, wherein each K is independently selected from methyl, trifluoromethyl, difluoromethyl, monofluoromethyl, trichloromethyl, dichloromethyl, monochloromethyl, ethyl, n-propyl, isopropyl, phenyl, halophenyl, or alkylphenyl.

8. The compound according to claim 1, wherein 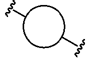 is a four-, five-, or six-membered saturated cycloalkyl containing one or two nitrogen atoms.

9. The compound according to claim 1, wherein the compound has any one of the structures shown below:

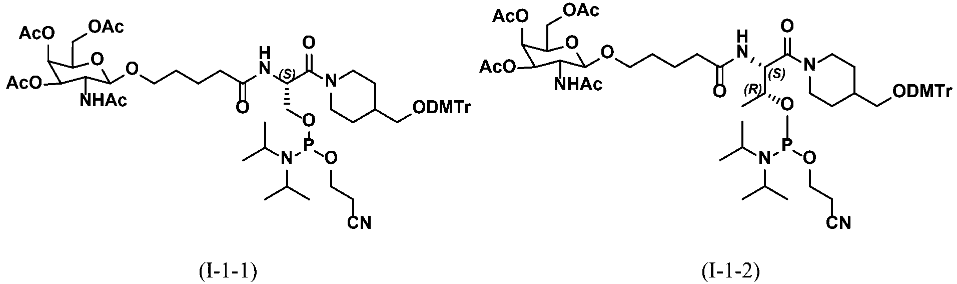

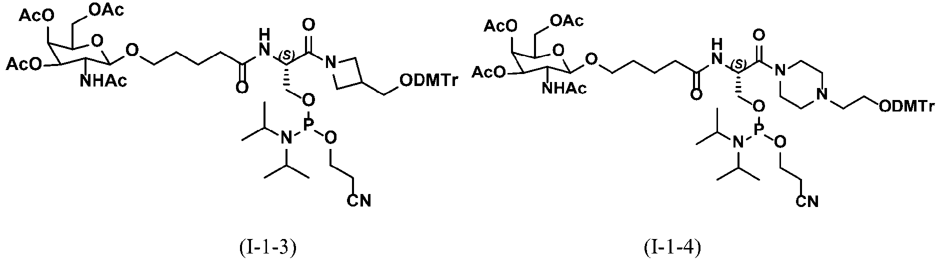

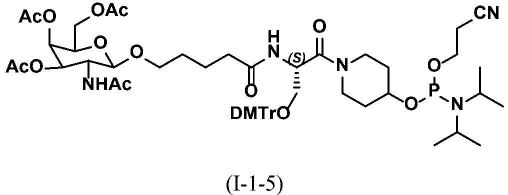

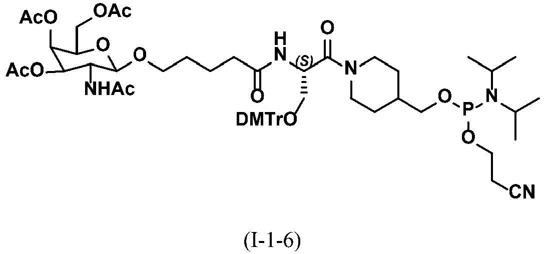

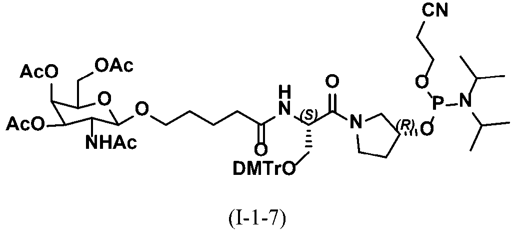

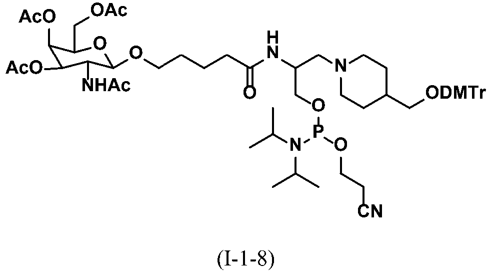

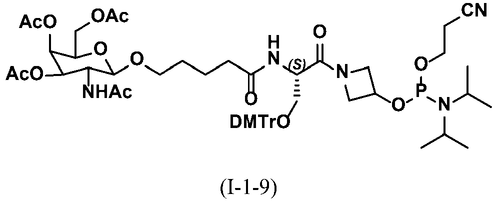

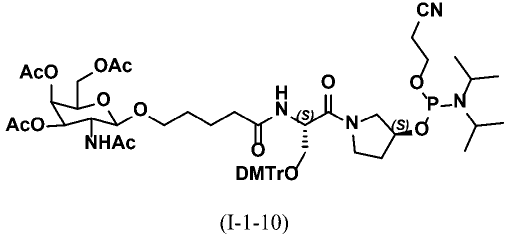

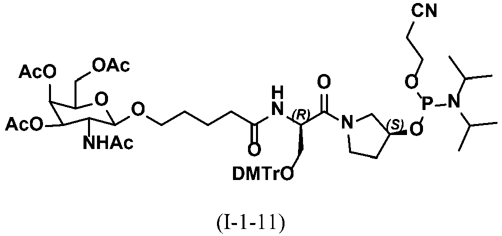

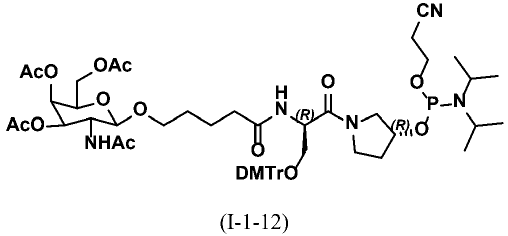

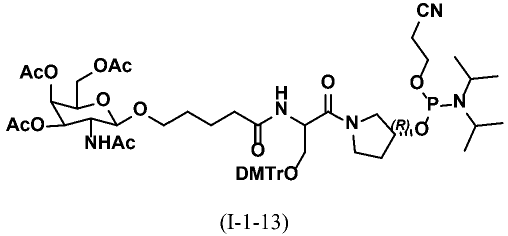

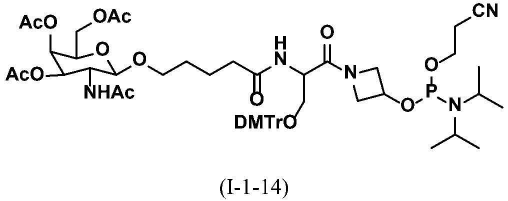

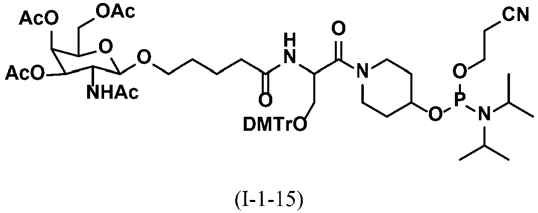

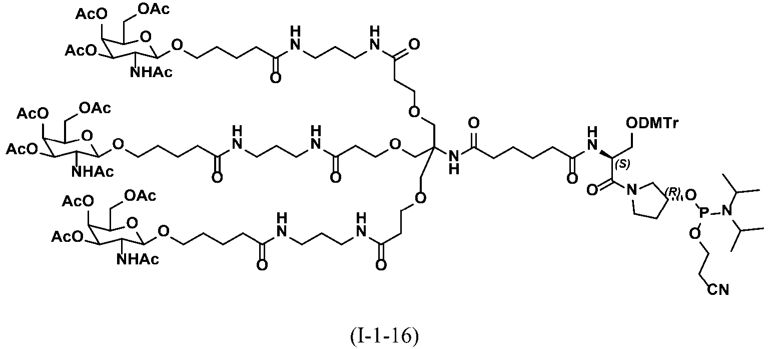

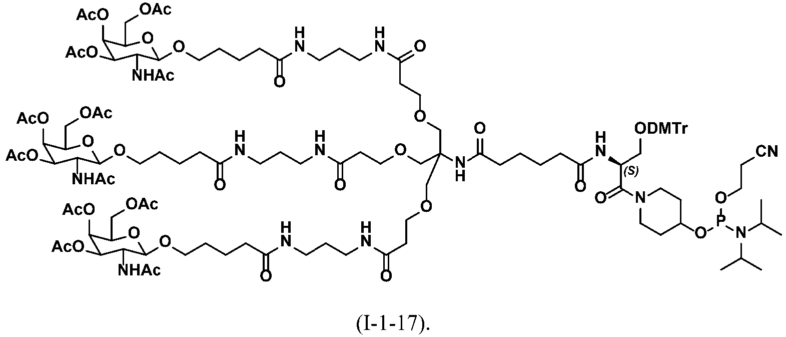

10. A nucleic acid conjugate, wherein the conjugate contains one or more structures as shown in Formula (II) connected to any position on an oligonucleotide sequence: 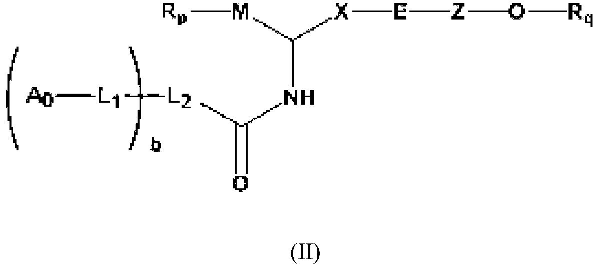 wherein,
*Rₚ* and *R_{q}* are each H or have the structure shown in Formula A16, provided that at least one of *Rₚ* and
*R_{q}* has the structure shown in Formula A16; 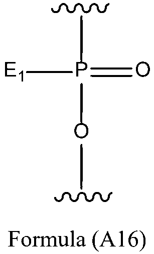
wherein, 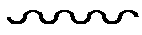 represents the site of covalent bond attachment of the group;
*E*₁ is OH, SH, or *BH*₂,
*A*₀ represents a ligand having affinity for the asialoglycoprotein receptor on the surface of mammalian liver cells, or represents a group formed by replacing all or part of the hydroxyl groups in the ligand with a KCOO- group, wherein each K is independently selected from the group consisting of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl;
b is an integer in the range of 1-4;
*L*₁ and *L*₂ independently represent a linking combination of one or more groups selected from Formula A1-A11: 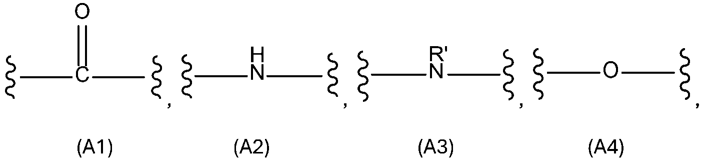 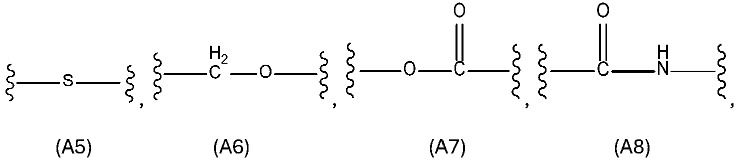 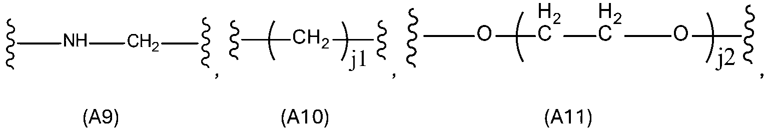
wherein, j1 is an integer from 1-20; j2 is an integer from 1-20; R' is a *C*₁ - *C*₁₀ alkyl;
M represents a structural formula as shown in Formula (A12): 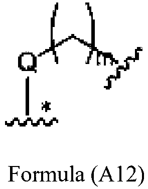
wherein, 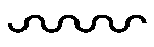 represents the site of covalent bond attachment of the group; m represents an integer from 0-6; * represents the bond connected to *Rₚ*,
Q represents a structural formula as shown in Formula (A13): 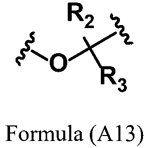
wherein, 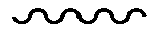 represents the site of covalent bond attachment of the group, 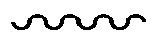 connected to O represents the bond connected to *Rₚ*; *R*₂ and *R*₃ are the same or different, and are independently selected from H, *C*₁ - *C*₂₀ alkyl, *C*₁ - *C*₂₀ alkoxy, *C*₂ - *C*₂₀ alkenyl, or *C*₂ - *C*₂₀ alkynyl, preferably H, *C*₁ - *C*₆ alkyl, *C*₁ - *C*₆ alkoxy, *C*₂ - *C*₆ alkenyl, or *C*₂ - *C*₆ alkynyl;
Z is absent or represents a *C*₁ - *C*₁₀ alkylene;
X represents a structural formula as shown in Formula (A14-1) or (A14-2): 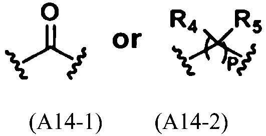
wherein *R*₄ and *R*₅ are the same or different, and are independently selected from H, fluoro, hydroxyl, *C*₁ - *C*₂₀ alkyl, *C*₁ - *C*₂₀ alkoxy, *C*₂ - *C*₂₀ alkenyl, or *C*₂ - *C*₂₀ alkynyl, preferably H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is an integer from 1-6; optionally, *R*₄ and *R*₅ are directly connected to form a three- to six-membered saturated carbocyclic ring;
E represents a structural formula as shown in Formula (A15): 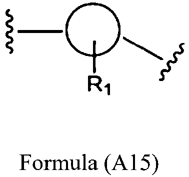
wherein, 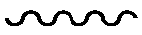 represents the site of covalent bond attachment of the group; 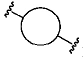 represents a *C*₃ - *C*₁₈ cycloalkyl or a *C*₃ - *C*₁₈ heterocyclyl, and *R*₁ is selected from H, fluoro, hydroxyl, cyano, *C*₁ - *C*₂₀ alkyl, *C*₁ - *C*₂₀ alkoxy, *C*₂ - *C*₂₀ alkenyl, or *C*₂ - *C*₂₀ alkynyl.

11. The nucleic acid conjugate according to claim 10, wherein the conjugate contains one or more structures as shown in Formula (II-A) connected to any position on an oligonucleotide sequence: 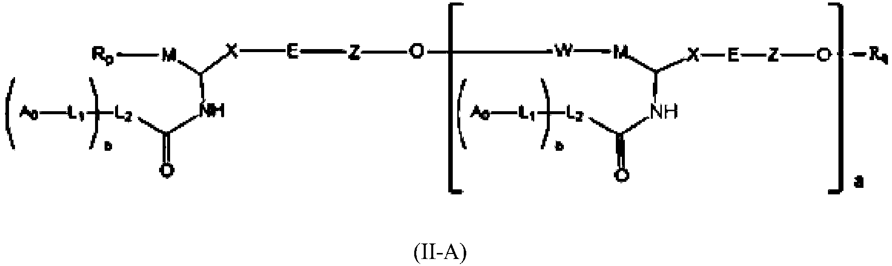 wherein,
a represents an integer from 0-7;
the structure of W is as shown in the following formula: 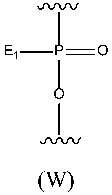
wherein, 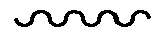 represents the site of covalent bond attachment of the group; *E*₁ is OH, SH, or *BH*₂.

12. The nucleic acid conjugate according to claim 11, wherein a represents an integer of 0, 1, 2, or 3.

13. The nucleic acid conjugate according to claim 10 or 11, wherein *L*₁ and *L*₂ are independently selected from a linking combination of one or more of the following: A1, A2, A4, A6, A7, A8, A9, A10, and A11;
the ligand is independently selected from any one of the following: D-mannopyranose, L-mannopyranose, D-arabinose, D-xylofuranose, L-xylofuranose, D-glucose, L-glucose, D-galactose, L-galactose, *α*-D-mannofuranose, *β*-D-mannofuranose, *α*-D-mannopyranose, *β*-D-mannopyranose, *α*-D-glucopyranose, *β*-D-glucopyranose, *α*-D-glucofuranose, *β*-D-glucofuranose, *α*-D-fructofuranose, *α*-D-fructopyranose, *α*-D-galactopyranose, *β*-D-galactopyranose, *α*-D-galactofuranose, *β*-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-*β*-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-*α*-neuraminic acid, 5-thio-*β*-D-glucopyranose,methyl 2,3,4-tri-O-acetyl-1-thio-6-O-trityl-*α*-D-glucopyranoside, 4-thio-*β*-D-galactopyranose, ethyl 3,4,6,7-tetra-O-acetyl-2-deoxy-1,5-dithio-*α*-D-glucoheptopyranoside, 2,5-anhydro-D-allononitrile, ribose, D-ribose, D-4-thioribose, L-ribose, and L-4-thioribose;
b is 1, 2, or 3;
m is 0, 1, or 2;
*R*₂, *R*₃ are independently selected from H, *C*₁ - *C*₆ alkyl, *C*₁ - *C*₆ alkoxy, *C*₂ - *C*₆ alkenyl, or *C*₂ - *C*₆ alkynyl;
Z is absent or represents a *C*₁ - *C*₃ alkylene;
*R*₄ and *R*₅ are independently selected from H, fluoro, hydroxyl, *C*₁ - *C*₆ alkyl, *C*₁ - *C*₆ alkoxy, *C*₂ - *C*₆ alkenyl, or *C*₂ - *C*₆ alkynyl; p is 1, 2, or 3; 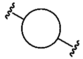
represents a *C*₄ - *C*₈ cycloalkyl or a *C*₃ - *C*₈ heterocyclyl; and *R*₁ is selected from H, fluoro, hydroxyl, cyano, *C*₁ - *C*₆ alkyl, *C*₁ - *C*₆ alkoxy, *C*₂ - *C*₆ alkenyl, or *C*₂ - *C*₆ alkynyl;
*B*₁ is methyl, ethyl, or isopropyl; *B*₂ is selected from one of *C*₁ - *C*₅ alkyl, cyanoethyl, cyanopropyl, or cyanobutyl.

14. The nucleic acid conjugate according to claim 13, wherein b is 1 or 3; 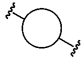
represents a *C*₃ - *C*₆ heterocyclyl;
*A*₀ is an N-acetylgalactosamine (GalNAc) group as shown below or a group formed by replacing all or part of the hydroxyl groups in the N-acetylgalactosamine group with a KCOO-group, wherein each K is independently selected from methyl, trifluoromethyl, difluoromethyl, monofluoromethyl, trichloromethyl, dichloromethyl, monochloromethyl, ethyl, n-propyl, isopropyl, phenyl, halophenyl, or alkylphenyl: 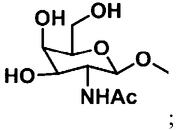
the hydroxyl protecting group is 4,4'-dimethoxytrityl;
the length of *L*₁ or *L*₂ is independently 1-25 atoms, wherein the length of *L*₁ or *L*₂ refers to the number of chain-forming atoms in the longest straight chain;
each j1 is independently an integer from 1-10, and each j2 is independently an integer from 1-10.

15. The nucleic acid conjugate according to claim 14, wherein 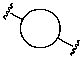 represents a four- to six-membered nitrogen-containing saturated cycloalkyl;
the length of *L*₁ or *L*₂ is independently 1-20 atoms;
each j1 is independently an integer from 1-8, and each j2 is independently an integer from 1-8.

16. The nucleic acid conjugate according to claim 10 or 11, wherein *L*₁ and *L*₂ are independently selected from a linking combination of at least 2 of A1, A2, A4, A8, A9, and A10.

17. The nucleic acid conjugate according to claim 10 or 11, wherein *L*₁ and *L*₂ are independently selected from a linking combination of at least 2 of A1, A4, A8, and A10.

18. The nucleic acid conjugate according to claim 10 or 11, which has any one of the structures shown below: 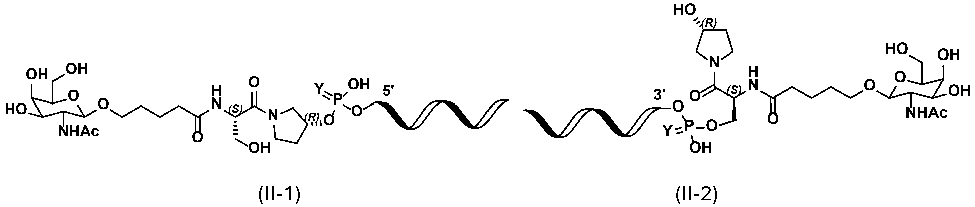 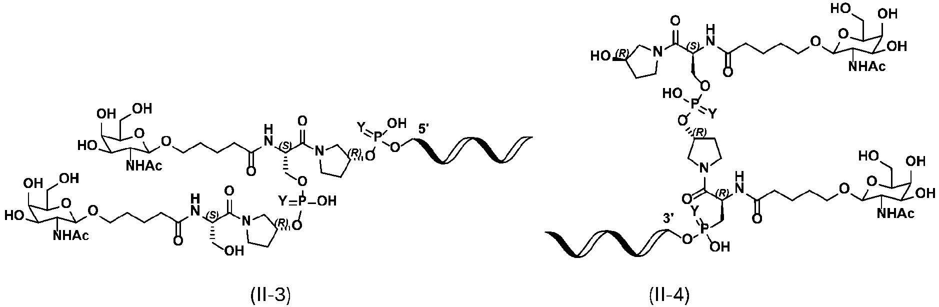 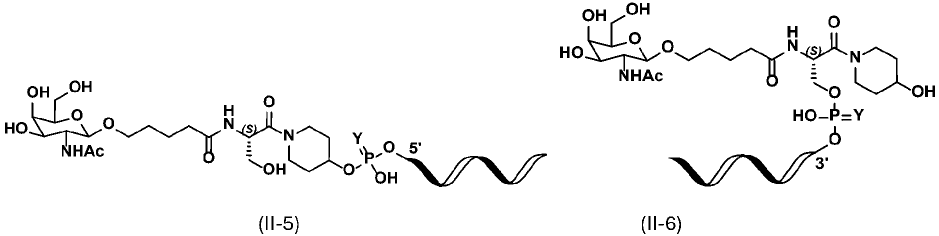 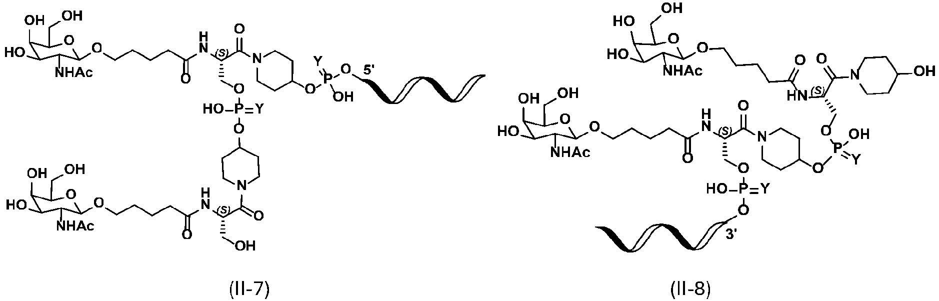 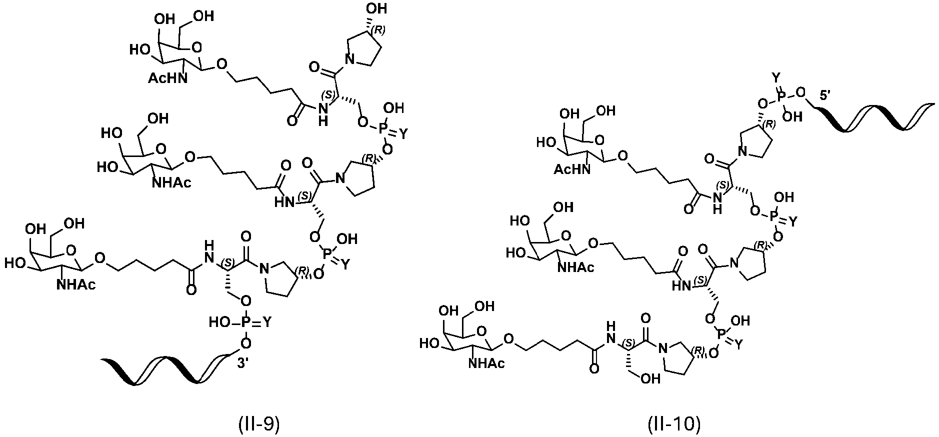 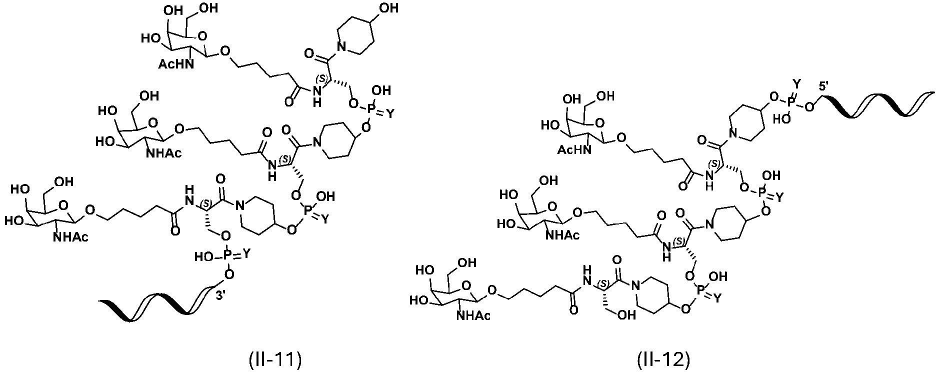 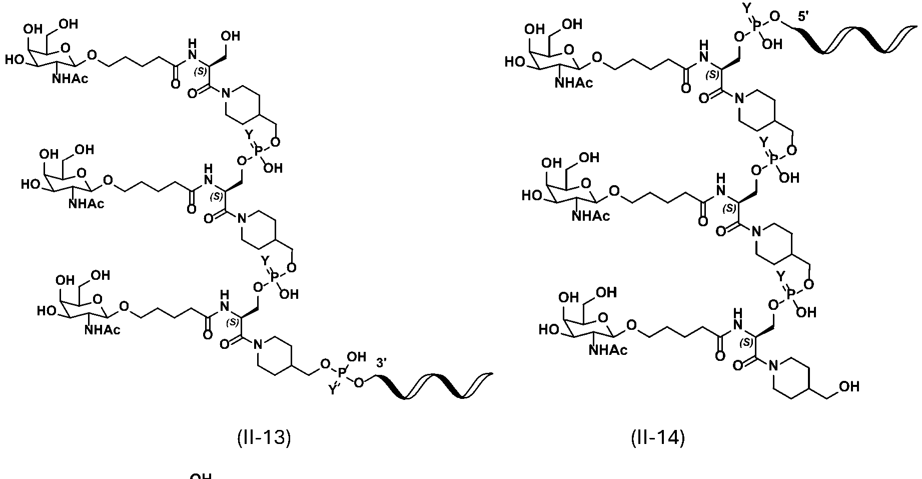 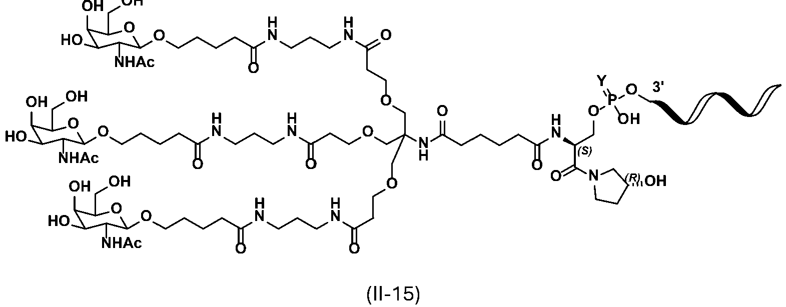 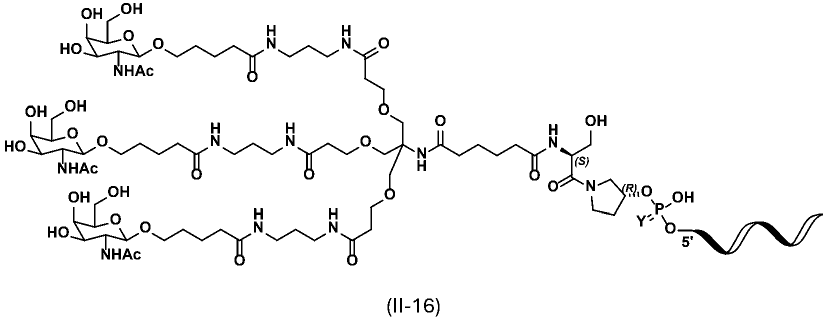 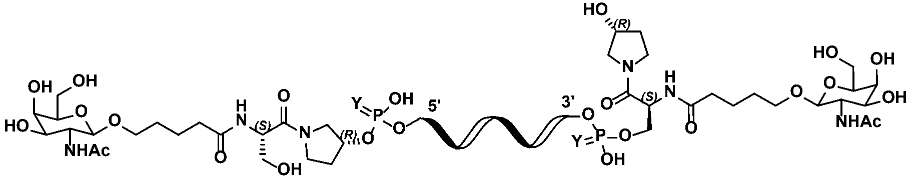 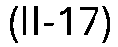 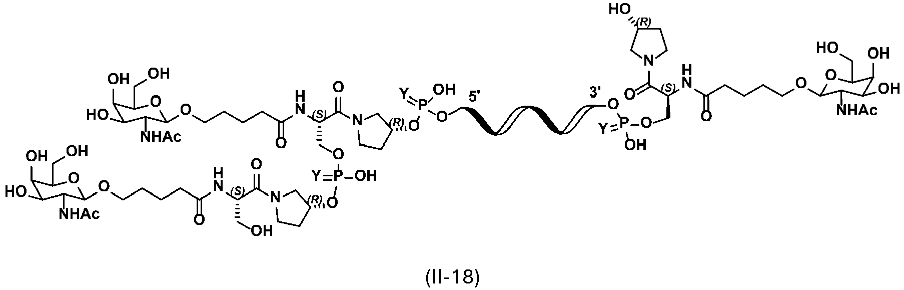 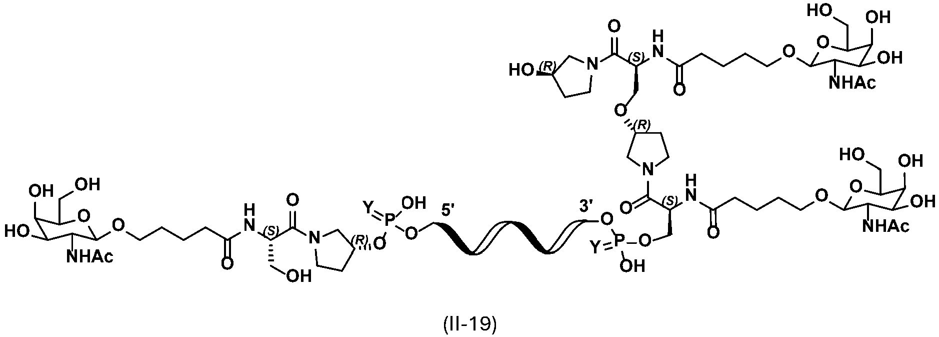 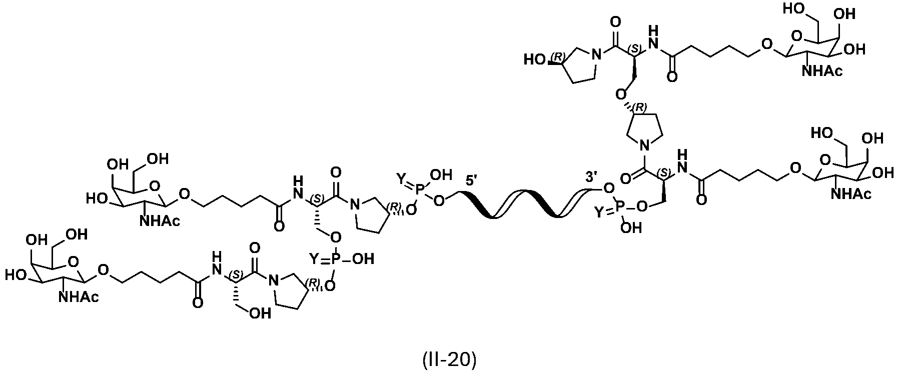 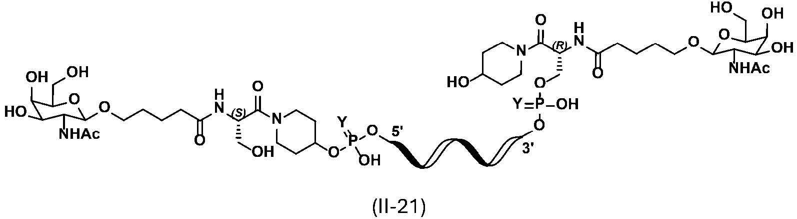 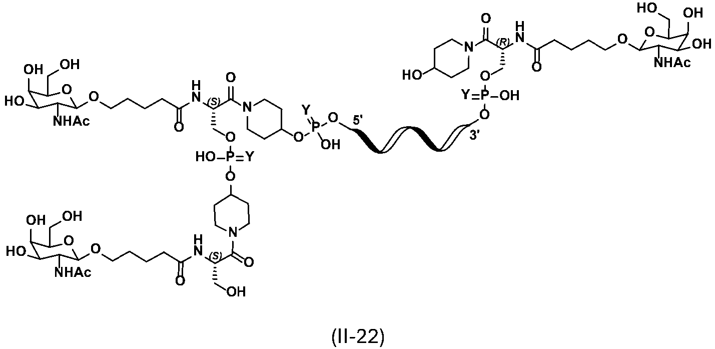 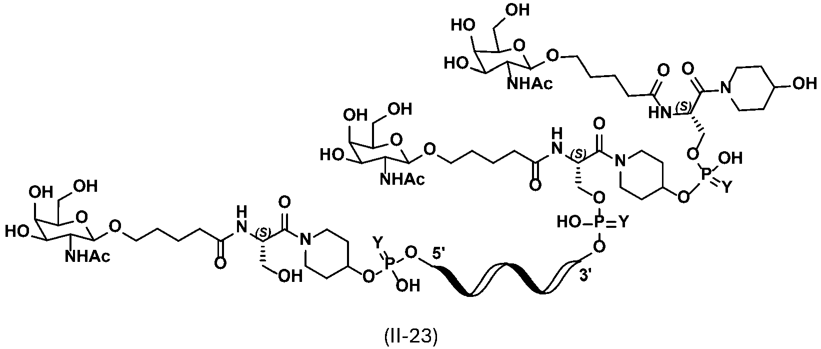 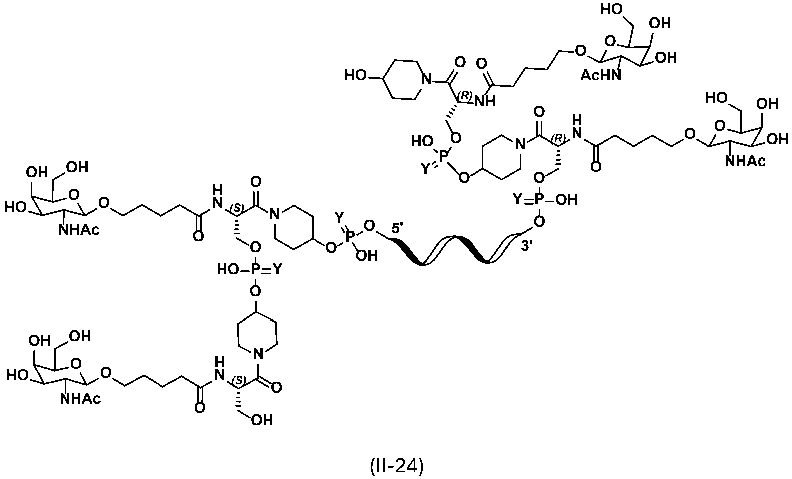 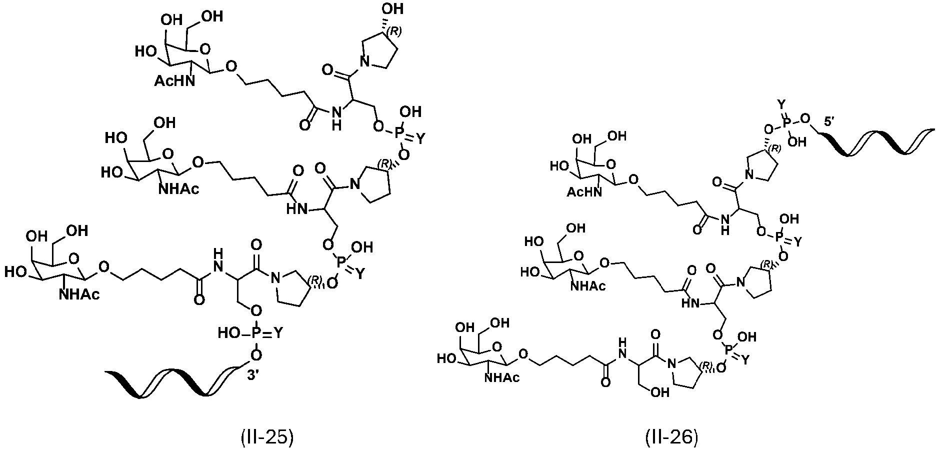 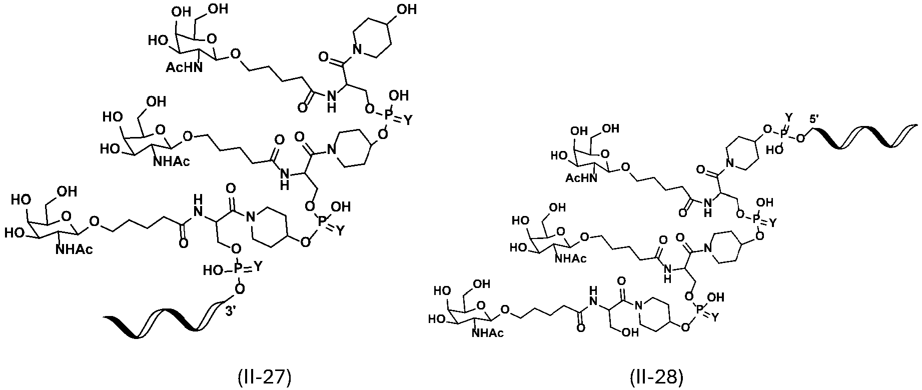 wherein Y is O or S, 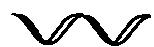 is an oligonucleotide.

19. The nucleic acid conjugate according to claim 10 or 11, wherein the oligonucleotide is selected from one of the following: small interfering RNA, microRNA, anti-microRNA, microRNA antagonist, microRNA mimetic, decoy oligonucleotide, immunostimulant, G-quadruplex, splice variant, single-stranded RNA, antisense nucleic acid, nucleic acid aptamer, stem-loop RNA, mRNA fragment, and activating RNA; optionally, the oligonucleotide is a single-stranded oligonucleotide or a double-stranded oligonucleotide; optionally, the oligonucleotide is a single-stranded oligonucleotide, the P atom in Formula (A16) is connected to an end of the single-stranded oligonucleotide, the end of the single-stranded oligonucleotide refers to the first 4 nucleotides from one end of the single-stranded oligonucleotide; optionally, the P atom in Formula (A16) is connected to a terminus of the single-stranded oligonucleotide; optionally, the P atom in Formula (A16) is connected to the 3' terminus of the single-stranded oligonucleotide; optionally, the oligonucleotide is a double-stranded oligonucleotide, the double-stranded oligonucleotide comprises a sense strand and an antisense strand, the P atom in the Formula (A16) is connected to an end of the double-stranded oligonucleotide, the end of the double-stranded oligonucleotide refers to the first 4 nucleotides from one end of the sense strand or the antisense strand; optionally, the P atom in Formula (A16) is connected to a terminus of the sense strand or the antisense strand; optionally, the P atom in Formula (A16) is connected to the 5' terminus of the antisense strand; optionally, the P atom in Formula (A16) is connected to the 2', 3', or 5' position of a nucleotide in the nucleic acid conjugate by forming a phosphodiester bond.

20. The nucleic acid conjugate according to claim 19, wherein the double-stranded oligonucleotide is an siRNA.

21. A method for preparing the nucleic acid conjugate according to any one of claims 10-20, comprising oxidizing the phosphoramidite functional group in the compound of Formula (I) according to any one of claims 1-9 to the structure shown in Formula (W), then coupling with an oligonucleotide, followed by cleavage and removal of protecting groups to obtain the conjugate; 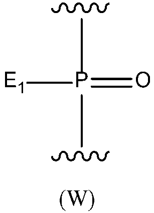
wherein, 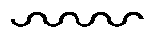 represents the site of covalent bond attachment of the group;
*E*₁ is OH, SH, or *BH*₂.

22. Use of the nucleic acid conjugate according to any one of claims 10-20 in the manufacture of a medicament for treating and/or preventing liver-derived diseases.

23. A method for treating a pathological condition or disease caused by the expression of a gene in hepatocytes, the method comprising administering the nucleic acid conjugate according to any one of claims 10-20 to a patient suffering from the disease.

24. A kit, wherein the kit comprises the nucleic acid conjugate according to any one of claims 10-20.
